# FASCICULE DE BREVET EUROPEEN

(11) **EP 1 659 112 B1**
(45) Date de publication et mention de la délivrance du brevet: **20.06.2007**
(21) Numéro de dépôt: 05292189.7
(22) Date de dépôt: 18.10.2005
(51) Int. Cl.: C07D 213/74, C07D 215/38, C07D 217/22, A61K 31/444, A61P 25/16

(54) **Dérivés d'arylpipérazine comme ligands sélectifs du récepteur D3 de la dopamine**
Arylpiperazinderivate als selektive Liganden für den Dopamin D3 Rezeptor
Arylpiperazuine derivatives as selective ligand for the dopamine D3 receptor

(30) Priorité: 22.10.2004 FR 0411303
(43) Date de publication de la demande: 24.05.2006
(73) Titulaire: BIOPROJET, 75003 Paris (FR)
(72) Inventeur: Capet, Marc, 35520 Melesse (FR); Danvy, Denis, 76190 Yvetot (FR); Levoin, Nicolas, 35310 Mordelles (FR); Morvan, Marcel, 35740 Pace (FR); Berrebi-Bertrand, Isabelle, 35740 Pace (FR); Calmels, Thierry, 35520 Melesse (FR); Robert, Philippe, 35740 Pace (FR); Schwartz, Jean-Charles, 75014 Paris (FR); Lecomte, Jeanne-Marie, 75003 Paris (FR)
(74) Mandataire: Colombet, Alain André

(56) Documents cités:
- WO-A-00/18767
- WO-A-97/43271
- WO-A-98/56786
- WO-A-99/21848

## Description

La présente invention concerne de nouveaux dérivés d'arylpipérazine, leur procédé de préparation et leur utilisation en tant qu'agents thérapeutiques.

Plus précisément, les composés selon la présente invention présentent la propriété d'être des ligands sélectifs du récepteur D3 de la dopamine.

De nombreux dérivés d'arylpipérazine sont déjà connus comme antagonistes de la dopamine au niveau de ses récepteurs de type D2, certains d'entre eux jouissant de propriétés neuroleptiques, ou encore comme antagonistes de la sérotonine ou de la noradrénaline.

Le brevet DE 2 143 730 décrit des dérivés d'arylpipérazine présentant des propriétés analgésiques et antihypertenseurs ; néanmoins ces composés présentent un cycle hétéroaryle, éventuellement substitué par divers groupes non aromatiques.

Les demandes WO 99/21848, WO 97/43271, WO00/18767, WO 98/56786 et DE 2258561 décrivent également des composés de structures proches.

Il a maintenant été découvert, et ce de façon totalement inattendue, que les composés selon l'invention, constituant une nouvelle série de dérivés d'arylpipérazine présentaient une forte affinité pour le récepteur D3 de la dopamine.

Ces ligands sélectifs de D3 sont utiles comme médicaments en neuropsychiatrie, notamment dans le traitement des états psychotiques ou dépressifs, dans le traitement de désordres moteurs tels que les dyskinésies ou tremblements essentiels. En outre, ils sont utiles dans le traitement des états de dépendance à la nicotine, la cocaïne, l'alcool, les morphiniques ainsi que pour faciliter la désaccoutumance chez les sujets pharmacodépendants.

La présente invention concerne donc les composés de formule (I) : 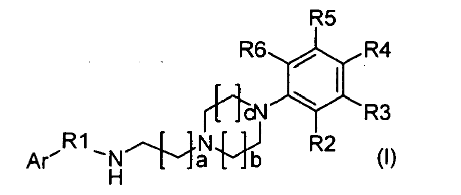 dans laquelle :
■ R1 représente un hétéroaryle à cinq ou six chaînons, contenant un ou plusieurs hétéroatomes, choisi parmi 2-pyridyle, 2-pyrimidinyle, 2-pyridazinyle, 2-pyrazinyle, 2-imidazolyle, 2-oxazolyle, 2-thiazolyle, 3-isoxazolyle, 3-isothiazolyle, 1,2,4-triazol-2-yle, 1,3,4-oxadiazol-2-yle, 1,3,4-thiadiazol-2-yle, éventuellement substitué par un ou plusieurs groupements identiques ou différents choisis parmi un atome d'halogène ou un groupe hydroxy, alkyle, monofluoroalkyle, polyfluoroalkyle, alkoxy, polyfluoro-alkoxy, alkylsulfanyle, polyfluoroalkylsulfanyle ;
■ Ar est un aryle ou hétéroaryle, éventuellement substitué par un ou plusieurs substituants identiques ou différents choisis parmi un atome d'halogène ou un groupe hydroxy, alkyle, monofluoroalkyle, polyfluoroalkyle, alkoxy, polyfluoroalkoxy, alkylsulfanyle, polyfluoroalkylsulfanyle, cyano, carbamoyle, dialkylcarbamoyle, Alkyl-C(=O)-, Alkyl-O-C(=O)-, HO-C(=O)-, (HO)Alkyl-, ou Ar est fusionné avec un cycle hydrocarboné ou hétérocycle, saturé, insaturé ou aromatique ;
■ a = 2, 3 ou 4 ;
■ b et c identiques ou différents représentent 1 ou 2 ;
■ R2, R3, R4, R5 et R6 représentent chacun indépendamment un atome d'hydrogène ou d'halogène ou un groupement hydroxy, alkyle, monofluoroalkyle, polyfluoroalkyle, alkoxy, polyfluoroalkoxy, alkylsulfanyle, polyfluoroalkylsulfanyle, cyano, -NRR', -COOR, -COR, -CONRR' ou bien deux des R2, R3, R4, R5 et R6 adjacents sont reliés entre eux pour former un cycle hydrocarboné ou un hétérocycle saturé ou insaturé, fusionné au noyau phényle auquel ils sont rattachés ;
où R, R' identiques ou différents représentent indépendamment un atome d'hydrogène, ou un groupe alkyle ; ,
ainsi que leurs stéréoisomères ou leur mélanges, leurs formes tautomères, leurs hydrates, solvates, leurs sels et esters pharmaceutiquement acceptables.

De préférence, la présente invention concerne les composés de formule (I) dans laquelle :
■ R1 représente un hétéroaryle à cinq ou six chaînons, contenant un ou plusieurs hétéroatomes, choisi parmi 2-pyridyle, 2-pyrimidinyle, 2-pyridazinyle, 2-pyrazinyle, 2-imidazolyle, 2-oxazolyle, 2-thiazolyle, 3-isoxazolyle, 3-isothiazolyle, 1,2,4-triazol-2-yle, 1,3,4-oxadiazol-2-yle, 1,3,4-thiadiazol-2-yle, éventuellement substitué par un ou plusieurs groupements identiques ou différents choisis parmi un atome d'halogène ou un groupe hydroxy, alkyle, monofluoroalkyle, polyfluoroalkyle, alkoxy, polyfluoro-alkoxy, alkylsulfanyle, polyfluoroalkylsulfanyle ;
■ Ar est un aryle ou hétéroaryle, éventuellement substitué par un ou plusieurs substituants identiques ou différents choisis parmi un atome d'halogène ou un groupe hydroxy, alkyle, monofluoroalkyle, polyfluoroalkyle, alkoxy, polyfluoroalkoxy, alkylsulfanyle, polyfluoroalkylsulfanyle, cyano, carbamoyle, dialkylcarbamoyle, Alkyl-C(=O)-, Alkyl-O-C(=O)-, HO-C(=O)-, (HO)Alkyl-, ou Ar est fusionné avec un cycle hydrocarboné ou hétérocycle, saturé, insaturé ou aromatique ;
■ a = 2, 3 ou 4 ;
■ b et c identiques ou différents représentent 1 ou 2 ;
■ R2, R3, R4, R5 et R6 représentent chacun indépendamment un atome d'hydrogène ou d'halogène ou un groupement hydroxy, alkyle, monofluoroalkyle, polyfluoroalkyle, alkoxy, polyfluoroalkoxy, alkylsulfanyle, polyfluoroalkylsulfanyle, cyano, -NRR', -COOR, -COR, -CONRR' ou bien deux des R2, R3, R4, R5 et R6 adjacents sont reliés entre eux pour former un cycle hydrocarboné ou un hétérocycle saturé ou insaturé, fusionné au noyau phényle auquel ils sont rattachés ;
où R, R' identiques ou différents représentent indépendamment un atome d'hydrogène, ou un groupe alkyle ;
ainsi que leurs stéréoisomères ou leur mélanges, leurs formes tautomères, leurs hydrates, solvates, leurs sels et esters pharmaceutiquement acceptables.

De préférence, R1 représente 2-pyridyle.

De préférence, Ar est un aryle, plus préférentiellement phényle, éventuellement substitué par un ou plusieurs substituants identiques ou différents choisis parmi un atome d'halogène ou un groupe alkyle.

De préférence, - a = 3.

De préférence, b et c représentent 1.

De préférence, R2, R3, R4, R5 et R6 représentent chacun indépendamment un atome d'hydrogène ou d'halogène ou un groupement polyfluoroalkyle, tel qu'un groupe perfluoroalkyle, tel que trifluorométhyle.

Les composés de formule (I) peuvent être notamment choisis parmi :
- 2-{4-[4-(2-fluorophényl)pipérazin-1-yl]butyl}amino-5-phénylpyridine
- 2-{4-[4-(2-fluorophényl)pipérazin-1-yl]butyl}amino-4-phénylpyridine
- 2-{4-[4-(3-trifluorométhylphényl)pipérazin-1-yl]butyl}amino-4-phénylpyridine
- Dichlorhydrate de 2-{4-[4-(3-trifluorométhylphényl)pipérazin-1-yl]butyl}amino-4-phénylpyridine
- 2-{4-[4-(3-trifluorométhylphényl)pipérazin-1-yl]butyl}amino-5-phénylpyridine
- 2-{4-[4-(2,3-dichlorophényl)pipérazin-1-yl]butyl}amino-4-phénylpyridine
- 2-{4-[4-(2,3-dichlorophényl)pipérazin-1-yl]butyl}amino-5-phénylpyridine
- 2-{4-[4-(2,3-dichlorophényl)pipérazin-1-yl]butyl}amino-5-(2-méthylphényl)pyridine
- 2-{4-[4-(3-trifluorométhylphényl)pipérazin-1-yl]butyl}amino-5-(2-méthylphényl)pyridine
- 2-{4-[4-(3-trifluorométhylphényl)pipérazin-1-yl]butyl}amino-5-(4-fluorophényl)pyridine
- 2-{4-[4-(3-trifluorométhylphényl)pipérazin-1-yl]butyl}amino-4-(2-méthylphényl)pyridine
- 2-{4-[4-(2,3-dichlorométhylphényl)pipérazin-1-yl]butyl}amino-4-(2-méthylphényl)pyridine
- 2-{4-[4-(2-cyano-3-trifluorométhylphényl)pipérazin-1-yl]butyl}amino-4-phénylpyridine
- 2-{4-[4-(3-chlorophényl)pipérazin-1-yl]butyl}amino-4-phénylpyridine
- dichlorhydrate du 2-{4-[4-(5,6,7,8-tétrahydro-1-naphtyl)pipérazin-1-yl]butyl}amino-4-phénylpyridine
- 2-{4-[4-(2-cyano-3-isopropoxyphényl)pipérazin-1-yl]butyl}amino-4-phénylpyridine
- 2-{4-[4-(3-trifluorométhylphényl)pipérazin-1-yl]butyl}amino-4-(4-fluorophényl)pyridine
- 2-{4-[4-(3-trifluorométhylphényl)pipérazin-1-yl]butyl}amino-4-(4-chlororophényl)pyridine
- 2-{4-[4-(3-trifluorométhylphényl)pipérazin-1-yl]butyl}amino-4-(3-fluorophényl)pyridine
- 2-{4-[4-(3-trifluorométhylphényl)pipérazin-1-yl]butyl}amino-4-(4-méthoxyphényl)pyridine
- 2-{4-[4-(3-trifluorométhylphényl)pipérazin-1-yl]butyl}amino-4-(3-thiényl)pyridine
- 2-{4-[4-(3-trifluorométhylphényl)pipérazin-1-yl]butyl}amino-4-(2-furyl)pyridine
- 2-{4-[4-(3-trifluorométhylphényl)pipérazin-1-yl]butyl}amino-4-(2-thiényl)pyridine
- 2-{4-[4-(3-trifluorométhylphényl)pipérazin-1-yl]butyl}amino-4-(2-fluorophényl)pyridine
- 2-{4-[4-(3-trifluorométhylphényl)pipérazin-1-yl]butyl}amino-[4,4']bipyridine
- 2-{4-[4-(3-trifluorométhylphényl)pipérazin-1-yl]butyl}amino-4-(3-méthylphényl)pyridine
- 2-{4-[4-(3-trifluorométhylphényl)pipérazin-1-yl]butyl}amino-4-(3-méthoxyphényl)pyridine
- 2-{4-[4-(3-trifluorométhylphényl)pipérazin-1-yl]butyl}amino[4,3']bipyridine
- 2-{4-[4-(3-trifluorométhylphényl)pipérazin-1-yl]butyl}amino-4-(4-trifluorométhoxyphényl)pyridine
- 2-{4-[4-(3-trifluorométhylphényl)pipérazin-1-yl]butyl}amino-4-(4-acétylphényl)pyridine
- 2-{4-[4-(3-trifluorométhylphényl)pipérazin-1-yl]butyl}amino-4-(3-acétylphényl)pyridine
- 2-{4-[4-(3-trifluorométhylphényl)pipérazin-1-yl]butyl}amino-4-(4-hydroxyméthylphényl)pyridine
- 2-{4-[4-(3-trifluorométhylphényl)pipérazin-1-yl]butyl}amino-4-(benzo[1,3]dioxol-5-yl)pyridine
- 2-{4-[4-(3-trifluorométhylphényl)pipérazin-1-yl]butyl}amino-4-(4-éthoxycarbonylphényl)pyridine
- 2-{4-[4-(3-trifluorométhylphényl)pipérazin-1-yl]butyl}amino-4-(4-(1-hydroxyéthyl)phényl)pyridine
- 2-{4-[4-(3-trifluorométhylphényl)pipérazin-1-yl]butyl}amino-4-(4-(1-hydroxy-1-méthyléthyl)phényl)pyridine
- 2-{4-[4-(3-trifluorométhylphényl)pipérazin-1-yl]butyl}amino-4-(3-(1-hydroxy-1-méthyléthyl)phényl)pyridine
- sel de sodium de la 2-{4-[4-(3-trifluorométhylphényl)pipérazin-1-yl]butyl}amino-4-(4-carboxyphényl)pyridine
- 2-{4-[4-(3-trifluorométhylphényl)pipérazin-1-yl]butyl}amino-4-phénylthiazole
- 2-{4-[4-(3-trifluorométhylphényl)pipérazin-1-yl]butyl}amino-5-phényloxazole ainsi que leurs stéréoisomères ou leur mélanges, leurs formes tautomères , leurs hydrates, solvates, leurs sels, formes libres et esters pharmaceutiquement acceptables.

Plus préférentiellement, les composés de formule (I) peuvent être choisis parmi :
- 2-{4-[4-(3-trifluorométhylphényl)pipérazin-1-yl]butyl}amino-4-phénylpyridine
- Dichlorhydrate de 2-{4-[4-(3-trifluorométhylphényl)pipérazin-1-yl]butyl}amino-4-phénylpyridine
- 2-{4-[4-(2,3-dichlorophényl)pipérazin-1-yl]buty}amino-4-phénylpyridine, ainsi que leurs stéréoisomères ou leur mélanges, leurs formes tautomères , leurs hydrates, solvates, leurs sels, formes libres et esters pharmaceutiquement acceptables.

Selon la présente invention, les radicaux Alkyle représentent des radicaux hydrocarbonés saturés, en chaîne droite ou ramifiée, de 1 à 20 atomes de carbone, de préférence de 1 à 5 atomes de carbone.

On peut notamment citer, lorsqu'ils sont linéaires, les radicaux méthyle, éthyle, propyle, butyle, pentyle, hexyle, octyle, nonyle, décyle, dodécyle, hexadécyle, et octadécyle.

On peut notamment citer, lorsqu'ils sont ramifiés ou substitués par un ou plusieurs radicaux alkyle, les radicaux isopropyle, tert-butyl, 2-éthylhexyle, 2-méthylbutyle, 2-méthylpentyle, 1-méthylpentyle et 3-méthylheptyle.

Les radicaux Alkoxy selon la présente invention sont des radicaux de formule -O-Alkyle, l'alkyle étant tel que défini précédemment.

Parmi les atomes d'Halogène, on cite plus particulièrement les atomes de fluor, de chlore, de brome et d'iode, de préférence le fluor.

Les radicaux Alkényle représentent des radicaux hydrocarbonés, en chaîne droite ou linéaire, et comprennent une ou plusieurs insaturations éthyléniques. Parmi les radicaux Alkényle, on peut notamment citer les radicaux allyle ou vinyle.

Parmi les atomes d'Halogène, on cite plus particulièrement les atomes de fluor, de chlore, de brome et d'iode, de préférence le fluor.

Les radicaux Alkényle représentent des radicaux hydrocarbonés, en chaîne droite ou linéaire, et comprennent une ou plusieurs insaturations éthyléniques. Parmi les radicaux Alkényle, on peut notamment citer les radicaux allyle ou vinyle.

Les radicaux Alkynyle représentent des radicaux hydrocarbonés, en chaîne droite ou linéaire, et comprennent une ou plusieurs insaturations acétyléniques. Parmi les radicaux Alkynyle, on peut notamment citer l'acétylène.

Le radical Cycloalkyle est un radical hydrocarboné mono-, bi- ou tri-cyclique saturé ou partiellement insaturé, non aromatique, de 3 à 10 atomes de carbone, tel que notamment le cyclopropyle, cyclopentyle, cyclohexyle ou adamantyle, ainsi que les cycles correspondants contenant une ou plusieurs insaturations.

Aryle désigne un système aromatique hydrocarboné, mono ou bicyclique de 6 à 10 atomes de carbone.

Parmi les radicaux Aryle, on peut notamment citer le radical phényle ou naphtyle, plus particulièrement substitué par un moins un atome d'halogène.

Parmi les radicaux -AlkyleAryle, on peut notamment citer le radical benzyle ou phénétyle.

Les radicaux Hétéroaryles désignent les systèmes aromatiques comprenant un ou plusieurs hétéroatomes choisis parmi l'azote, l'oxygène ou le soufre, mono ou bicyclique, de 5 à 10 atomes de carbone. Parmi les radicaux Hétéroaryles, on pourra citer le pyrazinyle, le thiényle, l'oxazolyle, le furazanyle, le pyrrolyle, le 1,2,4-thiadiazolyle, le naphthyridinyle, le pyridazinyle, le quinoxalinyle, le phtalazinyle, l'imidazo[1,2-a]pyridine, l'imidazo[2,1-b]thiazolyle, le cinnolinyle, le triazinyle, le benzofurazanyle, l'azaindolyle, le benzimidazolyle, le benzothiényle, le thiénopyridyle, le thiénopyrimidinyle, le pyrrolopyridyle, l'imidazopyridyle, le benzoazaindole, le 1,2,4-triazinyle, le benzothiazolyle, le furanyle, l'imidazolyle, l'indolyle, le triazolyle, le tétrazolyle, l'indolizinyle, l'isoxazolyle, l'isoquinolinyle, l'isothiazolyle, l'oxadiazolyle, le pyrazinyle, le pyridazinyle, le pyrazolyle, le pyridyle, le pyrimidinyle, le purinyle, le quinazolinyle, le quinolinyle, l'isoquinolyle, le 1,3,4-thiadiazolyle, le thiazolyle, le triazinyle, l'isothiazolyle, le carbazolyle, ainsi que les groupes correspondants issus de leur fusion ou de la fusion avec le noyau phényle.

L'expression « sels pharmaceutiquement acceptables » fait référence aux sels d'addition acide relativement non toxiques, inorganiques et organiques, et les sels d'addition de base, des composés de la présente invention. Ces sels peuvent être préparés *in situ* pendant l'isolement final et la purification des composés. En particulier, les sels d'addition acide peuvent être préparés en faisant réagir séparément le composé purifié sous sa forme épurée avec un acide organique ou inorganique et en isolant le sel ainsi formé. Parmi les exemples de sels d'addition acide on trouve les sels bromhydrate, chlorhydrate, sulfate, bisulfate, phosphate, nitrate, acétate, oxalate, valerate, oléate, palmitate, stéarate, laurate, borate, benzoate, lactate, phosphate, tosylate, citrate, maléate, fumarate, succinate, tartrate, naphthylate, mésylate, glucoheptanate, lactobionate, sulfamates, malonates, salicylates, propionates, méthylènebis-beta-hydroxynaphtoates, acide gentisique, iséthionates, di-p-toluoyltartrates, methanesulfonates, éthanesulfonates, benzenesulfonates, p-toluenesulfonates, cyclohexyl sulfamates et quinateslaurylsulfonate, et analogues. (Voir par exemple S.M. Berge et al. « Pharmaceutical Salts » J. Pharm. Sci, 66 :p.1-19 (1977) qui est incorporé ici en référence). Les sels d'addition acide peuvent également être préparés en faisant réagir séparément le composé purifié sous sa forme acide avec une base organique ou inorganique et en isolant le sel ainsi formé. Les sels d'addition acide comprennent les sels aminés et métalliques. Les sels métalliques adaptés comprennent les sels de sodium, potassium, calcium, baryum, zinc, magnésium et aluminium. Les sels de sodium et de potassium sont préférés. Les sels d'addition inorganiques de base adaptés sont préparés à partir de bases métalliques qui comprennent hydrure de sodium, hydroxyde de sodium, hydroxyde de potassium, hydroxyde de calcium, hydroxyde d'aluminium, hydroxyde de lithium, hydroxyde de magnésium, hydroxyde de zinc. Les sels d'addition aminés de base adaptés sont préparés à partir d'amines qui ont une alcalinité suffisante pour former un sel stable, et de préférence comprennent les amines qui sont souvent utilisées en chimie médicinale en raison de leur faible toxicité et de leur acceptabilité pour l'usage médical : ammoniac, éthylènediamine, N-méthyl-glucamine, lysine, arginine, ornithine, choline, N,N'-dibenzyléthylènediamine, chloroprocaïne, diéthanolamine, procaïne, N-benzylphénéthylamine, diéthylamine, pipérazine, tris(hydroxymethyl)-aminomethane, hydroxyde de tétraméthylammonium, triéthylamine, dibenzylamine, éphénamine, déhydroabiétylamine, N-éthyl-pipéridine, benzylamine, tétraméthylammonium, tétraéthylammonium, méthylamine, diméthylamine, triméthylamine, éthylamine, acides aminés basiques, par exemple lysine et arginine, et dicyclohexylamine, et analogues.

L'invention se rapporte également aux stéréoisomères ou leurs mélanges, les formes tautomères, les hydrates, solvates, sels et esters pharmaceutiquement acceptables des composés de formule (I).

Les composés de l'invention de formule (I) définis tels que précédemment, possédant une fonction suffisamment acide ou une fonction suffisamment basique ou les deux, peuvent inclure les sels correspondants d'acide organique ou minéral ou de base organique ou minérale pharmaceutiquement acceptables.

Les composés de formule générale (I) peuvent être préparés par application ou adaptation de toute méthode connue en soi de et/ou à la portée de l'homme du métier, notamment celles décrites par Larock dans Comprehensive Organic Transformations, VCH Pub., 1989, ou par application ou adaptation des procédés décrits dans les exemples qui suivent.

Selon un autre objet, la présente invention concerne donc également le procédé de préparation des composés de formule (I) précédemment décrits comprenant l'étape de couplage des composés de formule (II) et (III) correspondants selon le schéma suivant : 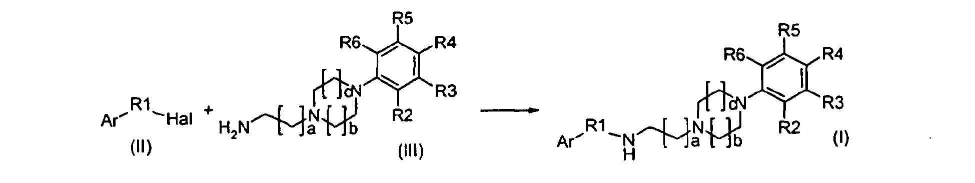 où, dans la formule (II) et (III), Ar, R1, R2, R3, R4, R5, R6, a, b et c ont la même signification que dans la formule (I) et Hal représente un atome d'halogène, de préférence le chlore.

Généralement, la substitution est effectuée en chauffant à 300-350°C ou en chauffant dans un four à micro-ondes. Cette réaction peut également être catalysée par des composés organiques (dérivés du phénol, 4-diméthylaminopyridine, trifluoroéthanol) ou inorganiques (fluorures alcalins ou alcalino terreux, métaux de transition comme le palladium ou le nickel).

Les dérivés de formule (II) dans laquelle R1, de formule 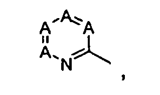 et A représente indépendamment un atome de carbone ou d'azote, représente un motif azine ou diazine et pour lesquels Ar a la même signification que dans la formule (I) peuvent être préparés à partir de leurs N-oxydes de formule (IV) dans laquelle Ar à la même signification que dans la formule (I) et chacun des A représente indépendamment un atome de carbone ou d'azote, selon le schéma suivant :

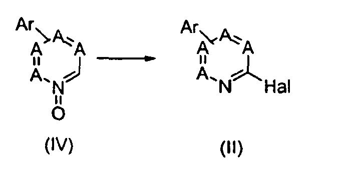

Cette réaction peut être effectuée par réaction sur un agent d'halogénation, notamment chloration comme l'oxychlorure de phosphore, le trichlorure de phosphore, le pentachlorure de phosphore ou le chlorure de sulfonyle, éventuellement en présence d'un chlorure inorganique (chlorure de sodium) dans un solvant organique tel que le toluène ou le chloroforme ou sans solvant à une température comprise entre 0°C et la température d'ébullition du milieu réactionnel.

Les N-oxydes d'azine ou de diazine de formule (IV) dans laquelle Ar a la même signification que dans la formule (I) et chacun des A représente indépendamment un atome de carbone ou d'azote peuvent être obtenus par couplage entre un arylboronique et un N-oxyde d'halogénoazine ou d'halogénodiazine (V) dans laquelle Ar à la même signification que dans la formule (I) et chacun des A représente indépendamment un atome de carbone ou d'azote :

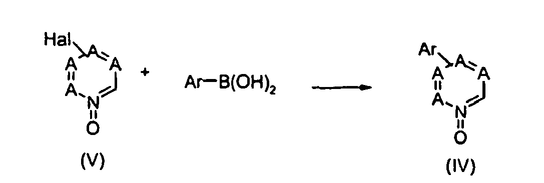

Cette réaction peut être effectuée en présence d'une quantité catalytique d'un métal de transition tel que le palladium.

Les dérivés de formule (I) dans laquelle R1 représente un motif azine ou diazine peuvent également être préparés par réduction de leurs N-oxydes de formule (VI) dans laquelle Ar, R2, R3, R4, R5, R6, a, b et c ont la même signification que dans la formule (I) et chacun des A représente indépendamment un atome de carbone ou d'azote

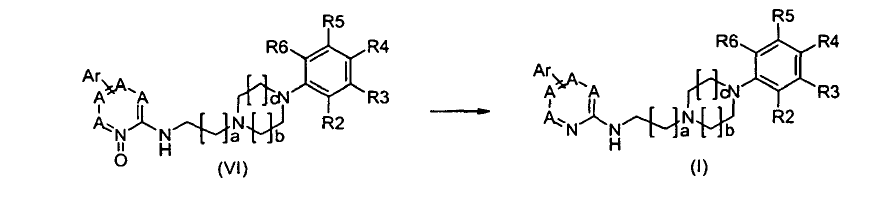

La réduction de la fonction N-oxyde peut être réalisée à l'aide de trichlorure de phosphore dans le chloroforme à température ambiante, par des phosphines comme la triphénylphosphine dans le toluène, par hydrogénation à l'aide de dihydrogène ou par transfert (acide formique, formiate, cyclohexène, cyclohexadiène) en présence d'un métal de transition (palladium sur charbon) dans un solvant organique comme le toluène ou un alcool.

Les N-oxydes de formule (VI) dans laquelle Ar, R2, R3, R4, R5, R6, a, b et c ont la même signification que dans la formule (I) et chacun des A représente indépendamment un atome de carbone ou d'azote peuvent être préparés à partir des N-oxydes d'halogénoazine ou N-oxydes d'halogénodiazine de formule (VII), notamment N-oxydes de chloroazine ou N-oxydes de chlorodiazine, dans laquelle Ar a la même signification que dans la formule (I) et chacun des A représente indépendamment un atome de carbone ou d'azote et des amines de formule (III) dans laquelle R2, R3, R4, R5, R6, a, b et c ont la même signification que dans la formule (I).

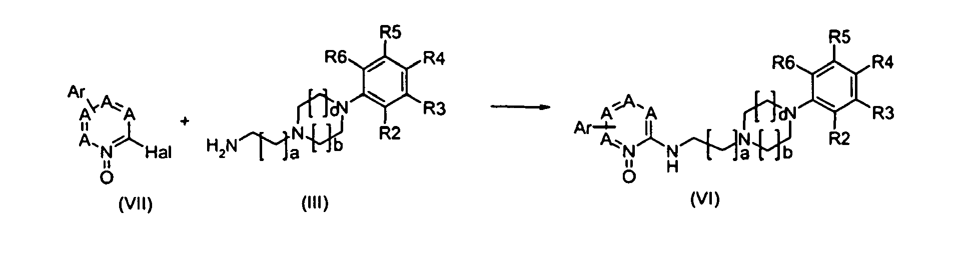

La substitution des dérivés de N-oxyde d'halogénoazine ou N-oxyde d'halogénodiazine de formule (VII) peut être effectuée en présence d'une base telle que l'hydrogénocarbonate de sodium par exemple dans l'alcool tert-amylique au reflux pendant une nuit. Cette réaction peut également être réalisée en chauffant à 300-350°C ou en chauffant dans un four à micro-ondes. Cette réaction peut aussi être catalysée par des composés organiques (dérivés du phénol, 4-diméthylaminopyridine, trifluoroéthanol) ou inorganiques (fluorures alcalins ou alcalino-terreux, métaux de transition comme le palladium ou le nickel).

Les N-oxydes d'halogénoazine ou N-oxydes d'halogénodiazine de formule (VII) dans laquelle Ar a la même signification que dans la formule (I) et chacun des A représente indépendamment un atome de carbone ou d'azote peuvent être obtenus par oxydation des halogénohétérocycles de formule (II) dans laquelle R1 représente un motif azine ou diazine et pour lesquels Ar a la même signification que dans la formule (I) et chacun des A représente indépendamment un atome de carbone ou d'azote

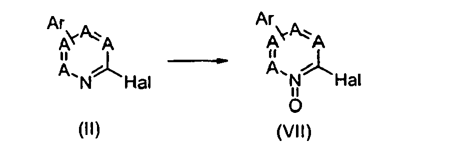

Cette oxydation peut être effectuée par l'acide méta-chloroperbenzoïque dans le chloroforme à température ambiante ou à chaud. Il peut être effectué aussi par d'autres agents oxydants tels que l'eau oxygénée en présence d'acide acétique, d'acide formique, d'acide trifluoroacétique, d'anhydride acétique ou d'anhydride trifluoroacétique ; par le complexe urée-eau oxygénée dans le dichlorométhane ou dans l'acide formique par exemple et éventuellement en présence de sels métalliques tels que des oxydes de rhénium ou de tungstène.

Les chlorohétérocycles de formule (II) dans laquelle R1 représente un motif azine ou diazine et pour lesquels Ar a la même signification que dans la formule (I) et chacun des A représente indépendamment un atome de carbone ou d'azote peuvent être préparées à partir des N-oxydes de formule (IV) dans laquelle Ar à la même signification que dans la formule (I) et chacun des A représente indépendamment un atome de carbone ou d'azote comme décrit plus haut. Ils peuvent également être préparées par couplage d'un arylboronique avec une halogénoazine ou une halogénodiazine de formule (VIII) dans laquelle Ar à la même signification que dans la formule (I), chacun des A représente indépendamment un atome de carbone ou d'azote et Hal' représente un brome, un iode ou un pseudohalogène (paratoluènesulfonate, mésylate, triflate) :

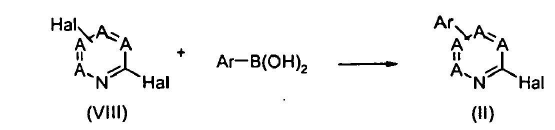

Cette réaction peut s'effectuer en présence d'une quantité catalytique d'un métal de transition tel que le palladium, en présence d'une base telle que le carbonate de sodium dans un solvant tel que le tétrahydrofurane ou le toluène à une température comprise entre 0°C et la température d'ébullition du milieu réactionnel.

Les amines de formule (III) dans laquelle R2, R3, R4, R5, R6, a, b et c ont la même signification que dans la formule (I) peuvent être préparées par réduction des nitriles de formule (IX) dans laquelle R2, R3, R4, R5, R6, a, b et c ont la même signification que dans la formule (I)

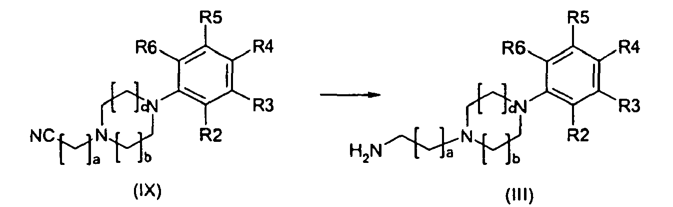

Cette réduction peut être réalisée par le dihydrogène en présence d'un métal de transition (nickel de Raney ou palladium par exemple) ou par un hydrure (hydrure double de lithium et d'aluminium par exemple).

Les nitriles de formule (IX) dans laquelle R2, R3, R4, R5, R6, a, b et c ont la même signification que dans la formule (I) peuvent être préparés à partir des amines cycliques (X) dans laquelle R2, R3, R4, R5, R6, a, b et c ont la même signification que dans la formule (I) 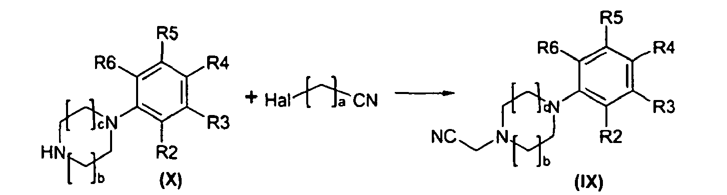 où Hal représente un atome d'halogène, de préférence le brome.

Cette réaction peut être réalisée en présence d'une base inorganique (carbonate de potassium) et en présence ou non d'une quantité catalytique d'iodure de potassium dans l'acétonitrile au reflux.

Les amines de formule (III) dans laquelle R2, R3, R4, R5, R6, a, b et c ont la même signification que dans la formule (I) peuvent également être préparées à partir des dérivés du phtalimide (XI) dans laquelle R2, R3, R4, R5, R6, a, b et c ont la même signification que dans la formule (I)

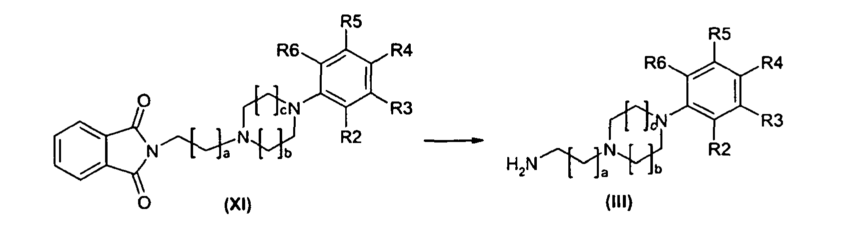

La déprotection de la fonction phtalimide peut être effectuée avec de l'hydrazine monohydratée ou la méthylhydrazine dans un alcool (méthanol ou éthanol) à une température comprise entre 0°C et 40°C ou encore en utilisant les méthodes décrites ou citées par J. O. Osby, M. G. Martin et B. Ganem, Tetrahedron Lett. 25(20) 2093-2096 (1984).

Les dérivés du phtalimide (XI) dans laquelle R2, R3, R4, R5, R6, a, b et c ont la même signification que dans la formule (I) peuvent être obtenus à partir des dérivés d'amine cyclique (X) dans laquelle R2, R3, R4, R5, R6, b et c ont la même signification que dans la formule (I) 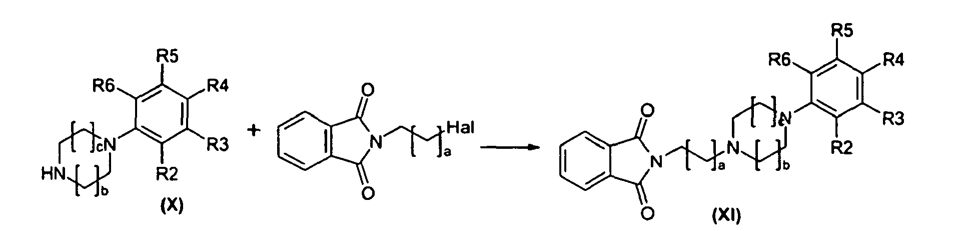 où Hal représente un atome d'halogène, de préférence le brome.

Cette alkylation peut être réalisée avec le N-(ω-bromoalkyl)phtalimide en présence d'une base inorganique comme un carbonate ou un hydrogénocarbonate, en présence ou non d'iodure de potassium et dans un solvant organique comme l'acétonitrile, le N,N-diméthylformamide, le diméthylsulfoxyde, le N,N-diméthylacétamide ou le toluène à une température comprise entre 20°C et la température d'ébullition du milieu réactionnel.

Les amines cycliques de formule (X) dans laquelle R2, R3, R4, R5, R6, b et c ont la même signification que dans la formule (I) peuvent être préparés par dialkylation d'anilines de formule (XII) dans laquelle R2, R3, R4, R5, R6, b et c ont la même signification que dans la formule (I) : 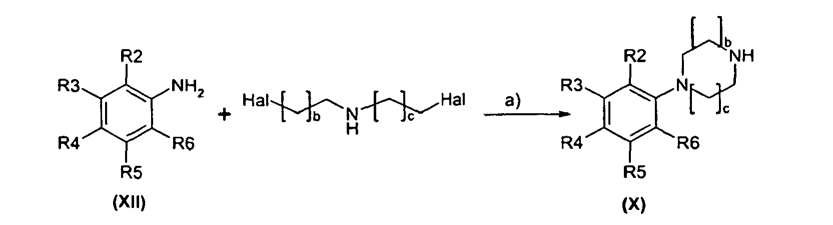 où Hal représente un atome d'halogène, de préférence le chlore.

Cette réaction peut être réalisée par chauffage dans un solvant aromatique.

Les amines cycliques de formule (X) dans laquelle R2, R3, R4, R5, R6, b et c ont la même signification que dans la formule (I) peuvent également être préparés par substitution d'halogénoaromatiques, de préférence fluoro-aromatiques de formule (XIII) dans laquelle R2, R3, R4, R5 et R6 ont la même signification que dans la formule (I) lorsque ce dernier porte un groupement électroattracteur avec une diamine de formule (XIV) dans laquelle b et c ont la même signification que dans la formule (I) 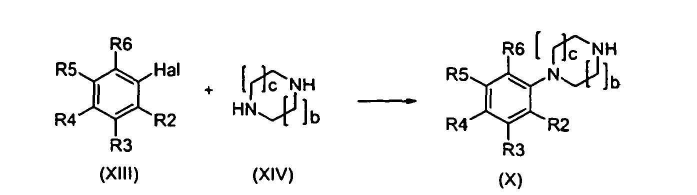 où Hal représente un atome d'halogène, de préférence le fluor.

Généralement, cette réaction est réalisée par chauffage dans un solvant organique.

Les amines cycliques de formule (X) peuvent également être préparées par substitution d'halogénoaromatique, l'halogène étant de préférence un iode, un brome ou un chlore ou d'un pseudohalogénoaromatique, le pseudohalogène étant un arylsulfonate ou un alkylsulfonate (paratoluènesulfonate, mésylate, triflate par exemple) de formule (XIV).

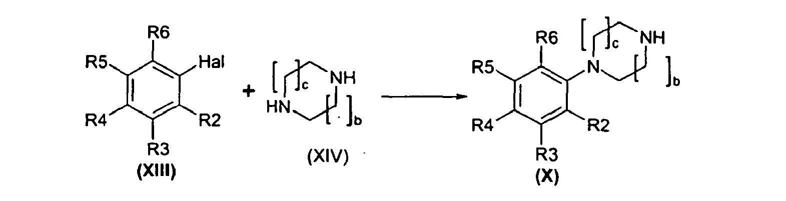

Cette réaction peut éventuellement être catalysée par des métaux de transition.

Les dérivés de formule (I) peuvent également être préparés à partir des amines de formule (III) dans laquelle R2, R3, R4, R5, R6, a, b et c ont la même signification que dans la formule (I) par construction de la partie hétérocyclique.

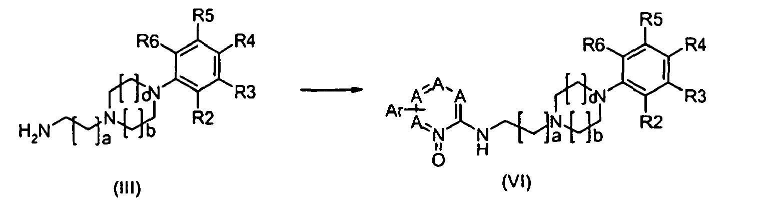

Ces réactions de construction d'hétérocycles peuvent être réalisées selon les méthodes décrites dans Bull. Soc. Chim. Fr. 1163 (1956), J. Med. Chem. 1158 (1983), J. Het. Chem. 1377 (1979) J. Prakt. Chem. 249 (1979) Zh. Ob. Khim. (Engl. Tr.) 2129 (1979), J. Org. Chem. 3736 (1974), J. Het. Chem. 873 (1976), Carbohydrate Res. 307 (1987), J. Amer. Chem. Soc. 2292 (1994), Ber 2110 (1966), Rec. Trav. Chim. Pays Bas 463 (1942), J. Org. Chem. 2069 (1981), Org. Lett. 2091 (2002), Phos. Sulf. Sil. Rel. El. 81 (1991), Bioorg. Med. Chem. Lett. 3305 (2002), Bioorg. Med. Chem. Lett. 1345 (2003), Bioorg. Med. Chem. Lett. 3557 (2003), Bull. Chem. Soc. Japan 2450 (1984), Heterocycles 149 (1995), J. Med. Chem. 3977 1994, Helv. Chim. Acta 1981 (1999), Tetrahedron Lett. 7825 (2003), Pharm. Pharmacol. Commun. 31 (2000), J. Het. Chem. 191 (2003), J. Het. Chem. 121 (2003), Bioorg. Med. Chem. 769 (2003), Farmaco 577 (2002).

Eventuellement, ledit procédé peut également comprendre l'étape consistant à isoler le produit obtenu.

Dans les réactions décrites ici, il peut être nécessaire de protéger les groupes fonctionnels réactifs, par exemples les groupes hydroxy, amino, imino, thio, carboxy, lorsqu'ils sont souhaités dans le produit final, pour éviter leur participation indésirable dans les réactions. Les groupes de protection traditionnels peuvent être utilisés conformément à la pratique standard, pour des exemples voir T.W. Greene et P.G.M. Wuts dans Protective Groups in Organic Chemistry, John Wiley and Sons, 1991 ; J.F.W. McOmie in Protective Groups in Organic Chemistry, Plenum Press, 1973.

Le composé ainsi préparé peut être récupéré à partir du mélange de la réaction par les moyens traditionnels. Par exemple, les composés peuvent être récupérés en distillant le solvant du mélange de la réaction ou si nécessaire après distillation du solvant du mélange de la solution, en versant le reste dans de l'eau suivi par une extraction avec un solvant organique immiscible dans l'eau, et en distillant le solvant de l'extrait. En outre, le produit peut, si on le souhaite, être encore purifié par diverses techniques, telles que la recristallisation, la reprécipitation ou les diverses techniques de chromatographie, notamment la chromatographie sur colonne ou la chromatographie en couche mince préparative.

Il sera apprécié que les composés utiles selon la présente invention puissent contenir des centres asymétriques. Ces centres asymétriques peuvent être indépendamment en configuration R ou S. Il apparaîtra à l'homme du métier que certains composés utiles selon l'invention peuvent également présenter une isomérie géométrique. On doit comprendre que la présente invention comprend des isomères géométriques individuels et des stéréoisomères et des mélanges de ceux-ci, incluant des mélanges racémiques, de composés de formule (I) ci-dessus. Ce type d'isomères peuvent être séparés de leurs mélanges, par l'application ou l'adaptation de procédés connus, par exemple des techniques de chromatographie ou des techniques de recristallisation, ou ils sont préparés séparément à partir des isomères appropriés de leurs intermédiaires.

Aux fins de ce texte, il est entendu que les formes tautomériques sont comprises dans la citation d'un groupe donné, par exemple thio/mercapto ou oxo/hydroxy.

Les sels d'additions acides sont formés avec les composés utiles selon l'invention dans lesquels une fonction de base tels qu'un groupe amino, alkylamino ou dialkylamino est présente. Les sels d'addition acide pharmaceutiquement acceptables, c'est-à-dire non toxiques, sont préférés. Les sels sélectionnés sont choisis de façon optimale pour être compatibles avec les véhicules pharmaceutiques habituels et adaptés pour l'administration orale ou parentérale. Les sels d'addition acide des composés utiles selon cette invention peuvent être préparés par réaction de la base libre avec l'acide approprié, par l'application ou l'adaptation de procédés connus. Par exemple, les sels d'addition acide des composés utiles selon cette invention peuvent être préparés soit en dissolvant la base libre dans de l'eau ou dans une solution aqueuse alcoolisée ou des solvants adaptés contenant l'acide approprié et en isolant le sel en évaporant la solution, ou en faisant réagir la base libre et l'acide dans un solvant organique, auquel cas le sel se sépare directement ou peut être obtenu par concentration de la solution. Parmi les acides adaptés pour l'usage dans la préparation de ces sels on trouve acide chlorhydrique, acide bromhydrique, acide phosphorique, acide sulfurique, divers acides carboxyliques et sulfoniques organiques, tels que acide acétique, acide citrique, acide propionique, acide succinique, acide benzoïque, acide tartrique, acide fumarique, acide mandélique, acide ascorbique, acide malique, acide méthanesulfonique, acide toluène-sulfonique, acides gras, adipate, alginate, ascorbate, aspartate, benzènesulfonate, benzoate, propionate de cyclopentane, digluconate, dodécylsulfate, bisulfate, butyrate, lactate, laurate, sulfate de lauryle, malate, hydroiodide, 2-hydroxyéthanesulfonate, glycéro-phosphate, picrate, pivalate, pamoate, pectinate, persulfate, 3-phénylpropionate, thiocyanate, 2-naphtalènesulfonate, undécanoate, nicotinate, hémisulfate, heptonate, hexanoate, camphorate, camphresulfonate et autres.

Les sels d'addition acide des composés utiles selon cette invention peuvent être régénérés à partir des sels par l'application ou l'adaptation de procédés connus. Par exemple, les composés parents utiles selon l'invention peuvent être régénérés à partir de leurs sels d'addition acide par traitement avec un alkali, par exemple une solution de bicarbonate de sodium aqueuse ou une solution d'ammoniac aqueuse.

Les composés utiles selon cette invention peuvent être régénérés à partir de leurs sels d'addition de base par l'application ou l'adaptation de procédés connus. Par exemple, les composés parents utiles selon l'invention peuvent être régénérés à partir de leurs sels d'addition de base par le traitement avec un acide, par exemple un acide chlorhydrique.

Les sels d'addition de base peuvent être formés lorsque le composé utile selon l'invention contient un groupe carboxyle, ou un bioisostère suffisamment acide. Les bases qui peuvent être utilisées pour préparer les sels d'addition de base comprennent de préférence celle qui produisent, lorsqu'elles sont associées à un acide libre, des sels pharmaceutiquement acceptables, c'est-à-dire des sels dont les cations ne sont pas toxiques pour le patient dans les doses pharmaceutiques des sels, de sorte que les effets inhibiteurs bénéfiques inhérents à la base libre ne soient pas annulés par les effets secondaires imputables aux cations. Les sels pharmaceutiquement acceptables, comprenant ceux dérivés des sels de métal alcalino-terreux, dans la portée de l'invention comprennent ceux dérivés des bases suivantes : hydrure de sodium, hydroxyde de sodium, hydroxyde de potassium, hydroxyde de calcium, hydroxyde d'aluminium, hydroxyde de lithium, hydroxyde de magnésium, hydroxyde de zinc, ammoniac, éthylènediamine, N-méthyl-glucamine, lysine, arginine, ornithine, choline, N,N'-dibenzyléthylènediamine, chloroprocaïne, diéthanolamine, procaïne, N-benzylphénéthylamine, diéthylamine, pipérazine, tris(hydroxy-méthyl)aminométhane, hydroxyde de tétraméthylammonium et analogues.

Les composés utiles selon la présente invention peuvent être facilement préparés, ou formés pendant le processus de l'invention, sous forme de solvates (par exemple hydrates). Les hydrates des composés utiles selon la présente invention peuvent être facilement préparés par la recristallisation d'un mélange de solvant aqueux/organique, en utilisant des solvants organiques tels que dioxane, tétrahydrofurane ou méthanol.

Les produits de bases ou les réactifs utilisés sont disponibles commercialement et/ou peuvent être préparés par l'application ou l'adaptation de procédés connus, par exemple des procédés tels que décrits dans les Exemples de Référence ou leurs équivalents chimiques évidents.

Selon la présente invention, les composés de formule (I) présentent une activité de ligand sélectif du récepteur D3.

La présente invention a également pour objet les compositions pharmaceutiques comprenant un composé selon l'invention avec un véhicule ou excipient pharmaceutiquement acceptable.

De préférence, ladite composition contient une quantité efficace du composé selon l'invention.

Selon un autre objet, la présente invention concerne également l'utilisation d'un composé de formule générale (I) : 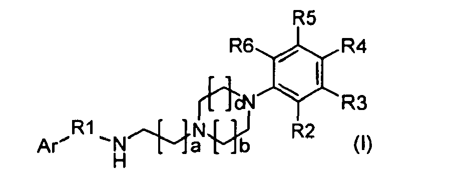 dans laquelle :
■ R1 représente un hétéroaryle à cinq ou six chaînons, contenant un ou plusieurs hétéroatomes, choisi parmi 2-pyridyle, 2-pyrimidinyle, 2-pyridazinyle, 2-pyrazinyle, 2-imidazolyle, 2-oxazolyle, 2-thiazolyle, 3-isoxazolyle, 3-isothiazolyle, 1,2,4-triazol-2-yle, 1,3,4-oxàdiàzol-2-yle, 1,3,4-thiadiazol-2-yle, éventuellement substitué par un ou plusieurs groupements identiques ou différents choisis parmi un atome d'halogène ou un groupe hydroxy, alkyle, monofluoroalkyle, polyfluoroalkyle, alkoxy, polyfluoro-alkoxy, alkylsulfanyle, polyfluoroalkylsulfanyle ;
■ Ar est un aryle ou hétéroaryle, éventuellement substitué par un ou plusieurs substituants identiques ou différents choisis parmi un atome d'halogène ou un groupe hydroxy, alkyle, monofluoroalkyle, polyfluoroalkyle, alkoxy, polyfluoroalkoxy, alkylsulfanyle, polyfluoroalkylsulfanyle, cyano, carbamoyle, dialkylcarbamoyle, Alkyl-C(=O)-, Alkyl-O-C(=O)-, HO-C(=O)-, (HO)Alkyl-, ou Ar est un cycle hydrocarboné ou hétérocycle, saturé, insaturé ou aromatique;
■ a = 2, 3 ou 4 ;
■ b et c identiques ou différents représentent 1 ou 2 ;
■ R2, R3, R4, R5 et R6 représentent chacun indépendamment un atome d'hydrogène ou d'halogène ou un groupement hydroxy, alkyle, monofluoroalkyle, polyfluoroalkyle, alkoxy, polyfluoroalkoxy, alkylsulfanyle, polyfluoroalkylsulfanyle, cyano, -NRR', -COOR, -COR, -CONRR' ou bien deux des R2, R3, R4, R5 et R6 adjacents sont reliés entre eux pour former un cycle hydrocarboné ou un hétérocycle saturé ou insaturé, fusionné au noyau phényle auquel ils sont rattachés ;
où R, R' identiques ou différents représentent indépendamment un atome d'hydrogène, ou un groupe alkyle ;
ainsi que leurs stéréoisomères ou leur mélanges, leurs formes tautomères, leurs hydrates, solvates, leurs sels et esters pharmaceutiquement acceptables,
pour la préparation de compositions pharmaceutiques destinées à agir comme ligand du récepteur D3 de la dopamine.

De préférence, ledit ligand est un antagoniste de D3 ; encore plus préférentiellement un antagoniste sélectif de D3.

Selon un autre objet, la présente invention concerne également l'utilisation de composés de formule générale (I) pour la préparation de compositions pharmaceutiques destinées à prévenir et/ou traiter une affection neuro-psychiatrique ou toute affection thérapeutique faisant intervenir le récepteur D3 de la dopamine. Lesdites affections sont choisies de préférence parmi la pharmacodépendance, les troubles de la sphère sexuelle, les troubles moteurs, la maladie de Parkinson, la psychose ou état psychotique, la dépression ou pharmacodépendance.

Selon l'invention, on entend par pharmacodépendance tout état lié à la désaccoutumance, l'abstinence et/ou à la désintoxication d'un sujet dépendant de tout agent, notamment les agents actifs thérapeutiques ; tels que les

Selon l'invention, on entend par pharmacodépendance tout état lié à la désaccoutumance, l'abstinence et/ou à la désintoxication d'un sujet dépendant de tout agent, notamment les agents actifs thérapeutiques; tels que les morphiniques, et/ou drogues telles que la cocaïne, l'héroïne, ou encore l'alcool et/ou la nicotine.

Selon l'invention, on entend notamment par troubles de la sphère sexuelle, l'impuissance, notamment l'impuissance masculine.

Selon l'invention, ladite prévention et/ou traitement de la maladie de Parkinson est de préférence un traitement complémentaire de la maladie de Parkinson.

Selon l'invention, on entend notamment par troubles moteurs les dyskinésies essentielles ou iatrogènes, et/ou les tremblements essentiels ou iatrogènes.

Selon un autre objet, la présente invention concerne également les méthodes de traitement thérapeutiques citées ci-dessus comprenant l'administration d'un composé selon l'invention à un patient qui en a besoin.

De préférence, ladite composition est administrée à un patient qui en a besoin.

Les compositions pharmaceutiques selon l'invention peuvent être présentées sous des formes destinées à l'administration par voie parentérale, orale, rectale, permuqueuse ou percutanée.

Elles seront donc présentées sous forme de solutés ou de suspensions injectables ou flacons multi-doses, sous forme de comprimés nus ou enrobés, de dragées, de capsules, de gélules, de pilules, de cachets, de poudres, de suppositoires ou de capsules rectales, de solutions ou de suspensions, pour l'usage percutané dans un solvant polaire, pour l'usage permuqueux.

Les excipients qui conviennent pour de telles administrations sont les dérivés de la cellulose ou de la cellulose microcristalline, les carbonates alcalino-terreux, le phosphate de magnésium, les amidons, les amidons modifiés, le lactose pour les formes solides.

Pour l'usage rectal, le beurre de cacao ou les stéarates de polyéthylèneglycol sont les excipients préférés.

Pour l'usage parentéral, l'eau, les solutés aqueux, le sérum physiologique, les solutés isotoniques sont les véhicules les plus commodément utilisés.

La posologie peut varier dans les limites importantes (0,5 mg à 1000 mg) en fonction de l'indication thérapeutique et de la voie d'administration, ainsi que de l'âge et du poids du sujet.

Les exemples suivants illustrent l'invention, sans toutefois la limiter. Les produits de départs utilisés sont des produits connus ou préparés selon des modes opératoires connus.

Les pourcentages sont exprimés en poids, sauf mention contraire.

### EXEMPLES

### Exemple 1 : préparation de la 2-{4-[4-(2-fluorophényl)pipérazin-1-yl]butyl}amino-5-phénylpyridine

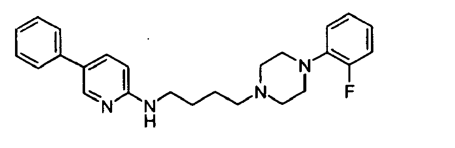

### Etape a : préparation du 4-[4-(2-fluorophényl)pipérazin-1-yl]butyronitrile

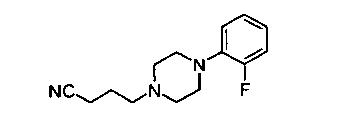

A une solution de 7,2 g (40 mmol) de 1-(2-fluorophényl)pipérazine dans 100 mL d'acétonitrile on ajoute successivement une pointe de spatule d'iodure de potassium, 5,5 g (40 mmol) de carbonate de potassium et de 6,0 g (40 mmol) de 4-bromobutyronitrile. La suspension est portée au reflux pendant une nuit.

Le milieu réactionnel est concentré sous vide, et le résidu est repris avec de l'oxyde de diéthyle et de l'eau. Après séparation de la phase aqueuse, la phase organique est séchée sur sulfate de magnésium, filtrée et concentrée sous vide.

On obtient ainsi 9,25 g (93%) de 4-[4-(2-fluorophényl)pipérazin-1-yl]butyronitrile sous forme d'huile visqueuse utilisée telle quelle dans les synthèses ultérieures.

### Etape b : préparation de la 4-[4-(2-fluorophényl)pipérazin-1-yl]butylamine

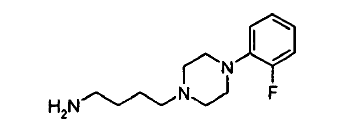

A une solution de 9,25 g (37,4 mmol) de 4-[4-(2-fluorophényl)pipérazin-1-yl]butyronitrile obtenu précédemment, dans un mélange de 50 mL d'une solution aqueuse d'ammoniaque concentré et 50 mL d'une solution d'éthanol ammoniacal environ 8N, on ajoute environ 1 g de nickel de Raney préalablement lavé avec de l'éthanol.

La suspension est mise à hydrogéner sous 3 bars d'hydrogène à 30°C pendant 3 heures.

Le mélange est filtré sur célite, rincé avec de l'éthanol et concentré sous vide. Le résidu huileux est repris avec 50 mL d'éthanol et concentré. Cette opération est renouvelée une fois.

On obtient ainsi 8,8 g (94%) de 4-[4-(2-fluorophényl)pipérazin-1-yl]butylamine sous forme d'huile visqueuse utilisée telle quelle dans les synthèses ultérieures.

### Etape c : préparation de la 2-chloro-5-phénylpyridine

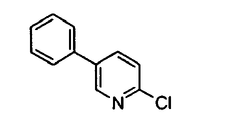

Une solution de 0,7 g (5,7 mmol) d'acide phénylboronique dans 3 mL d'éthanol est ajoutée à un mélange de 0,18 g (0,15 mmol) de tetrakis(triphénylphosphine)palladium, de 1 g (5,15 mmol) de 5-bromo-2-chloropyridine, de 5,7 mL d'une solution aqueuse de carbonate de sodium 2M et de 10 mL de toluène. Le mélange est agité vigoureusement et chauffé à 80°C pendant 90 minutes.

Après retour à température ambiante, le milieu réactionnel est extrait avec 20 mL d'acétate d'éthyle puis lavé avec 10 mL d'eau. La phase organique est séchée sur sulfate de magnésium, filtrée et concentrée sous vide. L'huile marron obtenue est purifiée par chromatographie sur gel de silice (élution avec un gradient d'acétate d'éthyle/heptane) pour conduire à 0,7 g (70%) de 2-chloro-5-phénylpyridine sous forme de cristaux blancs.

Rf = 0,6 heptane/acétate d'éthyle 2/1

RMN ¹H (DMSO d₆) : 8,7 (multiplet, 1 H) ; 8,1 (multiplet, 1H); 7,8 à 7,65 (massif, 2H) ; 7,6 (multiplet, 1 H) ; 7,55 à 7,35 (massif, 3H)

### Etape d : préparation de la 2-{4-[4-(2-fluorophényl)pipérazin-1-yl]butyl}amino-5-phénylpyridine

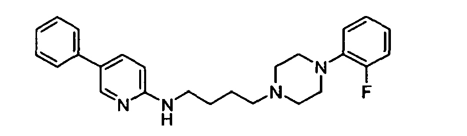

Dans un tube à essai, 0,189 g (1,0 mmol) de 2-chloro-5-phénylpyridine obtenue précédemment et 0,25 g (1,0 mmol) de 4-[4-(2-fluorophényl)pipérazin-1-yl]butylamine (exemple 1 étape b) sont chauffés pendant une minute dans un four à micro-ondes. Le mélange est dilué avec 3 mL de dichlorométhane et chromatographié sur gel de silice (éluant dichlorométhane/éthanol 98/2).

Après concentration des fractions d'élution, le produit cristallise. Après trituration dans de l'oxyde de diisopropyle, filtration et séchage, on obtient 20 mg de 2-{4-[4-(2-fluorophényl)pipérazin-1-yl]butyl}amino-5-phénylpyridine sous forme de solide crème.

Point de fusion : 95°C

RMN ¹H (CDCl₃) : 8,35 (singulet, 1 H) ; 7,65 (doublet, 1 H) ; 7,6 à 7,35 (massif, 4H) ; 7,3 (multiplet, 1 H) ; 7,15 à 6,85 (massif, 4H) ; 6,45 (doublet, 1H) ; 4,95 à 4,7 (massif large, 1 H) ; 3,35 (triplet large, 2H) ; 3,2 à 3,0 (massif, 4H) ; 2,85 à 2,65 (massif, 4H) ; 2,5 (triplet, 2H) ; 1,85 à 2,5 (massif, 4H)

### Exemple 2 : 2-{4-[4-(2-fluorophényl)pipérazin-1-yl]butyl}amino-4-phénylpyridine

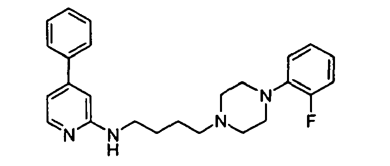

La {2-{4-[4-(2-fluorophényl)pipérazin-1-yl]butyl}amino-4-phénylpyridine est préparée par réaction de la 2-chloro-4-phénylpyridine (préparation décrite dans EP 036 638) et de la 4-[4-(2-fluorophényl)pipérazin1-yl]butylamine (exemple 1 étape b) selon le même mode opératoire que celui décrit dans l'exemple 1, étape d.

Rendement : 10% (huile rouge)

RMN ¹H : (CDCl₃) : 8,1 (doublet, 1H) ; 7,7 à 7,5 (massif, 2H) ; 7,5 à 7,3 (massif, 3H) ; 7,1 à 6,85 (massif, 4H) ; 6,8 (multiplet, 1 H) ; 6,55 (singulet, 1 H) ; 4,8 (singulet large, 1H) ; 3,4 (triplet large, 2H) ; 3,25 à 3,0 (massif, 4H) ; 3,1 à 2,55 (massif, 4H) ; 2,5 (triplet, 2H) ; 1,8 à 2,55 (massif, 4H)

### Exemple 3 : 2-{4-[4-(3-trifluorométhylphényl)pipérazin-1-yl]butyl}amino-4-phénylpyridine

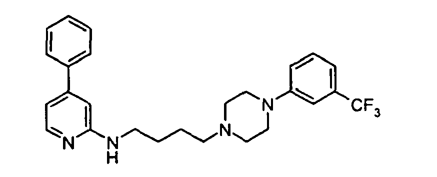

### Etape a : préparation du 4-[4-(3-trifluorométhylphényl)pipérazin-1-yl]butyronitrile

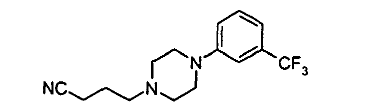

Un mélange de 12,02 g (52 mmol) de 1-(3-trifluorométhylphényl)pipérazine, de 7,9 g (57 mmol) de carbonate de potassium, de 8,5 g (57 mmol) de 4-bromobutyronitrile et de 120 mL d'acétonitrile est porté au reflux pendant une nuit.

Le milieu réactionnel est concentré, repris avec de d'acétate d'éthyle et lavé avec de l'eau. Après séparation de la phase aqueuse, la phase organique est séchée sur sulfate de magnésium, filtrée et concentrée sous vide. On obtient ainsi 15 g (97%) de 4-[4-(3-trifluorométhylphényl)pipérazin-1-yl]butyronitrile sous forme d'une huile visqueuse utilisée telle quelle dans les synthèses ultérieures.

### Etape b : préparation de la 4-[4-(3-trifluorométhylphényl)pipérazin-1-yl]butylamine

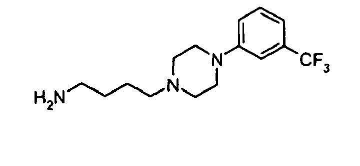

La 4-[4-(3-trifluorométhylphényl)pipérazin-1-yl]butylamine peut être obtenue par réduction du 4-[4-(3-trifluorométhylphényl)pipérazin-1-yl]butyronitrile selon le même mode opératoire que celui utilisé dans l'exemple 1, étape b.

Rendement : 92% (huile visqueuse)

Le produit est engagé brut dans l'étape suivante.

### Etape c : préparation de la 2-{4-[4-(3-trifluorométhylphényl)pipérazin-1-yl]butyl}amino-4-phénylpyridine

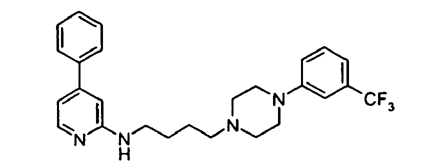

Dans un tube à essai, 0,38 g (2,0 mmol) de 2-chloro-4-phénylpyridine, 0,6 g (2,0 mmol) de 4-[4-(3-trifluorométhylphényl)pipérazin-1-yl]butylamine et une pointe de spatule de 4-diméthylaminopyridine sont chauffés pendant 3 minutes à environ 300-350°C. Le mélange est dilué avec de l'acétate d'éthyle et chromatographié sur gel de silice (éluant dichlorométhane/éthanol 90/10).

Après concentration des fractions d'élution, le produit cristallise. Après trituration dans de l'oxyde de diéthyle, filtration et séchage, on obtient 80 mg de 2-{4-[4-(3-trifluorométhylphényl)pipérazin-1-yl]butyl}amino-4-phénylpyridine sous forme de solide blanc.

Point de fusion : 120°C (tube)

RMN ¹H (CDCl₃) : 8,1 (doublet, 1H) ; 7,65 à 7,55 (massif, 2H) ; 7,5 à 7,25 (massif, 4H) ; 7,2 à 7,0 (massif, 3H) ; 6,8 (doublet, 1 H) ; 6,55 (singulet, 1 H) ; 4,8 (triplet large, 1H) ; 3,35 (multiplet, 2H) ; 3,35 à 3,15 (massif, 4H) ; 2,7 à 2,55 (massif, 4H) ; 2,45 (triplet, 2H) ; 1,85 à 1,65 (massif, 4H)

### Exemple 4 : préparation du dichlorhydrate de 2-{4-[4-(3-trifluorométhyl-phényl)pipérazin-1-yl]butyl}amino-4-phénylpyridine

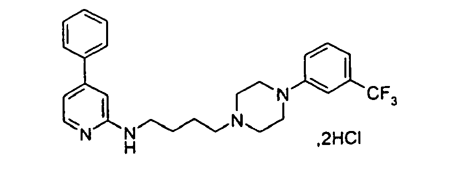

A une solution de 0,15 g (0,33 mmol) de 2-{4-[4-(3-trifluorométhylphényl)pipérazin-1-yl]butyl}amino-4-phénylpyridine (exemple 3) dans 5 mL d'acétone est ajouté, à température ambiante, 0,5 mL d'une solution saturée de chlorure d'hydrogène dans l'oxyde de diéthyle. La précipitation est immédiate. La suspension est agitée pendant 15 minutes, filtrée et séchée sous vide jusqu'à masse constante. On obtient ainsi 0,12 g de dichlorhydrate de 2-{4-[4-(3-trifluorométhylphényl)pipérazin-1-yl]butyl}amino-4-phénylpyridine sous forme de solide blanc.

Point de fusion : 217°C

RMN ¹H (DMSO d₆): 13,8 (singulet large, 1H); 11,0 (singulet large, 1H); 9,1 (singulet large, 1 H) ; 7,95 (doublet, 1 H) ; 7,9 à 7,7 (massif, 2H) ; 7,6 à 7,5 (massif, 3H) ; 7,5 à 7,3 (massif, 2H) ; 7,3 à 1,05 (massif, 4H) ; 3,95 (triplet large, 2H) ; 3,7 à 3,55 (massif, 4H) ; 3,55 à 2,9 (massif, 6H) ; 2,0 à 1,8 (massif, 2H) ; 1,8 à 1,5 (massif, 2H)

### Exemple 5 : préparation de la 2-{4-[4-(3-trifluorométhylphényl)pipérazin-1-yl]butyl}amino-5-phénylpyridine

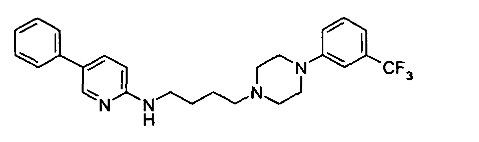

La 2-{4-[4-(3-trifluorométhylphényl)pipérazin-1-yl]butyl}amino-5-phénylpyridine peut être obtenue par réaction de la 2-chloro-5-phénylpyridine (exemple 1, étape c) avec la 4-[4-(3-trifluorométhylphényl)pipérazin-1-yl]butylamine (exemple 3, étape b) selon le même mode opératoire que celui décrit dans l'exemple 3, étape c.

Rendement : 12%

Point de fusion : 115°C (tube)

RMN ¹H (DMSO d₆) : 8,25 (singulet, 1 H) ; 7,65 (doublet, 1 H) ; 7,55 à 7,4 (massif, 2H) ; 7,45 à 7,3 (massif, 3H) ; 7,3 à 7,15 (massif, 2H) ; 7,1 (singulet, 1H) ; 7,0 (doublet, 1 H) ; 6,65 (triplet, 1 H) ; 6,5 (doublet, 1 H) ; 3,3 (triplet large, 2H) ; 3,2 à 3,05 (massif, 4H) ; 2,55 à 2,4 (massif, 4H) ; 2,3 (triplet large, 2H) ; 1,65 à 1,4 (massif, 4H)

### Exemple 6 : préparation de la 2-{4-[4-(2,3-dichlorophényl)pipérazin-1-yl]butyl}amino-4-phénylpyridine

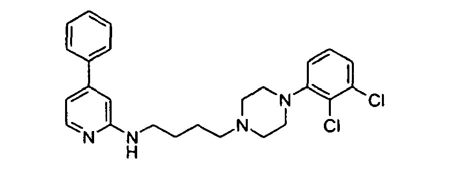

### Etape a : préparation du 4-[4-(2,3-dichlorophényl)pipérazin-1-yl]butyronitrile

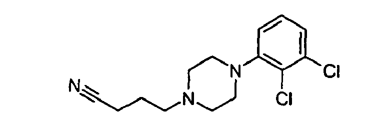

Le 4-[4-(2,3-dichlorophényl)pipérazin-1-yl]butyronitrile peut être obtenu par alkylation du chlorhydrate de la 1-(2,3-dichlorophényl)pipérazine par le 4-bromobutyronitrile selon le même mode opératoire que celui à l'exemple 1, étape a.

Rendement : 65% (huile visqueuse). Le produit est engagé brut dans l'étape suivante.

### Etape b : préparation de la 4-[4-(2,3-dichlorophényl)pipérazin-1-yl]butylamine

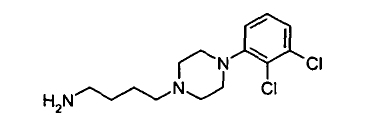

La 4-[4-(2,3-dichlorophényl)pipérazin-1-yl]butylamine peut être obtenue par réduction du 4-[4-(2,3-dichlorophényl)pipérazin-1-yl]butyronitrile selon le même mode opératoire que celui utilisé dans l'exemple 1, étape b.

Rendement : 91% (huile visqueuse)

Le produit est engagé brut dans l'étape suivante.

### Etape c : préparation de la 2-{4-[4-(2,3-dichlorophényl)pipérazin-1-yl]butyl}amino-4-phénylpyridine

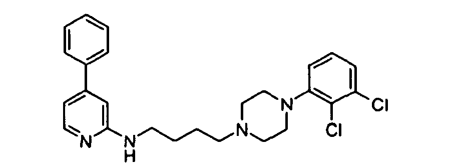

Dans un tube à essai, 0,19 g (2,0 mmol) de 2-chloro-4-phénylpyridine, 0,3 g (1,0 mmol) de 4-[4-(2,3-dichlorophényl)pipérazin-1-yl]butylamine et une pointe de spatule de 4-diméthylaminopyridine sont chauffés pendant 4 minutes dans un four à micro-onde. Le mélange est dilué avec de l'acétate d'éthyle et chromatographié sur gel de silice (éluant acétate d'éthyle/éthanol).

Après concentration des fractions d'élution, on obtient 20 mg (4%) de 2-{4-[4-(2,3-dichlorophényl)pipérazin-1-yl]butyl}amino-4-phénylpyridine sous forme d'huile visqueuse jaunâtre.

RMN ¹H (CDCl₃) : 8,15 (doublet, 1 H) ; 7,65 à 7,5 (massif, 2H) ; 7,5 à 7,35 (massif, 3H) ; 7,2 à 7,05 (massif, 2H) ; 6,9 (multiplet, 1 H) ; 6,75 (multiplet, 1 H) ; 6,55 (singulet, 1 H) ; 4,9 (singulet large, 1H) ; 3,4 (triplet, 2H) ; 3,2 à 3,0 (massif, 4H) ; 2,8 à 2,5 (massif, 4H) ; 2,5 (triplet, 2H) ; 1,8 à 1,6 (massif, 4H)

### Exemple 7 : préparation de la 2-{4-[4-(2,3-dichlorophényl)pipérazin-1-yl]butyl}amino-5-phénylpyridine

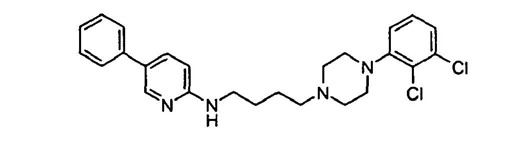

Dans un tube à essai, 0,2 g (1,0 mmol) de 2-chloro-5-phénylpyridine (exemple 1, étape c), 0,3 g (1,0 mmol) de 4-[4-(2,3-dichlorophényl)pipérazin-1-yl]butylamine (exemple 6, étape b) sont chauffés pendant 4 minutes à 300-350°C. Le mélange est dilué avec de l'acétate d'éthyle et chromatographié sur gel de silice (éluant acétate d'éthyle/méthanol).

Après concentration des fractions d'élution, le précipité est trituré dans de l'oxyde de diéthyle, filtré et séché sous vide. On obtient ainsi 20 mg (4%) de 2-{4-[4-(2,3-dichlorophényl)pipérazin-1-yl]butyl}amino-5-phénylpyridine sous forme de solide blanc.

Point de fusion : 110°C (tube)

RMN ¹H (DMSO d₆) : 8,25 (singulet, 1 H) ; 7,6 (doublet, 1 H) ; 7,55 à 7,45 (multiplet, 2H) ; 7,4 à 7,3 (multiplet, 2H) ; 7,3 à 7,15 (multiplet, 2H) 7,15 à 7,5 (multiplet, 1 H) ; 6,6 (triplet, 1 H) ; 6,5 (doublet, 1 H) ; 5,7 (singulet, 1 H) ; 3,4 à 3,2 (massif, 4H) ; 3,1 (triplet large, 2H) ; 3,0 à 2,85 (massif, 4H) ; 2,3 (triplet large, 2H) ; 1,6 à 1,4 (massif, 4H)

### Exemple 8 : préparation de la 2-{4-[4-(2,3-dichlorophényl)pipérazin-1-yl]butyl}amino-5-(2-méthylphényl)pyridine

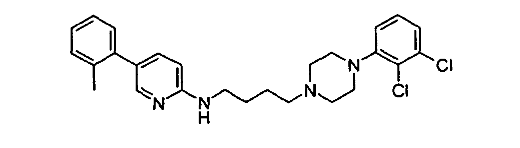

### Etape a : préparation de la 2-chloro-5-(2-méthylphényl)pyridine

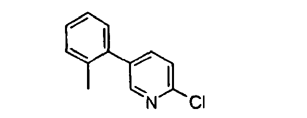

La 2-chloro-5-(2-méthylphényl)pyridine peut être préparée par réaction de Suzuki en partant de 2-chloro-5-bromopyridine et d'acide 2-méthyl-phénylboronique selon le même mode opératoire que celui décrit dans l'exemple 1, étape c.

Rendement : 47% (produit cristallisé)

RMN ¹H (CDCl₃) : 8,4 (singulet, 1 H) ; 7,6 (multiplet, 1 H) ; 7,5 à 7,1 (massif, 5H) ; 2,25 (singulet, 3H)

### Etape b : préparation de la 2-{4-[4-(2,3-dichlorophényl)pipérazin-1-yl]butyl}amino-5-(2-méthylphényl)pyridine

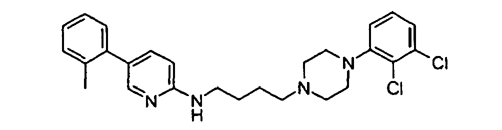

La 2-{4-[4-(2,3-dichlorophényl)pipérazin-1-yl]butyl}amino-5-(2-méthylphényl)pyridine peut être préparée à partir de la 2-chloro-5-(2-méthylphényl)pyridine obtenue précédemment et de 4-[4-(2,3-dichlorophényl)pipérazin-1-yl]butylamine (exemple 6, étape b) selon le même procédé que celui décrit à l'exemple 7.

Rendement : 7% (huile orangée)

RMN ¹H (CDCl₃) : 8,05 (singulet, 1 H) ; 7,4 (doublet, 1 H) ; 7,3 à 7,05 (massif, 6H) ; 6,95 (multiplet, 1 H) ; 6,45 (doublet, 1 H) ; 4,85 (singulet large, 1 H) ; 3,35 (triplet large, 2H) ; 3,2 à 3,0 (massif, 4H) ; 2,8 à 2,55 (massif, 4H) ; 2,5 (triplet,2H) ; 2,3 (singulet, 3H) ; 1,8 à 1,6 (massif, 4H)

### Exemple 9 : préparation de la 2-{4-[4-(3-trifluorométhylphényl)pipérazin-1-yl]butyl}amino-5-(2-méthylphényl)pyridine

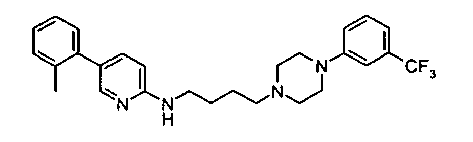

La 2-{4-[4-(3-trifluorométhylphényl)pipérazin-1-yl]butyl}amino-5-(2-méthylphényl)pyridine peut être préparée à partir de la 2-chloro-5-(2-méthylphényl)pyridine (exemple 8, étape a) et de la 4-[4-(3-trifluorométhylphényl)pipérazin-1-yl]butylamine (exemple 3, étape b) selon le même procédé que celui décrit à l'exemple 7.

Rendement : 7% (huile orangée)

RMN ¹H (CDCl₃) : 8,05 (doublet, 1 H) ; 7,5 à 7,0 (massif, 9H) ; 6,45 (doublet, 1 H) ; 4,85 (singulet large, 1 H) ; 3,35 (triplet large, 2H) ; 3,3 à 3,15 (massif, 4H) ; 2,75 à 2,55 (massif, 4H) ; 2,5 (triplet, 2H) ; 2,3 (singulet, 3H) ; 1,9 à 1,55 (massif, 4H)

### Exemple 10 : préparation de la 2-{4-[4-(3-trifluorométhylphényl)pipérazin-1-yl]butyl}amino-5-(4-fluorophényl)pyridine

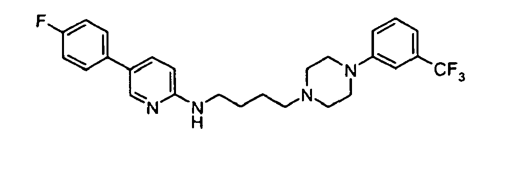

### Etape a : préparation de la 2-chloro-5-(4-fluorophényl)pyridine

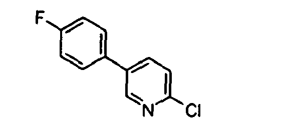

La 2-chloro-5-(4-fluorophényl)pyridine peut être préparée par réaction de Suzuki à partir de la 2-chloro-5-bromopyridine et de l'acide 4-fluorophénylboronique selon le même mode opératoire que celui décrit dans l'exemple 1, étape c.

Rendement : 74% (produit cristallisé)

RMN ¹H (CDCl₃) : 8,9 (singulet, 1 H) ; 8,55 (doublet, 1 H) ; 8,05 (doublet, 1 H) ; 7,8 à 7,5 (multiplet, 2H) ; 7,35 à 7,05 (multiplet, 2H)

### Etape b : préparation de la 2-{4-[4-(3-trifluorométhylphényl)pipérazin-1-yl]butyl}amino-5-(4-fluorophényl)pyridine

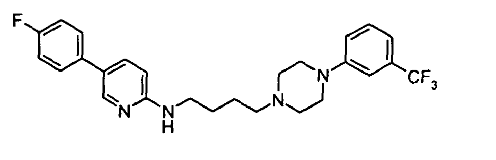

La 2-{4-[4-(3-trifluorométhylphényl)pipérazin-1-yl]butyl}amino-5-(4-fluorophényl)pyridine peut être préparée à partir de la 2-chloro-5-(4-fluorophényl)pyridine obtenue précédemment et de la 4-[4-(3-trifluorométhylphényl)pipérazin-1-yl]butylamine (exemple 3,étape b) selon le même mode opératoire que celui décrit à l'exemple 7.

Rendement : 4% (huile visqueuse rouge)

RMN ¹H (CDCl₃) : 8,3 (doublet, 1 H) ; 7,65 à 7,55 (massif, 2H) ; 7,5 à 7,2 (massif, 3H) ; 7,2 à 7,0 (massif, 4H) ; 6,45 (doublet, 1 H) ; 4,9 (singulet large, 1H) ; 3,35 (triplet, 2H) ; 3,3 à 3,1 (massif, 4H) ; 2,7 à 2,5 (massif, 4H) ; 2,5 (triplet, 2H) ; 1,85 à 1,5 (massif, 4H)

### Exemple 11 : préparation de la 2-{4-[4-(3-trifluorométhylphényl)pipérazin-1-yl]butyl}amino-4-(2-méthylphényl)pyridine

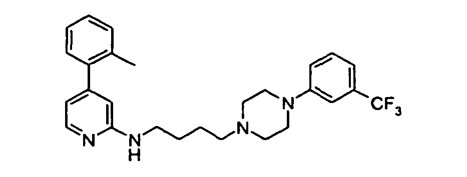

### Etape a : préparation du N-oxyde de 4-(2-méthylphényl)pyridine

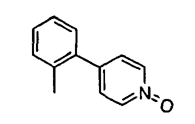

Un mélange de 10 mL de toluène, 0,268 g (0,23 mmol) de tetrakis(triphénylphosphine)palladium, 0,776 g (5,71 mmol) d'acide 2-méthylphénylboronique, de 2,85 mL (5,71 mmol) d'une solution aqueuse de carbonate de sodium 2M et de 0,737 g (5,19 mmol) de N-oxyde de 4-chloropyridine est chauffé au reflux pendant 4 heures.

Le milieu réactionnel est ensuite concentré sous vide, repris avec de l'acétate d'éthyle. La phase organique est lavée avec de l'eau, séchée sur sulfate de magnésium, filtrée et concentrée sous vide. Le résidu est chromatographié sur gel de silice (élution avec un mélange dichlorométhane/éthanol 95/5). On obtient ainsi 0,6 g (56%) de N-oxyde de 4-(2-méthylphényl)pyridine utilisé tel quel dans les synthèses ultérieures.

### Etape b : préparation de la 2-chloro-4-(2-méthylphényl)pyridine

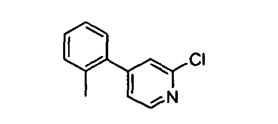

Un mélange de 0,6 g (3,24 mmol) de N-oxyde de 4-(2-méthylphényl)pyridine obtenu précédemment, de 1,8 g (31 mmol) de chlorure de sodium et de 3,3 mL (35,5 mmol) d'oxychlorure de phosphore est porté au reflux pendant une nuit.

Le milieu réactionnel est ensuite concentré sous vide. Le résidu est dilué dans du toluène et concentré de nouveau sous vide. Le résidu huileux est refroidi à environ 5°C et alcalinisé avec une solution aqueuse de soude normale jusqu'à pH = 9 puis extrait avec de l'acétate d'éthyle. La phase organique est lavée à l'eau, séchée sur sulfate de magnésium, filtrée et concentrée sous vide. Le résidu huileux est chromatographié sur gel de silice (élution avec un mélange heptane/acétate d'éthyle 3/1). On obtient ainsi 0,5 g (74%) de 2-chloro-4-(2-méthylphényl)pyridine sous forme d'une huile colorée utilisée telle quelle dans les synthèses ultérieures.

### Etape c : préparation de la 2-{4-[4-(3-trifluorométhylphényl)pipérazin-1-yl]butyl}amino-4-(2-méthylphényl)pyridine

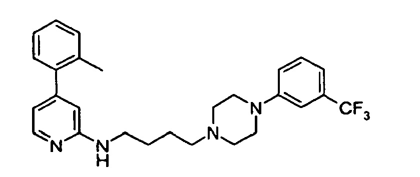

La 2-{4-[4-(3-trifluorométhylphényl)pipérazin-1-yl]butyl}amino-4-(2-méthylphényl)pyridine peut être obtenue à partir de la 2-chloro-4-(2-méthylphényl)pyridine préparée précédemment et de la 4-[4-(3-trifluorométhylphényl)pipérazin-1-yl]butylamine (exemple 3, étape b) selon le même mode opératoire que celui décrit dans l'exemple 3, étape c.

Rendement : 10% (huile rougeâtre)

RMN¹H (CDCl₃) : 8,1 (doublet, 1H) ; 7,35 à 7,15 (massif, 5H) ; 7,15 à 6,95 (massif, 3H) ; 6,55 (doublet, 1 H) ; 6,35 (singulet, 1 H) ; 5,15 (singulet large, 1 H) ; 3,3 à 3,15 (massif, 4H) ; 3,35 (triplet large, 2H) ; 2,8 à 2,6 (massif, 4H) ; 2,5 (triplet, 2H) ; 2,3 (singulet, 3H) ; 1,85 à 1,6 (massif, 4H)

### Exemple 12 : préparation de la 2-{4-[4-(2,3-dichlorométhylphényl)pipérazin-1-yl]butyl}amino-4-(2-méthylphényl)pyridine

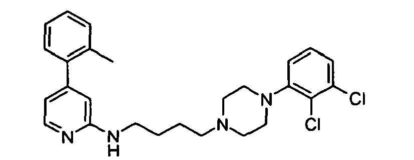

La 2-{4-[4-(2,3-dichlorométhylphényl)pipérazin-1-yl]butyl}amino-4-(2-méthylphényl)pyridine peut être préparée à partir de la 2-chloro-4-(2-méthylphényl)pyridine (exemple 11, étape b) et la 4-[4-(2,3-dichlorophényl)pipérazin-1-yl]butylamine (exemple 6, étape b) selon le même procédé que celui décrit dans l'exemple 7.

Rendement : 7% (huile orangée)

RMN ¹H (CDCl₃) : 8,1 (doublet, 1H) ; 7,3 à 7,1 (massif, 6H) ; 6,95 (multiplet, 1 H) ; 6,55 (doublet, 1 H) ; 6,3 (singulet, 1 H) ; 4,8 (singulet large, 1 H) ; 3,3 (triplet, 2H) ; 3,15 à 3,0 (massif, 4H) ; 2,7 à 2,5 (massif, 4H) ; 2,45 (triplet, 2H) ; 2,3 (singulet, 3H) ; 1,8 à 1,55 (massif, 4H)

### Exemple 13 : préparation de la 2-{4-[4-(2-cyano-3-trifluorométhylphényl)pipérazin-1-yl]butyl}amino-4-phénylpyridine

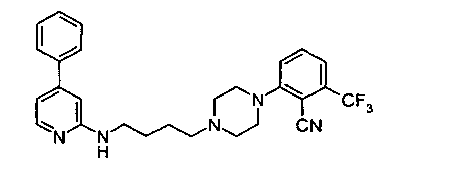

### Etape a : préparation de la 4-(2-cyano-3-trifluorométhylphényl)pipérazine

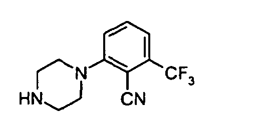

Un mélange de 3 g (15,8 mmol) de 2-fluoro-6-trifluorométhylbenzonitrile, 7,5 g (87 mmol) de pipérazine et de 24 mL de dioxanne est chauffé au reflux pendant 5 heures.

Le milieu réactionnel est concentré sous vide et le résidu est repris avec de l'acétate d'éthyle. Après lavage avec de l'eau, la phase organique est séchée sur sulfate de magnésium, filtrée et concentrée. Le produit cristallise à température ambiante. Après séchage sous vide, on obtient 3,6 g (82%) de 4-(2-cyano-3-trifluorométhylphényl)pipérazine utilisée telle quelle dans les synthèses ultérieures.

### Etape b : préparation du 2-{4-[4-(2-cyano-3-trifluorométhylphényl)pipérazin-1-yl]butyl}phtalimide.

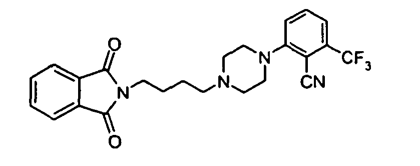

Un mélange de 2,55g (10 mmol) de 4-(2-cyano-3-trifluorométhylhényl)pipérazine préparée précédemment, de 1,5 g (10,9 mmol) de carbonate de potassium, de 3,1 g (10,9 mmol) de N-(4-bromobutyl)phtalimide et de 30 mL d'acétonitrile est porté au reflux pendant une nuit.

Le milieu réactionnel est ensuite concentré sous vide puis est repris avec de l'acétate d'éthyle. Après un lavage à l'eau, la phase organique est séchée sur sulfate de magnésium, filtrée et concentrée sous vide. Le résidu huileux est agité avec de l'oxyde de diisopropyle. Le surnageant est éliminé et le résidu est séché sous vide. On obtient ainsi 3,8 g (100%) de 2-{4-[4-(2-cyano-3-trifluorométhylphényl)pipérazin-1-yl]butyl}phtalimide sous forme d'une huile visqueuse utilisée telle quelle dans les synthèses ultérieures.

### Etape c : préparation du 4-(4-aminobutyl)-1-(2-cyano-3-trifluorométhylphényl)pipérazine

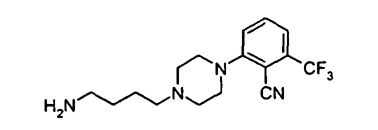

Une solution de 1 g (2,57 mmol) de 2-{4-[4-(2-cyano-3-trifluorométhylphényl)pipérazin-1-yl]butyl}phtalimide préparé précédemment et de 1 mL d'hydrazine hydratée dans 10 mL d'éthanol est agitée pendant une nuit à température ambiante.

La solution est concentrée sous vide. Le résidu est repris avec de l'eau, concentré de nouveau sous vide, acidifié avec une solution aqueuse d'acide chlorhydrique 0,5 N jusqu'à pH 1 et lavé avec de l'acétate d'éthyle. La phase aqueuse est refroidie. Une solution aqueuse de soude normale est ajoutée jusqu'à pH 9. La phase aqueuse basique est extraite deux fois avec de l'acétate d'éthyle. Les phases d'extraction sont réunies, lavées avec de l'eau, séchées sur sulfate de magnésium, filtrées et concentrées. On obtient ainsi 0,7 g (83%) de 4-(4-aminobutyl)-1-(2-cyano-3-trifluorométhylphényl)pipérazine sous forme d'une huile utilisée telle quelle dans les synthèses ultérieures.

### Etape d : préparation de la 2-{4-[4-(2-cyano-3-trifluorométhylphényl)pipérazin-1-yl]butyl}amino-4-phénylpyridine

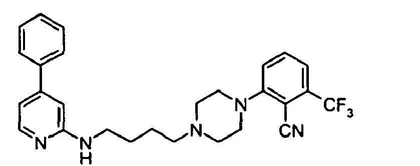

La 2-{4-[4-(2-cyano-3-trifluorométhylphényl)pipérazin-1-yl]butyl}amino-4-phénylpyridine peut être obtenue à partir de la 4-(4-aminobutyl)-1-(2-cyano-3-trifluorométhylphényl)pipérazine préparée précédemment et de 2-chloro-4-phénylpyridine selon le même mode opératoire que celui décrit dans l'exemple 3, étape c.

Rendement : 4% (solide crème)

Point de fusion : 102°C (tube)

RMN ¹H (CDCl₃) : 8,1 (doublet, 1H) ; 7,7 à 7,5 (massif, 3H) ; 7,5 à 7,35 (massif, 3H) ; 7,3 (doublet, 1H) ; 7,2 (multiplet, 1H) ; 6,8 (doublet, 1 H) ; 6,55 (singulet, 1H) ; 4,95 à 4,7 (singulet large, 1 H) ; 3,35 (triplet large, 2H) ; 3,3 à 3,2 (massif, 4H) ; 2,8 à 2,6 (massif, 4H) ; 2,5 (triplet, 2H) ; 1,85 à 1,6 (massif, 4H)

### Exemple 14 : préparation du dichlorhydrate de la 2-{5-[4-(3-trifluorométhylphényl)pipérazin-1-yl]pentyl}-amino-4-phénylpyridine

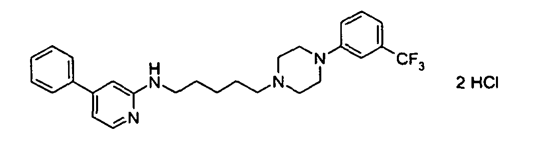

### Etape a : préparation de la 2-chloro-4-phénylpyridine-1-oxyde

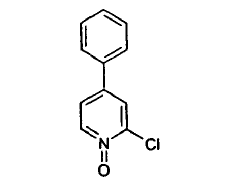

A une solution de 3,3 g (17,4 mmol) de 2-chloro-4-phénylpyridine dans 35 mL de dichlorométhane refroidie à 5°C sont ajoutés par portion, 10,73 g (62,2 mmol) d'acide méta-chloroperbenzoïque. La suspension est agitée pendant une nuit à température ambiante.

Le milieu réactionnel est dilué avec 150 mL d'acétate d'éthyle et lavé successivement avec de l'eau, avec une solution aqueuse saturée de métabisulfite de sodium, avec une solution aqueuse saturée de carbonate de sodium et avec de l'eau.

La phase organique est ensuite séchée sur sulfate de magnésium, filtrée et concentrée.
Le résidu solide est agité avec un mélange acétonitrile/oxyde de diisopropyle, filtré et séché sous vide.

Rendement : 51 %

Point de fusion : 152°C

RMN ¹H (CDCl₃) : 8,4 (doublet, 1H, J = 6,7 Hz) ; 7,7 (d, 1H, J = 2,5 Hz) ; 7,65 à 7,35 (massif, 6H).

### Etape b : préparation de la 2-{5-[4-(3-trifluorométhylphényl)pipérazin-1-yl]pentyl}-amino-4-phénylpyridine-1-oxyde

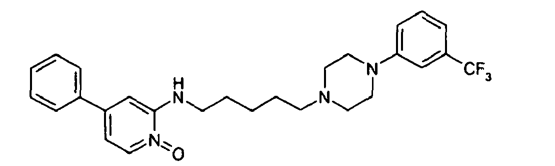

A une solution de 0,41 g (2 mmol) de 2-chloro-4-phénylpyridine-1-oxyde dans 5 mL d'alcool tertio-amylique sont ajoutés 0,75 g (2,4 mmol) de 3-[5-(3-trifluorométhylphényl)pipérazin-1-yl]pentylamine et 0,18 g (2,2 mmol) d'hydrogénocarbonate de sodium. Le mélange est chauffé au reflux pendant une nuit.

Après concentration sous vide, le résidu est repris avec de l'acétate d'éthyle et lavé deux fois avec de l'eau. La phase organique est séchée sur sulfate de magnésium, filtrée et concentrée. L'huile obtenue est purifiée par chromatographie sur gel de silice (éluant dichlorométhane/méthanol 98/2).

Rendement : 66% (huile visqueuse marron).

RMN ¹H (CDCl₃) : 8,15 (d, 1 H, J = 6,7 Hz) ; 7,65 à 7,55 (massif, 2H) ; 7,55 à 7,3 (massif, 4H) ; 7,2 à 7,0 (massif, 3H) ; 6,9 à 6,7 (massif, 3H) ; 3,35 (multiplet, 2H, J = 6 Hz) ; 3,25 (multiplet, 4H) ; 2,6 (multiplet, 4H) ; 2,4 (triplet, 2H, J = 7 Hz) ; 1,9 à 1,4 (massif, 6H)

### Etape c : préparation du dichlorhydrate de la 2-{5-[4-(3-trifluorométhylphényl)pipérazin-1-yl]pentyl}-amino-4-phénylpyridine

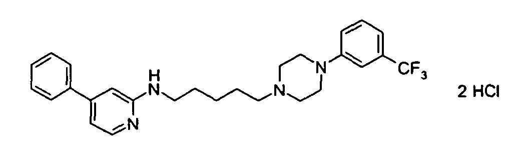

Une solution de 0,51 g (1,05 mmol) de 2-{5-[4-(3-trifluorométhylphényl)pipérazin-1-yl]pentyl}amino-4-phénylpyridine-1-oxyde dans 5 mL de chloroforme est refroidie à 5°C. Après addition de 0,2 mL (2,3 mmol) de trichlorure de phosphore, le milieu réactionnel est agité à température ambiante pendant une nuit.

Le mélange est concentré sous vide puis est hydrolysé avec 10 mL d'eau et 2 mL d'une solution aqueuse de soude N. Le produit est extrait avec de l'acétate d'éthyle (deux fois 25 mL). Les phases organiques sont réunies, séchées sur sulfate de magnésium, filtrées et concentrées. L'huile obtenue est purifiée par chromatographie sur gel de silice (éluant dichlorométhane/méthanol 98/2).

L'huile obtenue est dissoute dans de l'oxyde de diéthyle. Une solution saturée de chlorure d'hydrogène dans de l'oxyde de diéthyle est ajoutée jusqu'à l'obtention d'un précipité. Le solide est filtré, lavé avec de l'acétate d'éthyle et séché sous vide.

Solide blanc.

Point de fusion : 94°C

RMN ¹H(CDCl₃) : 8,15 (d, 1H, J = 5Hz) ; 7, 7 à 7,55 (massif, 2H) ; 7,55 à 7,25 (massif, 4H) ; 7,2 à 7,0 (massif, 3H) ; 6,8 (doublet, 1H, J = 5 Hz); 6,6 (singulet, 1H) ; 4,6 (triplet large, 1H) ; 3,4 (multiplet, 2H) ; 3,2 (multiplet, 4H) ; 2,6 (multiplet, 4H) ; 2,4 (triplet, 2H, J = 7 Hz) ; 1,8 à 1,4 (massif, 6H)

Les produits des exemples 15 à 20 sont préparés selon la même suite réactionnelle que celle décrite dans l'exemple 14.

### Exemple 15 : préparation du dichlorhydrate de 2-{4-[4-(1,2-diméthylphényl)-pipérazin-1-yl]butyl}amino-4-phénylpyridine

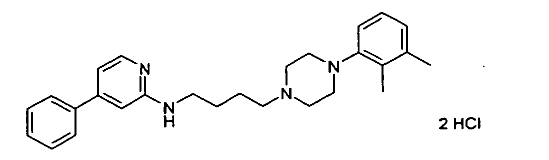

L'huile marron obtenue après l'étape de réduction par le trichlorure de phosphore (voir exemple 14, étape c) est dissoute dans de l'oxyde de diéthyle. Une solution saturée de chlorure d'hydrogène dans de l'oxyde de diéthyle est ajoutée jusqu'à l'obtention d'un précipité. Le solide est filtré, lavé avec de l'acétate d'éthyle et séché sous vide.
Solide blanc.

Point de fusion : 101°C

RMN ¹H(DMSO D₆) : 8,0 (doublet, 1H, J = 6,7 Hz) ; 7,85 à 7,7 (massif, 2H) ; 7,65 à 7,5 (massif, 3H) ; 7,35 (singulet, 1H) ; 7,2 (doublet, 1H, J = 5Hz) ; 7,05 (triplet, 1H, J = 6,7 Hz) ; 6,95 à 6,8 (massif, 2H) ; 3,5 (multiplet, 4H) ; 3,2 à 3,0 (massif, 8H) ; 2,2 (singulet, 3H) ; 2,15 (singulet, 3H) ; 2,0 à 1,75 (massif, 2H) ; 1,75 à 1,55 (massif, 2H)

### Exemple 16: Préparation de la 2-{4-[4-(2-cyano-3-méthylphényl)pipérazin-1-yl]butyl}amino-4-phénylpyridine

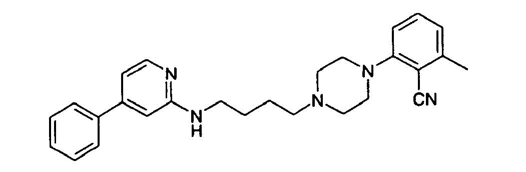

Huile marron clair

RMN ¹H (CDCl₃) : 8,1 (doublet, 1H, J = 5 Hz) ; 7,7 à 7,55 (massif, 2H) ; 7,55 à 7,3 (massif, 4H) ; 6,95 à 6,75 (massif, 3H) ; 6,6 (singulet, 1H) ; 5,5 (singulet large, 1H) ; 3,4 (multiplet, 2H) ; 3,2 (multiplet, 4H) ; 2,7 (multiplet, 4H) ; 2,65 à 2,4 (massif, 5H) ; 1,85 à 1,6 (massif, 4H)

### Exemple 17 : préparation de la 2-{4-[4-(3,5-dichlorophényl)pipérazin-1-yl]butyl}amino-4-phénylpyridine

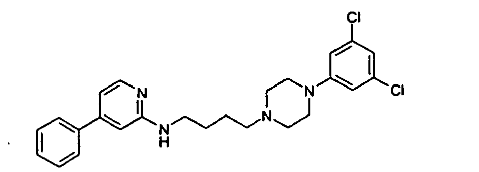

Solide blanc.

Point de fusion : 108°C

RMN ¹H (CDCl₃) : 8,1 (doublet, 1H, J= 5 Hz) ; 7,75 à 7,55 (massif, 2H) ; 7,55 à 7,35 (massif, 3H) ; 6,85 à 6,7 (massif, 4H) ; 6,5 (singulet, 1H) ; 4,9 (singulet large, 1H); 3,4 (multiplet, 2H) ; 3,2 (multiplet, 4H) ; 2,6 (multiplet, 4H) ; 2,45 (triplet, 2H, J = 6,7 Hz) ; 1,85 à 1,6 (massif, 4H)

### Exemple 18 : préparation de la 2-{4-[4-(3-chlorophényl)pipérazin-1-yl]butyl}amino-4-phénylpyridine

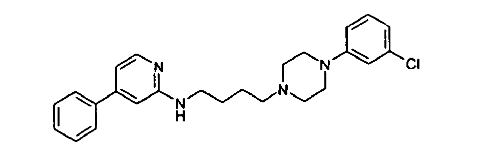

Solide blanc.

Point de fusion : 104°C RMN ¹H (CDCl₃) : 8,1 (doublet, 1H, J = 5 Hz) ; 7,7 à 7,55 (massif, 2H) ; 7,55 à 7,35 (massif, 3H) ; 7,2 (triplet, 1H, J = 7 Hz) ; 6,9 (singulet, 1H) ; 6,9 à 6,75 (massif, 3H) ; 6,55 (singulet, 1H) ; 4,8 (singulet large, 1 H) ; 3,35 (multiplet, 2H) ; 3,2 (multiplet, 4H) ; 2,6 (multiplet, 4H) ; 2,45 (triplet, 2H, J = 6,7 Hz) ; 1,85 à 1,5 (massif, 4H)

### Exemple 19 : préparation du dichlorhydrate du 2-{4-[4-(5,6,7,8-tétrahydro-1-naphtyl)pipérazin-1-yl]butyl}amino-4-phénylpyridine

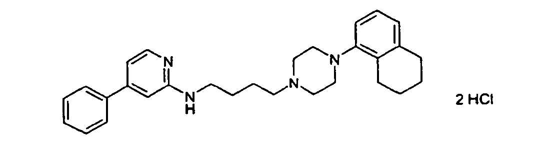

Solide blanc

Point de fusion : 168°C
RMN ¹H (DMSO D₆) : 8,0 (doublet, 1H, J = 7 Hz) ; 7,7 à 7,55 (massif, 2H) ; 7,55 à 7,35 (massif, 3H) ; 7,1 (triplet, 1H, J = 7 Hz) ; 6,9 à 7,65 (massif, 4H) ; 3,55 à 3,2 (massif, 6H) ; 3,2 à 2,95 (massif, 6H) ; 2,8 à 2,55 (massif, 4H) ; 1,85 à 1,45 (massif, 8H)

### Exemple 20 : préparation de la 2-{4-[4-(2-cyano-3-isopropoxyphényl)pipérazin-1-yl]butyl}amino-4-phénylpyridine

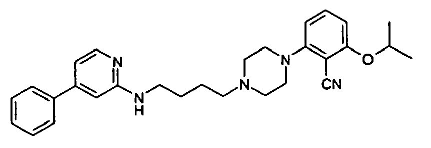

Solide cristallisé jaune

Point de fusion : 148°C

RMN ¹H (CDCl₃) : 8,1 (doublet, 1H, J = 5 Hz) ; 7,65 à 7,55 (massif, 2H) ; 7,5 à 7,35 (massif, 3H) ; 7,3 (triplet, 1H, J = 7Hz) ; 6,8 (multiplet, 1H) ; 6,65 à 6,45 (massif, 3H) ; 5,0 (singulet large, 1H) ; 4,6 (septuplet, 1H, J = 6 Hz) ; 3,35 (multiplet, 2H) ; 3,25 (multiplet, 4H) ; 2,7 (multiplet, 4H) ; 2,5 (triplet, 1H, J = 7 Hz) ; 1,85 à 1,6 (massif, 4H) ; 1,4 (doublet, 6H, J = 6 Hz)

### Exemple 21 : préparation de la 2-{4-[4-(3-trifluorométhylphényl)pipérazin-1-yl]butyl}amino-4-(4-fluorophényl)pyridine

### Etape a : préparation de la 4-(4-fluorophényl)pyridine

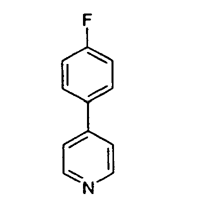

Un mélange de 0,34 g (0,3 mmol) de tétrakis(triphénylphosphine)-palladium, de 1,94 g (10 mmol) de chlorhydrate de la 4-bromopyridine, de 19 mL de toluène, de 15 mL d'une solution aqueuse de carbonate de sodium et de 1,54 5 g (11 mmol) d'acide 4-fluoroboronique est chauffé à 80°C sous argon pendant 5 heures.

Le milieu est repris avec de l'acétate d'éthyle, lavé à l'eau, séché sur sulfate de magnésium, filtré et concentré. Le résidu obtenu est purifié par chromatographie sur gel de silice (éluant heptane/acétate d'éthyle 2/1).

Rendement : 70% (solide blanc)

RMN ¹H (CDCl₃) : 8,75 à 8,6 (massif, 2H) ; 7,7 à 7,55 (massif, 2H) ; 7,55 à 7,4 (massif, 2H) ; 7,3 à 7,1 (massif, 2H)

### Etape b : préparation de la 4-(4-fluorophényl)pyridine-1-oxyde

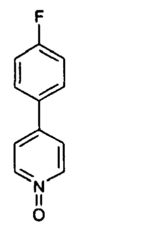

A une solution de 1,18 g (6,81 mmol) de 4-(4-fluorophényl)pyridine dans 5 mL de chloroforme refroidie à 0°C sont ajoutés 3,5 g (10,2 mmol) d'acide méta-chloroperbenzoïque. La suspension est agitée pendant 30 minutes.

Le produit est purifié par chromatographie sur silice (éluant acétate d'éthyle puis dichlorométhane/méthanol 90/10.

Rendement : 70% (solide blanc)

RMN ¹H (CDCl₃) : 8,3 à 8,15 (massif, 2H) ; 7,7 à 7,55 (massif, 2H) ; 7,55 à 7,4 (massif, 2H) ; 7,3 à 7,1 (massif, 2H)

### Etape c : préparation de la 4-(4-fluorophényl)-2-chloropyridine

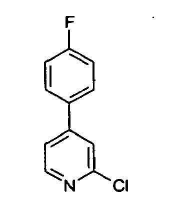

Un mélange de 0,9 g (4,8 mmol) de 4-(4-fluorophényl)pyridine-1-oxyde et de 10 mL de chlorure de phosphoryle est chauffé au reflux pendant une nuit.

Après distillation sous vide de l'excès de chlorure de phosphoryle, le résidu est repris avec 10 mL de toluène puis est concentré de nouveau. Le résidu est solubilisé dans 25 mL de toluène, lavé avec 10 mL d'eau et avec 10 mL d'une solution aqueuse saturée d'hydrogénocarbonate de sodium. La phase organique est séchée sur sulfate de magnésium, filtrée et concentrée. Le solide obtenu est repris dans de l'heptane et est filtré sous vide.

Rendement : 64% (solide blanc)

RMN ¹H (CDCl₃) : 8,4 (doublet, 1H, J = 5 Hz) ; 7,7 à 7,55 (massif, 2H) ; 7,5 (singulet, 1H) ; 7,4 (doublet, 1H, J = 5 Hz) ; 7,3 à 7,1 (massif, 2H)

### Etape d : préparation de la 2-{4-[4-(3-trifluorométhylphényl)pipérazin-1-yl]butyl}amino-4-(4-fluoro-phényl)pyridine

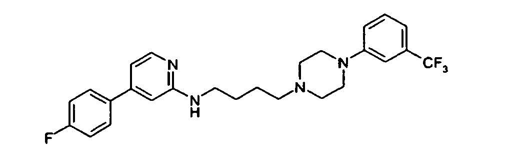

La 2-{4-[4-(3-trifluorométhylphényl)pipérazin-1-yl]butyl}amino-4-(4-fluorophényl)pyridine est ensuite obtenue à partir de la 4-(4-fluorophényl)-2-chloropyridine en utilisant la même suite réactionnelle que celle décrite à l'exemple 14, étape a à c.

Solide blanc

Point de fusion : 85°C

RMN ¹H (CDCl₃) : 8,1 (doublet, 1H, J = 5 Hz) ; 7,7 à 7,5 (massif, 2H) ; 7,35 (triplet, 1H , J = 7 Hz) ; 7,25 (massif, 5H) ; 6,75 (doublet, 1H , J = 5 Hz) ; 6,55 (singulet, 1H) ; 5,2 (singulet large, 1 H) ; 3,4 (multiplet, 2H) ; 3,3 (multiplet, 4H) ; 2,6 (multiplet, 4H) ; 2,55 (triplet, 2H, J = 7 Hz) ; 2,05 à 1,65 (massif, 4H)

### Exemple 22: préparation de la 2-{4-[4-(3-trifluorométhylphényl)pipérazin-1-yl]butyl}amino-4-(4-chlororophényl)pyridine

### Etape a : préparation de la 4-(4-chlorophényl)pyridine

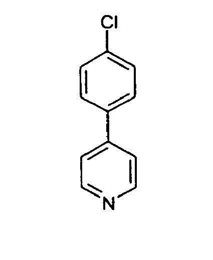

La 4-(4-chlorophényl)pyridine est obtenue par à partir du chlorhydrate de la 4-bromopyridine et de l'acide 4-chlorophénylboronique en utilisant le même mode opératoire que celui décrit dans l'exemple 21, étape a.

Rendement : 90% (solide blanc)

RMN ¹H (CDCl₃) : 8,75 à 8,6 (massif, 2H) ; 7,6 à 7,5 (massif, 2H) ; 7,55 à 7,4 (massif, 4H)

### Etape b : préparation de la 4-(4-chlorophényl)-2-chloropyridine

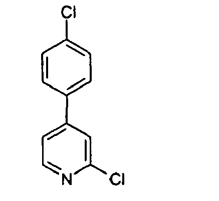

La 4-(4-chlorophényl)-2-chloropyridine est préparée à partir de la 4-(4-chlorophényl)pyridine selon la même suite réactionnelle que celle décrite pour la préparation de 4-(4-fluorophényl)-2-chloropyridine (exemple 21, étape b et c).

RMN ¹H (CDCl₃) : 8,45 (doublet, 1H, J = 5 Hz) ; 7,65 à 7,35 (massif, 6H)

### Etape c : préparation de la 2-{4-[4-(3-trifluorométhylphényl)pipérazin-1-yl]butyl}amino-4-(4-chlorophényl)pyridine

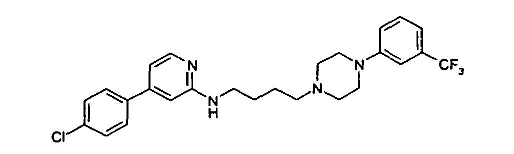

La 2-{4-[4-(3-trifluorométhylphényl)pipérazin-1-yl]butyl}amino-4-(4-chlorophényl)pyridine est préparée à partir de la 4-(4-chlorophényl)-2-chloropyridine en utilisant la même suite réactionnelle que celle décrite pour l'exemple 14, étape a à c.

Solide blanc

RMN ¹H (CDCl₃) : 8,1 (doublet, 1H, J = 5 Hz) ; 7 ,6 à 7,25 (massif, 5H) ; 7,15 à 7,0 (massif, 3H) ; 6,75 (multiplet, 1H) ; 6,5 (singulet, 1H) ; 5,0 (singulet large, 1H) ; 3,4 (multiplet, 2H) ; 3,3 (multiplet, 4H) ; 2,6 (multiplet, 4H) ; 2,5 (triplet, 2H, J = 7 Hz) ; 1,85 à 1,6 (massif, 4H)

### Exemple 23: préparation de la 2-{4-[4-(3-trifluorométhylphényl)pipérazin-1-yl]butyl}amino-4-(3-fluorophényl)pyridine

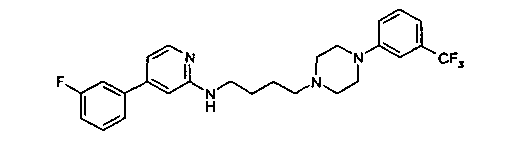

### Etape a : préparation de la 2-chloro-4-bromopyridine

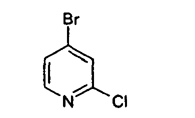

81,5 mL d'une solution aqueuse d'acide bromhydrique à 48% sont ajoutés, à 0°C, à 8,9 g (69,2 mmol) de 2-chloro-4-aminopyridine. Une addition de 33,4 g (208,75 mmol) de dibrome est ensuite effectuée pendant 10 minutes.

La solution est refroidie à -10°C et une solution de 10,65 g (154 mmol) de nitrite de sodium dans 20 mL d'eau est coulée sur une période de 30 minutes. La solution est agitée encore pendant 10 minutes à -10°C puis pendant 1 h30 à température ambiante.

La solution est refroidie à 5°C et une solution aqueuse saturée de sulfite de sodium est ajoutée jusqu'à décoloration du milieu réactionnel. Le milieu réactionnel est basifié à l'aide d'une solution aqueuse de soude à 35%. La phase aqueuse basique est extraite deux fois avec de l'oxyde de diéthyle. Les phases organiques sont réunies, séchées sur sulfate de magnésium, filtrées et concentrées. L'huile jaune obtenue (13 g) est chromatographiée sur gel de silice (éluant 1/9 acétate d'éthyle/heptane).

Rendement : 52% (huile légèrement colorée)

RMN ¹H (CDCl₃) : 8,25 (doublet, 1H, J = 5 Hz) ; 7,55 (singulet, 1H) ; 7,4 (doublet, 1H, J = 5 Hz)

### Etape b : préparation de la 2-chloro-4-bromopyridine-1-oxyde

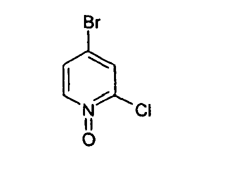

La 2-chloro-4-bromopyridine-1-oxyde est préparée à partir de la 2-chloro-4-bromopyridine selon le même mode opératoire que celui décrit à l'exemple 14, étape a.
Rendement : 46% (huile)

RMN ¹H (CDCl₃) : 8,2 (doublet, 1H, J = 7Hz) ; 7,65 (singulet, 1H) ; 7,35 (multiplet, 1H)

### Etape c : préparation de la 2-{4-[4-(3-trifluorométhylphényl)pipérazin-1-yl]butyl}-amino-4-bromopyridine-1-oxyde

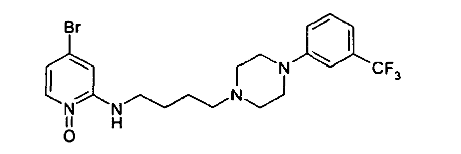

La 2-{ 4-[4-(3-trifluorométhylphényl)pipérazin-1-yl]butyl}amino-4-bromopyridine-1-oxyde est préparée à partir de la 2-chloro-4-bromopyridine-1-oxyde et de la 4-[4-(3-trifluorométhyl)phényl)pipérazin-1-yl]butylamine (exemple 3, étape b) selon le même mode opératoire que celui décrit dans l'exemple 14, étape b.
Rendement 72% (produit cristallisé)
RMN ¹H (CDCl₃) : 7,95 (doublet, 1H, J = 7 Hz) ; 7,4 (triplet, 1H, J = 7 Hz) ; 7,2 à 7,05 (massif, 3H) ; 7,0 (triplet large, 1H) ; 6,75 (singulet, 1 H) ; 6,65 (doublet, 1 H, J = 7 Hz) ; 3,4 à 3,2 (massif, 6H) ; 2,7 (massif, 4H) ; 2,5 (triplet, 2H, J = 7Hz) ; 1,9 à 1,6 (massif, 4H)

### Etape d : préparation de la 2-{4-[4-(3-trifluorométhylphényl)pipérazin-1-yl]butyl}-amino-4-(3-fluorophényl)pyridine-1-oxyde

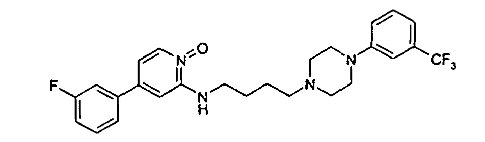

Un mélange de 0,4 g (1 mmol) de 2-{4-[4-(3-trifluorométhylphényl)pipérazin-1-yl]butyl}amino-4-bromopyridine-1-oxyde, de 3 mL de dioxanne, de 2,08 mL d'une solution aqueuse 2M de carbonate de sodium, de 36 mg (0,3 mmol) de tétrakis(triphénylphosphine)palladium et de 0,15 g (1,1 mmol) d'acide 3-fluorophénylboronique est chauffé au reflux pendant 8 heures.

Le milieu réactionnel est dilué avec de l'acétate d'éthyle et lavé deux fois avec de l'eau. La phase organique est séchée sur sulfate de magnésium, filtrée et concentrée. Le résidu huileux obtenu (0,3 g) est chromatographié sur gel de silice (éluant dichlorométhane/méthanol 95/5).

Rendement : 38% (huile visqueuse)

RMN ¹H (CDCl₃) : 8,15 (doublet, 1H, J = 7Hz) ; 7,5 à 7,2 (massif, 4H) ; 7,2 à 7,0 (massif, 4H) ; 6,9 (triplet large, 1H) ; 6,85 à 6,65 (massif, 2H) ; 3,4 (multiplet, 2H) ; 3,25 (multiplet, 4H) ; 2,6 (multiplet, 4H) ; 2,5 (triplet, 2H, J = 7 Hz) ; 1,9 à 1,6 (massif, 4H)

### Etape e : préparation de la 2-{4-[4-(3-trifluorométhylphényl)pipérazin-1-yl]butyl}-amino-4-(3-fluorophényl)pyridine

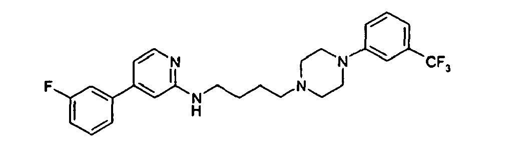

La 2-{4-[4-(3-trifluorométhylphényl)pipérazin-1-yl]butyl}amino-4-(3-fluorophényl)pyridine est obtenue par réduction de la 2-{4-[4-(3-trifluorométhylphényl)pipérazin-1-yl]butyl}amino-4-(3-fluorophényl)pyridine-1-oxyde par le trichlorure de phosphore selon le même mode opératoire que celui décrit à l'exemple 14, étape c.

Solide blanc

RMN ¹H (CDCl₃) : 8,1 (doublet, 1H, J = 5 Hz) ; 7,55 à 7,2 (massif, 4H) ; 7,2 à 7,0 (massif, 4H) ; 6,8 (doublet, 1H, J = 5 Hz) ; 6,6 (singulet, 1H) ; 5,25 (singulet large, 1H) ; 3,5 à 3,2 (massif, 6H) ; 2,7 (multiplet, 4H) ; 2,6 (triplet large, 2H) ; 1,85 à 1,65 (massif, 4H)

Les produits des exemples 24 à 38 sont préparés selon la même suite réactionnelle que celle décrite pour la préparation de l'exemple 23.

### Exemple 24: préparation de la 2-{4-[4-(3-trifluorométhylphényl)pipérazin-1-yl]butyl}amino-4-(4-méthoxyphényl)pyridine

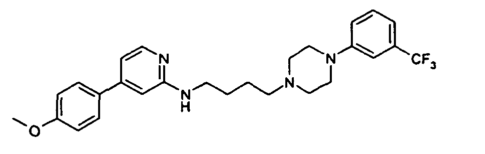

Huile très visqueuse

RMN ¹H (CDCl₃) : 8,1 (singulet large, 1H) ; 7,65 à 7,5 (massif, 2H) ; 7,35 (triplet, 1H, J = 7 Hz); 7,15 à 7,0 (massif, 3H) ; 7,0 à 6,9 (massif, 2H) ; 6,8 (doublet, 1H, J = 5Hz) ; 6,5 (singulet, 1H) ; 4,9 (singulet large, 1H) ; 3,85 (singulet, 3H) ; 3,4 (multiplet, 2H) ; 3,3 (multiplet, 4H) ; 2,6 (multiplet, 4H) ; 2,45 (triplet, 2H, J = 7 Hz) ; 1,85 à 1,6 (massif, 4H)

### Exemple 25: préparation de la 2-{4-[4-(3-trifluorométhylphényl)pipérazin-1-yl]butyl}amino-4-(3-thiényl)pyridine

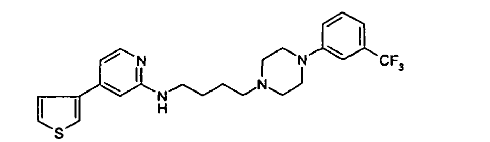

Solide blanc cristallisé

Point de fusion : 95°C

RMN ¹H (CDCl₃) : 8,05 (doublet, 1H, J = 5 Hz) ; 7,6 (singulet, 1H) ; 7,5 à 7,25 (massif, 3H) ; 7,2 à 7,0 (massif, 3H) ; 6,8 (doublet, 1H, J = 5 Hz) ; 6,55 (singulet, 1H) ; 4,9 (singulet large, 1H) ; 3,35 (multiplet, 2H) ; 3,25 (multiplet, 4H) ; 2,6 (multiplet, 4H) ; 2,5 (triplet, 2H, J = 7Hz) : 1,85 à 1,6 (massif, 4H)

### Exemple 26: préparation de la 2-{4-[4-(3-trifluorométhylphényl)pipérazin-1-yl]butyl}amino-4-(2-furyl)pyridine

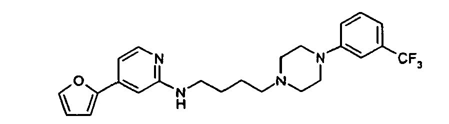

Solide blanc cristallisé

Point de fusion : 98°C

RMN ¹H (CDCl₃) : 8,05 (doublet, 1H, J = 5 Hz) ; 7,5 (singulet, 1H) ; 7,35 (triplet, 1H, J = 7 Hz) ; 7,2 à 7,0 (massif, 3H) ; 6,9 à 6,7 (massif, 2H) ; 6,65 (singulet, 1H) ; 6,5 (multiplet, 1H) ; 4,85 (singulet large, 1H) ; 3,4 (multiplet, 2H) ; 3,25 (multiplet, 4H) ; 2,6 (multiplet, 4H) ; 2,45 (triplet, 2H, J = 7 Hz) ; 1,85 à 1,55 (massif, 4H)

### Exemple 27: préparation de la 2-{4-[4-(3-trifluorométhylphényl)pipérazin-1-yl]butyl}amino-4-(2-thiényl)pyridine

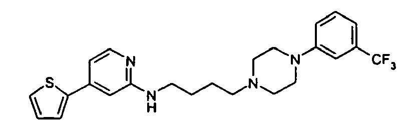

Solide blanc cristallisé

Point de fusion : 95°C

RMN ¹H (CDCl₃) : 8,05 (doublet, 1H, J = 5 Hz) ; 7,5 à 7,25 (massif, 3H) ; 7,2 (massif, 4H) ; 6,8 (doublet, 1H, J = 5 Hz) ; 6,5 (singulet, 1H) ; 5,0 (singulet large, 1H) ; 3,35 (multiplet, 2H) ; 3,25 (multiplet, 4H) ; 2,65 (multiplet, 4H) ; 2,5 (triplet, 2H, J = 7 Hz) ; 1,85 à 1,6 (massif, 4H)

### Exemple 28: préparation de la 2-{4-[4-(3-trifluorométhylphényl)pipérazin-1-yl]butyl}amino-4-(2-fluorophényl)pyridine

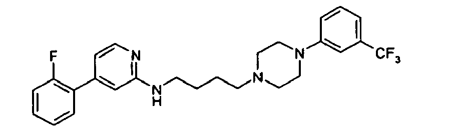

Solide blanc cristallisé

Point de fusion : 100°C

RMN ¹H (CDCl₃) : 8,15 (doublet, 1H, J = 7 Hz) ; 7,5 à 7,0 (massif, 8H) ; 6,75 (doublet, 1H, J = 5,5 Hz) ; 6,56 (singulet, 1H) ; 4,8 (triplet large, 1H) ; 3,35 (multiplet, 2H) ; 3,25 (multiplet, 4H) ; 2,6 (multiplet, 4H) ; 2,45 (triplet, 2H, J = 7 Hz) ; 1,85 à 1,6 (massif, 4H)

### Exemple 29: préparation de la 2-{4-[4-(3-trifluorométhylphényl)pipérazin-1-yl]butyl}amino[4,4']bipyridine

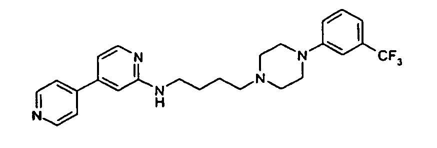

Huile visqueuse

RMN ¹H (CDCl₃) : 8,65 (doublet, 1 H, J = 5Hz) ; 8,1 (doublet, 1 H, J = 5 Hz) ; 7,5 à 7,2 (massif, 4H) ; 7,2 à 7,0 (massif, 3H) ; 6,8 (multiplet, 1H) ; 6,55 (singulet, 1H) ; 5,5 (singulet large, 1H) ; 3,4 (multiplet, 2H); 3,2 (massif, 4H) ; 2,65 (multiplet, 4H) ; 2,5 (triplet, 2H, J = 7 Hz) ; 1,85 à 1,5 (massif, 4H)

### Exemple 30 : préparation de la 2-{4-[4-(3-trifluorométhylphényl)pipérazin-1-yl]butyl}amino-4-(3-méthylphényl)pyridine

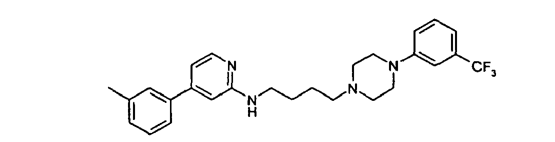

Solide blanc cristallisé

Point de fusion : 87°C

RMN ¹H (CDCl₃) : 8,05 (doublet, 1H, J = 5 Hz) ; 7,5 à 7,15 (massif, 5H) ; 7,2 à 7,0 (massif, 3H) ; 6,8 (multiplet, 1H) ; 6,6 (singulet, 1H) ; 4,4 (singulet large, 1H) ; 3,4 (multiplet, 2H) ; 3,3 (multiplet, 4H) ; 2,65 (multiplet, 4H) ; 2,5 (triplet, 2H, J = 7 Hz) ; 2,4 (singulet, 3H) ; 1,85 à 1,55 (massif, 4H)

### Exemple 31 : préparation de la 2-{4-[4-(3-trifluorométhylphényl)pipérazin-1-yl]butyl}amino-4-(3-méthoxyphényl)pyridine

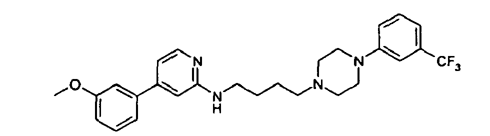

Solide blanc

Point de fusion : 73°C

RMN ¹H (CDCl₃) : 8,1 (doublet, 1H, J = 5 Hz) ; 7,45 à 7,25 (massif, 2H) ; 7,2 à 7,0 (massif, 5H) ; 6,95 (doublet, 1H, J = 5 Hz) ; 6,8 (multiplet, 1H) ; 6,55 (singulet, 1H) ; 5,05 (singulet large, 1H) ; 3,85 (singulet, 3H) ; 3,4 (multiplet, 2H) ; 3,3 (multiplet, 4H) ; 2,65 (multiplet, 4H) ; 2,45 (triplet, 2H, J = 7 Hz) ; 1,85 à 1,65 (massif, 4H)

### Exemple 32: préparation de la 2-{4-[4-(3-trifluorométhylphényl)pipérazin-1-yl]butyl}amino[4,3']bipyridine

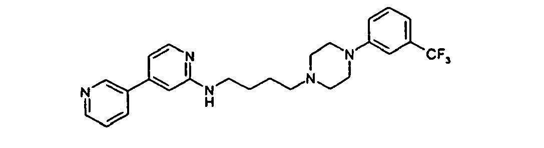

Solide cristallisé blanc

Point de fusion : 112°C

RMN ¹H (CDCl₃) : 8,85 (singulet, 1H) ; 8,65 (doublet, 1H, J = 5 Hz) ; 8,15 (doublet, 1H, J = 5 Hz) ; 7,9 (multiplet, 1H) ; 7,45 à 7,25 (massif, 2H) ; 7,2 à 7,0 (massif, 3H) ; 6,8 (multiplet, 1H) ; 6,55 (singulet, 1H) ; 5,0 (singulet large, 1H) ; 3,4 (multiplet, 2H) ; 3,3 (multiplet, 4H) ; 2,65 (multiplet, 4H) ; 2,5 (triplet, 2H, J = 7 Hz) ; 1,85 à 1,6 (massif, 4H)

### Exemple 33: préparation de la 2-{4-[4-(3-trifluorométhylphényl)pipérazin-1-yl]butyl}amino-4-(4-trifluorométhoxyphényl)pyridine

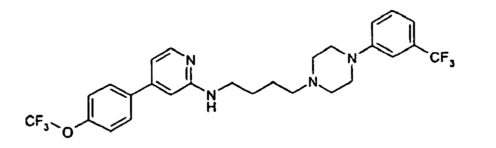

Solide cristallisé blanc

Point de fusion : 72°C

RMN ¹H (CDCl₃) : 8,1 (doublet, 1H, J = 5 Hz) ; 7,65 à 7,5 (massif, 2H) ; 7,45 à 7,2 (massif, 3H); 7,15 à 7,0 (massif, 3H); 6,75 (multiplet, 1H); 6,5 (singulet, 1H) ; 5,0 (singulet large, 1H) ; 3,35 (multiplet, 2H) ; 3,2 (multiplet, 4H) ; 2,65 (multiplet, 4H) ; 2,5 (triplet, 2H, J = 7 Hz) ; 1,85 à 1,55 (massif, 4H)

### Exemple 34: préparation de la 2-{4-[4-(3-trifluorométhylphényl)pipérazin-1-yl]butyl}amino-4-(4-acétylphényl)pyridine

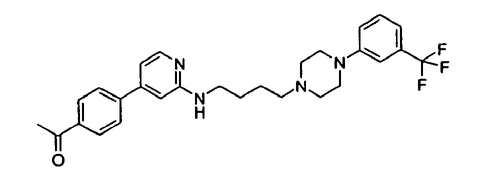

Huile visqueuse

RMN ¹H (CDCl₃) : 8,15 (doublet, 1H, J = 5 Hz) ; 8,1 à 7,95 (massif, 2H) ; 7,75 à 7,6 (massif, 2H) ; 7,35 (triplet, 1H, J = 7,5Hz) ; 7,2 à 7,0 (massif, 3H) ; 6,8 (doublet, 1H, J = 5 Hz) ; 6,6 (singulet, 1H) ; 5,0 (triplet large, 1H) ; 3,4 (multiplet, 2H) ; 3,3 (multiplet, 4H) ; 2,7 à 2,55 (massif, 7H) ; 2,5 (multiplet, 2H) ; 1,85 à 1,6 (massif, 4H)

### Exemple 35: préparation de la 2-{4-[4-(3-trifluorométhylphényl)pipérazin-1-yl]butyl}amino-4-(3-acétylphényl)pyridine

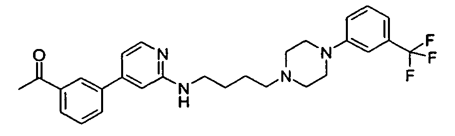

Huile visqueuse

RMN ¹H (CDCl₃) : 8,25 à 8,05 (massif, 2H) ; 8,0 (doublet, 1H, J = 7 Hz) ; 7,8 (doublet, 1H, J = 7 Hz) ; 7,55 (triplet, 1H, J = 7 Hz) ; 7,35 (triplet, 1H, J = 7 Hz) ; 7,2 à 7,0 (massif, 3H) ; 6,8 (singulet, 1H) ; 6,65 (singulet, 1H) ; 5,2 (triplet, large, 1H) ; 3,4 (multiplet, 2H) ; 3,3 (multiplet, 4H) ; 2,7 (multiplet, 4H) ; 2,65 (singulet, 3H) ; 2,55 (multiplet, 2H) ; 1,85 à 1,6 (massif, 4H)

### Exemple 36: préparation de la 2-{4-[4-(3-trifluorométhylphényl)pipérazin-1-yl]butyl}amino-4-(4-hydroxyméthylphényl)pyridine

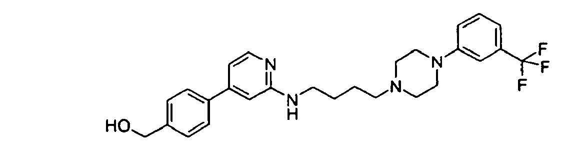

Huile visqueuse

RMN ¹H (CDCl₃) : 8,1 (doublet, 1H, J = 5 Hz) ; 7,65 à 7,5 (massif, 2H) ; 7,5 à 7,4 (massif, 2H) ; 7,35 (triplet, 1H, J = 7,5 Hz) ; 7,15 à 7,0 (massif, 3H) ; 7,75 (doublet, 1H, J = 5 Hz) ; 6,5 (singulet, 1H) ; 5,0 (triplet, large, 1H) ; 4,7 (singulet, 2H) ; 3,4 (multiplet, 2H) ; 3,25 (multiplet, 4H) ; 2,8 (singulet large, 1H) ; 2,6 (multiplet, 4H) ; 2,45 (multiplet, 2H) ; 1,85 à 1,55 (massif, 4H)

### Exemple 37: préparation de la 2-{4-[4-(3-trifluorométhylphényl)pipérazin-1-yl]butyl}amino-4-(benzo[1,3]dioxol-5-yl)pyridine

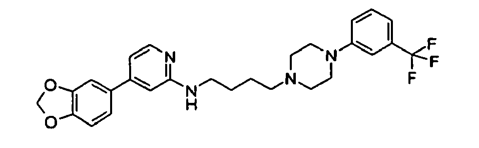

Solide blanc amorphe

RMN ¹H (CDCl₃) : 8,1 (doublet, 1H, J = 5 Hz) ; 7,35 (triplet, 1H, J = 7,5 Hz) ; 7,2 à 7,0 (massif, 5H) ; 6,9 (doublet, 1H, J = 7,5 Hz) ; 6,75 (multiplet, 1H) ; 6,5 (singulet, 1H); 6,0 (singulet, 2H) ; 4,8 (triplet, large, 1H); 3,35 (multiplet, 2H) ; 3,3 (multiplet, 4H) ; 2,6 (multiplet, 4H) ; 2,5 (multiplet, 2H) ; 1,8 à 1,55 (massif, 4H)

### Exemple 38: préparation de la 2-{4-[4-(3-trifluorométhylphényl)pipérazin-1-yl]butyl}amino-4-(4-éthoxycarbonylphényl)pyridine

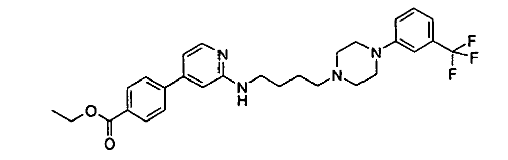

Solide jaune

Point de fusion : 101 °C

RMN ¹H (CDCl₃) : 8,25 à 8,0 (massif, 3H) ; 7,8 à 7,55 (massif, 2H) ; 7,35 (triplet, 1H, J = 7,5 Hz) ; 7,2 à 7,0 (massif, 3H) ; 6,8 (doublet, 1H , J = 5 Hz) ; 6,6 (singulet, 1H) ; 5,15 (triplet, large, 1H); 4,4 (quadruplet, 2H, J = 7,5 Hz) ; 3,4 (multiplet, 2H) ; 3,3 (multiplet, 4H) ; 2,7 (multiplet, 4H) ; 2,55 (multiplet, 2H) ; 1,9 à 1,65 (massif, 4H) ; 1,4 (triplet, 3H, J = 7,5 Hz)

### Exemple 39: préparation de la 2-{4-[4-(3-trifluorométhylphényl)pipérazin-1-yl]butyl}amino-4-(4-(1-hydroxyéthyl)phényl)pyridine

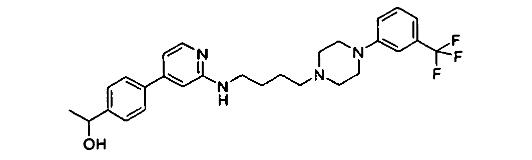

A une solution de 50 mg (0,1 mmol) de 2-{4-[4-(3-trifluorométhylphényl)pipérazin-1-yl]butyl}amino-4-(4-acétylphényl)pyridine (exemple 34) dans 5 mL de méthanol, sont ajoutés, à 5°C, 10 mg (2,6 mmol) de borohydrure de sodium. La solution est agitée pendant 2 heures à température ambiante.

Après une addition de 20 mL d'eau, le produit est extrait 2 fois avec 20 mL d'acétate d'éthyle. La phase organique est lavée à l'eau, séchée sur sulfate de magnésium, filtrée et concentrée. Le résidu huileux est purifié par chromatographie sur gel de silice (éluant 95/5 dichlorométhane/méthanol).

Huile visqueuse

RMN ¹H (CDCl₃) : 8,1 (doublet, 1H, J = 5 Hz) ; 7,65 à 7,5 (massif, 2H) ; 7,5 à 7,4 (massif, 2H) ; 7,3 (triplet, 1H, J = 7,5 Hz) ; 7,2 à 7,0 (massif, 3H) ; 6,8 (multiplet, 1H) ; 6,5 (singulet, 1H) ; 5,0 (triplet, large, 1H) ; 4,95 (quadruplet, 1H, J = 7 Hz) ; 3,35 (multiplet, 2H) ; 3,3 (multiplet, 4H) ; 2,6 (multiplet, 4H) ; 2,45 (multiplet, 2H) ; 2,4 (singulet large, 1H) ; 1,85 à 1,6 (massif, 4H) ; 1,5 (doublet, 3H, J = 7 Hz)

### Exemple 40: préparation de la 2-{4-[4-(3-trifluorométhylphényl)pipérazin-1-yl]butyl}amino-4-(4-(1-hydroxy-1-méthyléthyl)phényl)pyridine

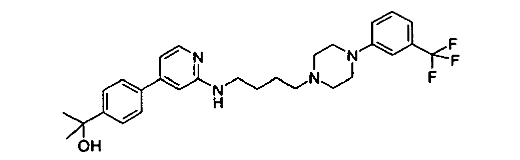

A une solution de 0,1 g (0,2 mmol de 2-{4-[4-(3-trifluorométhylphényl)pipérazin-1-yl]butyl}amino-4-(4-acétylphényl)pyridine (exemple 34) dans 2 mL de tétrahydrofurane anhydre, sont ajoutés sous argon et à 0°C, 2 mL d'une solution de chlorure de méthylmagnésium 2,6M dans le tétrahydrofurane. La température est maintenue à 0°C pendant 30 minutes.

Après retour à température ambiante, la solution est diluée avec de l'acétate d'éthyle, lavée à l'eau, séchée sur sulfate de magnésium, filtrée et concentrée. L'huile résiduelle est purifiée par chromatographie sur gel de silice (éluant dichlorométhane/méthanol 96/4).

Huile visqueuse

RMN ¹H (CDCl₃) : 8,1 (doublet, 1H, J = 5 Hz) ; 7,55 (singulet, 4H) ; 7,35 (triplet, 1H, J = 7,5 Hz) ; 7,15 à 7,0 (massif, 3H) ; 6,8 (multiplet, 1H) ; 6,55 (singulet, 1 H) ; 5,1 (triplet large, 1 H) ; 3,35 (multiplet, 2H) ; 3,25 (multiplet, 4H) ; 2,65 (multiplet, 4H) ; 2,5 (multiplet, 2H) ; 2,0 (singulet, large, 1H) ; 1,85 à 1,65 (massif, 4H) ; 1,6 (singulet, 6H)

### Exemple 41: préparation de la 2-{4-[4-(3-trifluorométhylphényl)pipérazin-1-yl]butyl}amino-4-(3-(1-hydroxy-1-méthyléthyl)phényl)pyridine

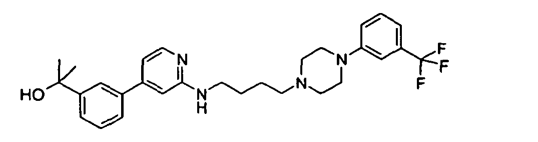

La 2-{4-[4-(3-trifluorométhylphényl)pipérazin-1-yl]butyl}amino-4-(3-(1-hydroxy-1-méthyléthyl)phényl)pyridine est préparée à partir de la 2-{4-[4-(3-trifluorométhylphényl)pipérazin-1-yl]butyl}amino-4-(3-acétylphényl)pyridine (exemple 35) selon le même mode opératoire que celui utilisé dans l'exemple 40.

Huile visqueuse

RMN ¹H (CDCl₃) : 8,1 (doublet, 1H, J = 5 Hz) ; 7,75 (singulet, 1H) ; 7,6 à 7,4 (massif, 3H) ; 7,35 (triplet, 1H, J = 7,5 Hz); 7,2 à 7,0 (massif, 3H) ; 6,8 (doublet, 1H, J = 5 Hz) ; 6,6 (singulet, 1H) ; 5,15 (triplet large, 1H) ; 3,4 (multiplet, 2H) ; 3,3 (multiplet, 4H) ; 2,65 (multiplet, 4H) ; 2,5 (multiplet, 2H) ; 2,2 (singulet large, 1H) ; 1,85 à 1,7 (massif, 4H) ; 1,65 (singulet, 6H)

### Exemple 42: préparation du sel de sodium de la 2-{4-[4-(3-trifluorométhylphényl)pipérazin-1-yl]butyl}amino-4-(4-carboxyphényl)pyridine

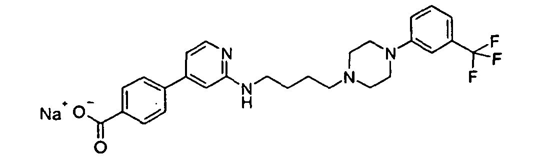

Un mélange de 80 mg (1,5 mmol) de 2-{4-[4-(3-trifluorométhylphényl)pipérazin-1-yl]butyl}amino-4-(4-éthoxycarbonylphényl)pyridine (exemple 38), de 10 mL d'éthanol et de 1,5 mL d'une solution aqueuse de soude 0,1N est chauffé au reflux pendant 4 heures.

La solution est concentrée sous vide. Le solide résiduel est trituré dans de l'oxyde de diéthyle, filtré et séché sous vide.

Solide

RMN ¹H (Méthanol D₄) : 8,1 à 7,85 (massif, 3H) ; 7,6 à 7,5 (massif, 2H) ; 7,35 (triplet, 1H, J = 7,5 Hz) ; 7,2 à 6,9 (massif, 3H) ; 6,9 à 6,65 (massif, 2H) ; 3,5 à 3,1 (massif, 6H) ; 2,6 (multiplet, 4H) ; 2,5 (multiplet, 2H) ; 1,8 à 1,5 (massif, 4H)

### Exemple 43: préparation du 2-{4-[4-(3-trifluorométhylphényl)pipérazin-1-yl]butyl}amino-4-phénylthiazole

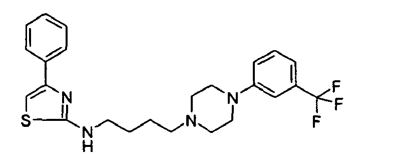

Un mélange de 0,19 g (1 mmol) de 2-chloro-4-phénylthiazole (*Bull. Soc*. *Chim. Fr.,* 2498, 1963) et de 0,3 g (1 mmol) de 4-[4-(3-trifluorométhylphényl)pipérazine-1-yl]butylamine (exemple 3, étape b) est chauffé à 150°C pendant 2 minutes.

Le mélange est dilué avec de l'acétate d'éthyle, lavé avec une solution aqueuse de soude 0,5N puis avec de l'eau. La phase organique est séchée sur sulfate de magnésium, filtrée et concentrée. Le résidu huileux obtenu est purifié par chromatographie sur gel de silice (éluant dichlorométhane/méthanol 95/5).

Solide jaune cristallisé : 155°C

Rendement : 7%

RMN ¹H (CDCl₃) : 7,8 (multiplet, 2H) ; 7,45 à 7,2 (massif, 4H) ; 7,2 à 7,0 (massif, 3H) ; 6,7 (singulet, 1H) ; 6,4 (triplet large, 1H) ; 3,45 à 3,3 (massif, 6H) ; 2,7 (multiplet, 4H) ; 2,5 (triplet, 2H, J = 7 Hz) ; 1,9 à 1,6 (massif, 4H)

### Exemple 44: préparation du 2-{4-[4-(3-trifluorométhylphényl)pipérazin-1-yl]butyl}amino-5-phényloxazole

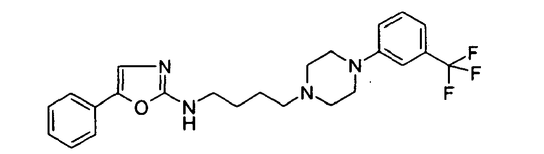

### Etape a : préparation du 2-chloro-5-phényloxazole

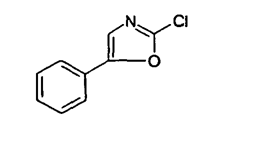

A un mélange de 0,5 g (2,8 mmol) de 5-phényl-3*H*-oxazole-2-thione (FR 1.450.443) et de 2,3 mL de chlorure de phosphoryle refroidi à 0°C, est ajouté 0,29 g (2,8 mmol) de triéthylamine. Le mélange est chauffé à 120°C pendant 3 heures.

Le milieu est dilué avec de l'acétate d'éthyle et lavé avec de l'eau. La phase organique est séchée sur sulfate de magnésium, filtrée et concentrée. Le produit est purifié par chromatographie sur gel de silice (éluant 15/85 acétate d'éthyle/heptane).

Huile visqueuse marron

Rendement: 18%

RMN ¹H (CDCl₃) : 7,7 à 7,55 (massif, 2H) ; 7,5 à 7,3 (massif, 3H) ; 7,25 (singulet, 1H)

### Etape b : préparation du 2-{4-[4-(3-trifluorométhylphényl)pipérazin-1-yl]butyl}amino-5-phényloxazole

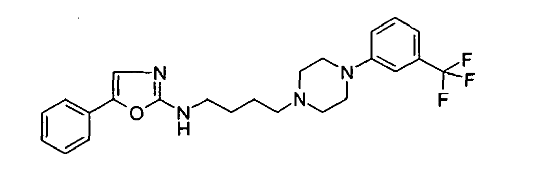

Une solution de 35 mg (0,2 mmol) de 2-chloro-5-phényloxazole dans 0,5 mL de tétrahydrofurane est ajoutée, à 0°C, à une solution de 62 mg (0,2 mmol) de 4-[4-(3-trifluorométhylphényl)pipérazine-1-yl]butylamine (exemple 3, étape b) et de 20 mg (0,2 mmol) de triéthylamine dans 0,2 mL de tétrahydrofurane. Le mélange est agité pendant une nuit sous argon.

Après concentration sous vide, le produit est purifié par chromatographie sur gel de silice (éluant dichlorométhane/méthanol 97/3).

Solide blanc

Rendement : 12%

Point de fusion : 151°C

RMN ¹H (CDCl₃) : 7,45 à 7,3 (massif, 4H) ; 7,2 à 7,05 (massif, 5H) ; 7,0 (singulet, 1H) ; 6,25 (singulet large, 1H) ; 3,5 à 3,3 (massif, 6H) ; 2,75 (multiplet, 4H) ; 2,55 (triplet large, 2H) ; 1,9 à 1,7 (massif, 4H)

### Exemple 45 : préparation du chlorhydrate de 2-{4-[4-(3-trifluorométhylphényl)pipérazin-1-yl]butyl}aminoquinoléine

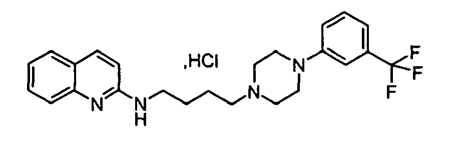

Un mélange de 0,2 g (0,66 mmol) de 4-[4-(3-trifluorométhylphényl)pipérazin-1-yl]butylamine (exemple 3, étape b) et de 0,5 g (3,06 mmol) de 2-chloroquinoléine commerciale est porté au reflux pendant une minute. Le milieu réactionnel est ensuite dilué avec 15 mL d'acétate d'éthyle puis lavé avec 10 mL d'eau. La phase organique est séchée sur sulfate de magnésium, filtrée et concentrée. Le résidu huileux est chromatographié sur gel de silice (élution à l'acétate d'éthyle). Après concentration des fractions d'élutions, 0,15 g de 2-{4-[4-(3-trifluorométhylphényl)pipérazine-1-yl]butyl}aminoquinoléine est obtenu.

La salification est effectuée par dissolution dans 10 mL d'oxyde de diéthyle et ajout de 1,5 ml d'une solution saturée de chlorure d'hydrogène dans de l'oxyde de diéthyle. Après agitation pendant 5 minutes, le précipité est filtré, lavé avec 5 mL d'oxyde de diéthyle et séché sous vide.

Masse obtenue : 0,16 g (52%), solide blanc.

Point de fusion : 170°C (tube)

RMN ¹H (D₂O) : 8,25 à 8,0 (massif, 1H) ; 7,85 à 7,6 (massif, 3H) ; 7,55 à 7,35 (massif, 2H) ; 7,35 à 7,15 (massif, 3H) ; 7,05 à 6,85 (massif, 1H) ; 3,9 (multiplet, 2H) ; 3,7 (multiplet, 2H) ; 3,55 (triplet, 2H) ; 3,35 à 3,05 (massif, 6H) ; 2,0 à 1,7 (massif, 4H)

### Exemple 46 : préparation du chlorhydrate de 1-{4-[4-(3-trifluorométhylphényl)pipérazin-1-yl]butyl}aminoisoquinoléine

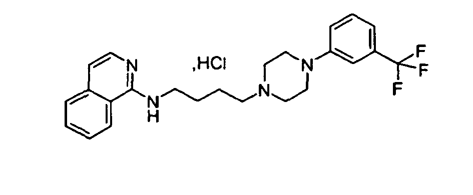

Le chlorhydrate de 1-{4-[4-(3-trifluorométhylphényl)pipérazin-1-yl]butyl}aminoisoquinoléine peut être obtenu à partir de la 1-chloroisoquinoléine commerciale et de la 4-[4-(3-trifluorométhylphényl)pipérazin-1-yl]butylamine (exemple 3, étape b) selon le même mode opératoire que celui décrit dans l'exemple 14.

Rendement : 33%, solide rosé.

Point de fusion : 260°C (tube)

RMN ¹H (D₂O) : 8,25 (doublet, 1H) ; 8,0 à 7,8 (massif, 2H) ; 7,7 (triplet, 1H) ; 7,55 à 7,3 (massif, 2H) ; 7,3 à 7,2 (massif, 3H) ; 7,15 (doublet, 1H); 3,9 (multiplet, 2H) ; 3,7 (multiplet, 2H) ; 3,6 (triplet, 2H) ; 3,35 à 3,0 (massif, 6H) ; 2,0 à 1,7 (massif, 4H)

### Exemple 47 : préparation du chlorhydrate de 2-{4-[4-(2,3-dichlorophényl)-pipérazin-1-yl]butyl}aminoquinoléine

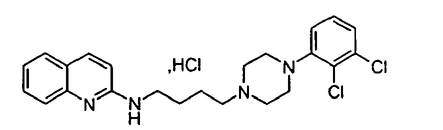

Le chlorhydrate de 2-{4-[4-(2,3-dichlorophényl)pipérazin-1-yl]butyl}aminoquinoléine peut être obtenu à partir de la 2-chloroquinoléine et de la 4-[4-(2,3-dichlorophényl)pipérazin-1-yl]butylamine (exemple 6, étape b) selon le même mode opératoire que celui décrit dans l'exemple 45.

Rendement : 38%, solide blanc.

Point de fusion : 280°C (tube)

RMN ¹H (D₂O) : 8,2 (doublet, 1H) ; 7,75 à 7,6 (massif, 2H) ; 7,55 (multiplet, 1H) ; 7,4 à 7,25 (massif, 3H) ; 7,15 (doublet, 1H) ; 7, 0 (doublet, 1H) ; 3,9 à 2,9 (multiplet, 12H) ; 2,0 à 1,7 (massif, 4H)

### Exemple 48 : préparation du chlorhydrate de 1-{4-[4-(2,3-dichlorophényl)-pipérazin-1-yl]butyl}aminoisoquinoléine

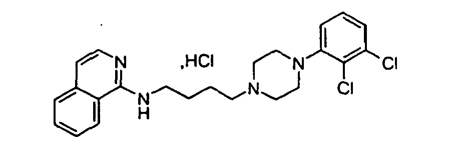

Le chlorhydrate de 1-{4-[4-(2,3-dichlorophényl)pipérazin-1-yl]butyl}aminoisoquinoléine peut être obtenu à partir de la 1-chloroisoquinoléine et de la 4-[4-(2,3-dichlorophényl)pipérazin-1-yl]butylamine (exemple 6, étape b) selon le même mode opératoire que celui décrit dans l'exemple 45.

Rendement : 44%, solide blanc.

Point de fusion : 150°C (tube)

RMN ¹H (D₂O) : 8,25 (doublet, 1 H) ; 8 à 7,8 (massif, 2H) ; 7,7 (triplet, 1H) ; 7,55 (doublet, 1 H) ; 7,4 à 7,2 (massif, 2H) ; 7,2 à 7,05 (massif, 2H) ; 3,9 à 2,9 (multiplet, 12H) ; 2,05 à 1,75 (massif, 4H)

### Exemple 49 : détermination de l'affinité des composés pour les récepteurs humains D3 à la dopamine et adrénergique alpha 1 humain.

### Liaison de [³H] spiperone

Des cellules CHO ont été transfectées par le gène codant pour le récepteur humain D3 à la dopamine (hD3). La liaison de [³H]spiperone (0,5 à 2 nM) s'effectue en présence de 5 à 10 µg de protéines membranaires dans un milieu contenant 120 mM NaCl, 5 mM KCI, et 50 mM Tris HCL pH 7,4 ; une incubation de 60 minutes à température ambiante est nécessaire. La liaison non spécifique est estimée en présence de 5 µM d'halopéridol. Les cellules non transfectées sont dépourvues de toute activité de liaison.

### Liaison de [³H] prasozine

Des cellules HEK 293 ont été transfectées par le gène codant pour le récepteur adrénergique alpha 1 humain (hα1). La liaison de [³H]prazosine (0,02 à 2 nM) s'effectue en présence de 5 à 10 µg de protéines membranaires dans un milieu contenant 0,5 mM EDTA et 50 mM Tris HCL pH 7,4 ; une incubation de 60 minutes à température ambiante est nécessaire. La liaison non spécifique est estimée en présence de 10µM de phentolamine. Les cellules non transfectées sont dépourvues de toute activité de liaison.

**Tableau 1: Effets antagonistes (Ki, nM) sur les récepteurs hD3 et hα1 mesurés par liaison de [³H] spiperone et de [³H] prasozine, respectivement.**

| N° d'exemple | hD3 | hα1 |
|---|---|---|
| | Ki (nM) | Ki (nM) |
| 4 | 0,76 | 162,5 |
| 6 | 0,55 | 135,7 |

## Revendications

1. Composés de formule (I) : 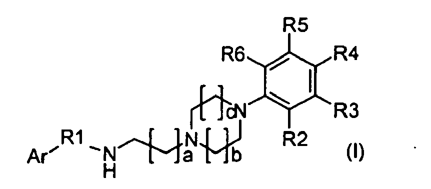 dans laquelle :
■ R1 représente un hétéroaryle à cinq ou six chaînons, contenant un ou plusieurs hétéroatomes, choisi parmi 2-pyridyle, 2-pyrimidinyle, 2-pyridazinyle, 2-pyrazinyle, 2-imidazolyle, 2-oxazolyle, 2-thiazolyle, 3-isoxazolyle, 3-isothiazolyle, 1,2,4-triazol-2-yle, 1,3,4-oxadiazol-2-yle, 1,3,4-thiadiazol-2-yle, éventuellement substitué par un ou plusieurs groupements identiques ou différents choisis parmi un atome d'halogène ou un groupe hydroxy, alkyle, monofluoroalkyle, polyfluoroalkyle, alkoxy, polyfluoroalkoxy, alkylsulfanyle, polyfluoroalkylsulfanyle ;
■ Ar est un aryle ou hétéroaryle, éventuellement substitué par un ou plusieurs substituants identiques ou différents choisis parmi un atome d'halogène ou un groupe hydroxy, alkyle, monofluoroalkyle, polyfluoroalkyle, alkoxy, polyfluoroalkoxy, alkylsulfanyle, polyfluoroalkylsulfanyle, cyano, carbamoyle, dialkylcarbamoyle, Alkyl-C(=O)-, Alkyl-O-C(=O)-, HO-C(=O)-, (HO)Alkyl-, ou Ar est fusionné avec un cycle hydrocarboné ou hétérocycle, saturé, insaturé ou aromatique ;
■ a=2, 3 ou 4;
■ b et c identiques ou différents représentent 1 ou 2 ;
■ R2, R3, R4, R5 et R6 représentent chacun indépendamment un atome d'hydrogène ou d'halogène ou un groupement hydroxy, alkyle, monofluoroalkyle, polyfluoroalkyle, alkoxy, polyfluoroalkoxy, alkylsulfanyle, polyfluoroalkylsulfanyle, cyano, -NRR', -COOR, -COR, -CONRR' ou bien deux des R2, R3, R4, R5 et R6 adjacents sont reliés entre eux pour former un cycle hydrocarboné ou un hétérocycle saturé ou insaturé, fusionné au noyau phényle auquel ils sont rattachés ;
où R, R' identiques ou différents représentent indépendamment un atome d'hydrogène, ou un groupe alkyle ;
ainsi que leurs stéréoisomères ou leur mélanges, leurs formes tautomères, leurs hydrates, solvates, leurs sels et esters pharmaceutiquement acceptables.

2. Composés selon la revendication 1 tels que R1 représente 2-pyridyle.

3. Composés selon la revendication 1 ou 2 tels que Ar est un aryle, éventuellement substitué par un ou plusieurs substituants identiques ou différents choisis parmi un atome d'halogène ou un groupe alkyle.

4. Composés selon l'une quelconque des revendications précédentes tels que a = 3.

5. Composés selon l'une quelconque des revendications précédentes tels que b et c représentent 1.

6. Composés selon l'une quelconque des revendications précédentes tels que R2, R3, R4, R5 et R6 représentent chacun indépendamment un atome d'hydrogène ou d'halogène ou un groupement polyfluoroalkyle.

7. Composés selon l'une quelconque des revendications précédentes choisis parmi :
- 2-{4-[4-(2-fluorophényl)pipérazin-1-yl]butyl}amino-5-phénylpyridine
- 2-{4-[4-(2-fluorophényl)pipérazin-1-yl]butyl}amino-4-phénylpyridine
- 2-{4-[4-(3-trifluorométhylphényl)pipérazin-1-yl]butyl}amino-4-phénylpyridine
- Dichlorhydrate de 2-{4-[4-(3-trifluorométhylphényl)pipérazin-1-yl]butyl}amino4-phénylpyridine
- 2-{4-[4-(3-trifluorométhylphényl)pipérazin-1-yl]butyl}amino-5-phénylpyridine
- 2-{4-[4-(2,3-dichlorophényl)pipérazin-1-yl]butyl}amino-4-phénylpyridine
- 2-{4-[4-(2,3-dichlorophényl)pipérazin-1-yl]butyl}amino-5-phénylpyridine
- 2-{4-[4-(2,3-dichlorophényl)pipérazin-1-yl]butyl}amino-5-(2-méthylphényl)pyridine
- 2-{4-[4-(3-trifluorométhylphényl)pipérazin-1-yl]butyl}amino-5-(2-méthylphényl)pyridine
- 2-{4-[4-(3-trifluorométhylphényl)pipérazin-1-yl]butyl}amino-5-(4-fluorophényl)pyridine
- 2-{4-[4-(3-trifluorométhylphényl)pipérazin-1-yl]butyl}amino-4-(2-méthylphényl)pyridine
- 2-{4-[4-(2,3-dichlorométhylphényl)pipérazin-1-yl]butyl}amino-4-(2-méthylphényl)pyridine
- 2-{4-[4-(2-cyano-3-trifluorométhylphényl)pipérazin-1-yl]butyl}amino-4-phénylpyridine
- 2-{4-[4-(3-chlorophényl)pipérazin-1-yl]butyl}-amino-4-phénylpyridine
- dichlorhydrate du 2-{4-[4-(5,6,7,8-tétrahydro-1-naphtyl)pipérazin-1-yl]butyl}amino-4-phénylpyridine
- 2-{4-[4-(2-cyano-3-isopropoxyphényl)pipérazin-1-yl]butyl}amino-4-phénylpyridine
- 2-{4-[4-(3-trifluorométhylphényl)pipérazin-1-yl]butyl}amino-4-(4-fluorophényl)pyridine
- 2-{4-[4-(3-trifluorométhylphényl)pipérazin-1-yl]butyl}amino-4-(4-chlororophényl)pyridine
- 2-{4-[4-(3-trifluorométhylphényl)pipérazin-1-yl]butyl}amino-4-(3-fluorophényl)pyridine
- 2-{4-[4-(3-trifluorométhylphényl)pipérazin-1-yl]butyl}amino-4-(4-méthoxyphényl)pyridine
- 2-{4-[4-(3-trifluorométhylphényl)pipérazin-1-yl]butyl}amino-4-(3-thiényl)pyridine
- 2-{4-[4-(3-trifluorométhylphényl)pipérazin-1-yl]butyl}amino-4-(2-furyl)pyridine
- 2-{4-[4-(3-trifluorométhylphényl)pipérazin-1-yl]butyl}amino-4-(2thiényl)pyridine
- 2-{4-[4-(3-trifluorométhylphényl)pipérazin-1-yl]butyl}amino-4-(2-fluorophényl)pyridine
- 2-(4-[4-(3-trifluorométhylphényl)pipérazin-1-yl]butyl)amino-[4 ,4']bipyridine
- 2-{4-[4-(3-trifluorométhylphényl)pipérazin-1-yl]butyl}amino-4-(3-méthylphényl)pyridine
- 2-{4-[4-(3-trifluorométhylphényl)pipérazin-1-yl]butyl}amino-4-(3-méthoxyphényl)pyridine
- 2-{4-[4-(3-trifluorométhylphényl)pipérazin-1-yl]butyl}amino-[4,3']bipyridine
- 2-{4-[4-(3-trifluorométhylphényl)pipérazin-1-yl]butyl}amino-4-(4-trifluorométhoxyphényl)pyridine
- 2-{4-[4-(3-trifluorométhylphényl)pipérazin-1-yl]butyl}amino-4-(4-acétylphényl)pyridine
- 2-{4-[4-(3-trifluorométhylphényl)pipérazin-1-yl]butyl}amino-4-(3-acétylphényl)pyridine
- 2-{4-[4-(3-trifluorométhylphényl)pipérazin-1-yl]butyl}amino-4-(4-hydroxyméthylphényl)pyridine
- 2-{4-[4-(3-trifluorométhylphényl)pipérazin-1-yl]butyl}amino-4-(benzo[1,3]dioxol-5-yl)pyridine
- 2-{4-[4-(3-trifluorométhylphényl)pipérazin-1-yl]butyl}amino-4-(4-éthoxycarbonylphényl)pyridine
- 2-{4-[4-(3-trifluorométhylphényl)pipérazin-1-yl]butyl}amino-4-(4-(1-hydroxyéthyl)phényl)pyridine
- 2-{4-[4-(3-trifluorométhylphényl)pipérazin-1-yl]butyl}amino-4-(4-(1-hydroxy-1-méthyléthyl)phényl)pyridine
- 2-{4-[4-(3-trifluorométhylphényl)pipérazin-1-yl]butyl}amino-4-(3-(1-hydroxy-1-méthyléthyl)phényl)pyridine
- sel de sodium de la 2-{4-[4-(3-trifluorométhylphényl)pipérazin-1-yl]butyl}amino-4-(4-carboxyphényl)pyridine
- 2-{4-[4-(3-trifluorométhylphényl)pipérazin-1-yl]butyl}amino-4-phénylthiazole
- 2-{4-[4-(3-trifluorométhylphényl)pipérazin-1-yl]butyl}amino-5-phényloxazole
ainsi que leurs stéréoisomères ou leur mélanges, leurs formes tautomères , leurs hydrates; solvates, leurs sels, formes libres et esters pharmaceutiquement acceptables.

8. Composés selon l'une quelconque des revendications précédentes choisis parmi :
- 2-{4-[4-(3-trifluorométhylphényl)pipérazin-1-yl]butyl}amino-4-phénylpyridine
- Dichlorhydrate de 2-{4-[4-(3-trifluorométhylphényl)pipérazin-1-yl]butyl}amino-4-phénylpyridine
- 2-{4-[4-(2,3-dichlorophényl)pipérazin-1-yl]butyl}amino-4-phénylpyridine, ainsi que leurs stéréoisomères ou leur mélanges, leurs formes tautomères , leurs hydrates, solvates, leurs sels, formes libres et esters pharmaceutiquement acceptables.

9. Procédé de préparation d'un composé selon l'une quelconque des revendications précédentes comprenant l'étape **caractérisée en ce que** l'on opère à partir d'un composé de formule (III) correspondant : 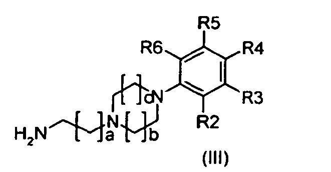 où, dans la formule (III), R2, R3, R4, R5, R6, a, b et c ont la même signification que dans la formule (I).

10. Procédé selon la revendication 9 tel que l'on opère par couplage dudit composé de formule (III) avec un composé de formule (II) correspondant : 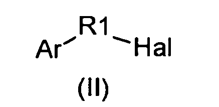 où Ar et R1 sont définis comme en formule (I).

11. Procédé selon la revendication 10 tel que la dite réaction de couplage est effectuée en chauffant à 300-350°C ou en chauffant dans un four à micro-ondes.

12. Procédé de préparation d'un composé selon l'une quelconque des revendications 1 à 8 comprenant l'étape **caractérisée en ce que** l'on opère à partir d'un composé de formule (VI) correspondant: 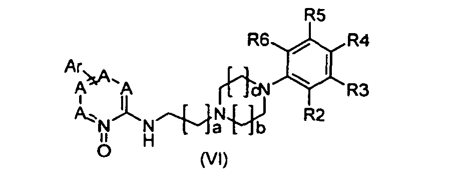 tel que R1 tel que défini en formule (I) est représenté par la formule : 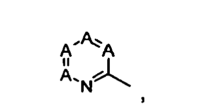 et A représente indépendamment un atome de carbone ou d'azote, où Ar, R2, R3, R4, R5, R6, a, b ; c sont définis comme en formule (I).

13. Procédé selon la revendication 12, tel que l'on opère par réduction de la fonction N-oxyde.

14. Procédé selon l'une quelconque des revendications 9 à 13 comprenant en outre l'étape d'isolation du produit de formule (I) obtenu.

15. Composition pharmaceutique, **caractérisée en ce qu'**elle comprend une quantité thérapeutiquement efficace d'au moins un dérivé selon l'une quelconque des revendications 1 à 8, sous forme d'hydrate, solvate, sel, forme libre ou ester pharmaceutiquement acceptable avec un véhicule ou excipient pharmaceutiquement acceptable.

16. Utilisation d'un composé de formule générale (I) : 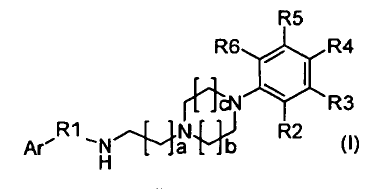 dans laquelle :
■ R1 représente un hétéroaryle à cinq ou six chaînons, contenant un ou plusieurs hétéroatomes, choisi parmi 2-pyridyle, 2-pyrimidinyle, 2-pyridazinyle, 2-pyrazinyle, 2-imidazolyle, 2-oxazolyle, 2-thiazolyle, 3-isoxazolyle, 3-isothiazolyle, 1,2,4-triazol-2-yle, 1,3,4-oxadiazol-2-yle, 1,3,4-thiadiazol-2-yle, éventuellement substitué par un ou plusieurs groupements identiques ou différents choisis parmi un atome d'halogène ou un groupe hydroxy, alkyle, monofluoroalkyle, polyfluoroalkyle, alkoxy, polyfluoroalkoxy, alkylsulfanyle, polyfluoroalkylsulfanyle ;
■ Ar est un aryle ou hétéroaryle, éventuellement substitué par un ou plusieurs substituants identiques ou différents choisis parmi un atome d'halogène ou un groupe hydroxy, alkyle, monofluoroalkyle, polyfluoroalkyle, alkoxy, polyfluoroalkoxy, alkylsulfanyle, polyfluoroalkylsulfanyle, cyano, carbamoyle, dialkylcarbamoyle, Alkyl-C(=O)-, Alkyl-O-C(=O)-, HO-C(=O)-, (HO)Alkyl-, ou Ar est un cycle hydrocarboné ou hétérocycle, saturé, insaturé ou aromatique ;
■ a = 2, 3 ou 4 ;
■ b et c identiques ou différents représentent 1 ou 2 ;
■ R2, R3, R4, R5 et R6 représentent chacun indépendamment un atome d'hydrogène ou d'halogène ou un groupement hydroxy, alkyle, monofluoroalkyle, polyfluoroalkyle, alkoxy, polyfluoroalkoxy, alkylsulfanyle, polyfluoroalkylsulfanyle, cyano, -NRR', -COOR, -COR, -CONRR' ou bien deux des R2, R3, R4, R5 et R6 adjacents sont reliés entre eux pour former un cycle hydrocarboné ou un hétérocycle saturé ou insaturé, fusionné au noyau phényle auquel ils sont rattachés ;
où R, R' identiques ou différents représentent indépendamment un atome d'hydrogène, ou un groupe alkyle ;
ainsi que leurs stéréoisomères ou leur mélanges, leurs formes tautomères, leurs hydrates, solvates, leurs sels et esters pharmaceutiquement acceptables,
pour la préparation de compositions pharmaceutiques destinées à agir comme ligand du récepteur D3 de la dopamine.

17. Utilisation d'un composé de formule générale (I) tel que défini selon l'une quelconque des revendications 1 à 8 pour la préparation de compositions pharmaceutiques destinées à prévenir et/ou traiter une affection neuro-psychiatrique.

18. Utilisation d'un composé de formule générale (I) telle que définie selon l'une quelconque des revendications 1 à 8 pour la préparation de compositions pharmaceutiques destinées à prévenir et/ou traiter les affections faisant intervenir le récepteur D3 de la dopamine.

19. Utilisation selon la revendication 18 telle que ladite affection est choisie parmi la pharmacodépendance, les troubles de la sphère sexuelle, les troubles moteurs, la maladie de Parkinson, la psychose ou état psychotique, la dépression ou pharmacodépendance.

20. Utilisation selon la revendication 18, telle que ladite pharmacodépendance comprend tout état lié à la désaccoutumance, l'abstinence et/ou à la désintoxication d'un sujet dépendant de tout agent, notamment les agents actifs thérapeutiques ; tels que les morphiniques, et/ou drogues telles que la cocaïne, l'héroïne, ou encore l'alcool et/ou la nicotine.

21. Utilisation selon la revendication 18 telle que lesdits troubles de la sphère sexuelle comprennent l'impuissance, notamment l'impuissance masculine.

22. Utilisation selon la revendication 18 telle que ladite prévention et/ou traitement de la maladie de Parkinson est un traitement complémentaire de la maladie de Parkinson.

23. Utilisation selon la revendication 18, telle que lesdits troubles moteurs comprennent les dyskinésies essentielles ou iatrogènes, et/ou les tremblements essentiels ou iatrogènes.

## Claims

1. Compounds of the Formula (I): 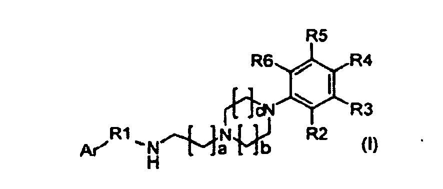 in which:
• R1 denotes a heteroaryl with five or six chain members, containing one or more heteroatoms, chosen from 2-pyridyl, 2-pyrimidinyl, 2-pyridazinyl, 2-pyrazinyl, 2-imidazolyl, 2-oxazolyl, 2-thiazolyl, 3-isoxazolyl, 3-isothiazolyl, 1,2,4-triazol-2-yl, 1,3,4-oxadiazol-2-yl, 1,3,4-thiadiazol-2-yl, optionally substituted by one or more identical or different groupings chosen from a halogen atom or a hydroxy, alkyl, monofluoroalkyl, polyfluoroalkyl, alkoxy, polyfluoroalkoxy, alkylsulfanyl, polyfluoroalkylsulfanyl group;
• Ar is an aryl or heteroaryl, optionally substituted by one or more identical or different substituents chosen from a halogen atom or a hydroxy, alkyl, monofluoroalkyl, polyfluoroalkyl, alkoxy, polyfluoroalkoxy, alkylsulfanyl, polyfluoroalkylsulfanyl, cyano, carbamoyl, dialkylcarbamoyl, alkyl-C(=O)-, alkyl-O-C(=O)-, HO-C(=O)-, (HO)alkyl-, or Ar is fused with a hydrocarbon ring or a saturated, unsaturated or aromatic heterocycle;
• a = 2, 3 or 4;
• b and c are identical or different and are 1 or 2;
• R2, R3, R4, R5 and R6 each independently denotes a hydrogen or halogen atom or a hydroxy, alkyl, monofluoroalkyl, polyfluoroalkyl, alkoxy, polyfluoroalkoxy, alkylsulfanyl, polyfluoroalkylsulfanyl, cyano, -NRR', -COOR, -COR, -CONRR' group, or two of the adjacent R2, R3, R4, R5 and R6 are joined to one another so as to form a hydrocarbon ring or a saturated or unsaturated heterocycle, fused to the phenyl nucleus to which they are attached;
where R, R', which are identical or different, independently denote a hydrogen atom or an alkyl group;
as well as their stereoisomers or their mixtures, their tautomeric forms, their hydrates and solvates, and their pharmaceutically acceptable salts and esters.

2. Compounds according to claim 1, where R1 denotes 2-pyridyl.

3. Compounds according to claim 1 or 2, where Ar is an aryl radical, optionally substituted by one or more identical or different substituents chosen from a halogen atom or an alkyl group.

4. Compounds according to any one of the preceding claims, in which a = 3.

5. Compounds according to any one of the preceding claims, in which b and c are 1.

6. Compounds according to any one of the preceding claims, in which R2, R3, R4, R5 and R6 each independently denotes a hydrogen or halogen atom or a polyfluoroalkyl group.

7. Compounds according to any one of the preceding claims, chosen from:
- 2-{4-[4-(2-fluorophenyl)piperazin-1-yl]butyl}amino-5-phenylpyridine
- 2-{4-[4-(2-fluorophenyl)piperazin-1-yl]butyl}amino-4-phenylpyridine
- 2-{4-[4-(3-trifluoromethylphenyl)piperazin-1-yl]butyl}amino-4-phenylpyridine
- 2-{4-(3-trifluoromethylphenyl)piperazin-1-yl]butyl}amino-4-phenylpyridine dihydrochloride
- 2-{4-[4-(3-trifluoromethylphenyl)piperazin-1-yl]butyl}amino-5-phenylpyridine
- 2-{4-[4-(2,3-dichlorophenyl)piperazin-1-yl]butyl}amino-4-phenylpyridine
- 2-{4-[4-(2,3-dichlorophenyl)piperazin-1-yl]butyl}amino-5-phenylpyridine
- 2-{4-[4-(2,3-dichlorophenyl)piperazin-1-yl)butyl}amino-5-(2-methylphenyl)pyridine
- 2-{4-[4-(3-trifluoromethylphenyl)piperazin-1-yl]butyl}amino-5-(2-methylphenyl)pyridine
- 2-{4-[4-(3-trifluoromethylphenyl)piperazin-1-yl]butyl}amino-5-(4-fluorophenyl)pyridine
- 2-{4-[4-(3-trifluoromethylphenyl)piperazin-1-yl)butyl}amino-4-(2-methylphenyl)pyridine
- 2-{4-[4-{2,3-dichloromethylphenyl)piperazin-1-yl)butyl}amino-4-{2-methylphenyl)pyridine
- 2-{4-[4-(2-cyano-3-trifluoromethylphenyl)piperazin-1-yl]butyl}amino-4-phenylpyridine
- 2-{4-[4-(3-chlorophenyl)piperazin-1-yl]butyl}-amino-4-phenylpyridine
- 2-{4-[4-(5,6,7,8-tetrahydro-1-naphthyl)piperazin-1-yl]butyl}amino-4-phenylpyridine dihydrochloride
- 2-{4-[4-(2-cyano-3-isopropoxyphenyl)piperazin-1-yl]butyl}amino-4-phenylpyridine
- 2-{4-[4-(3-trifluoromethylphenyl)piperazin-1-yl]butyl}amino-4-{4-fluorophenyl)pyridine
- 2-{4-[4-{3-trifluoromethylphenyl)piperazin-1-yl]butyl}amino-4-(4-chlororophenyl)pyridine
- 2-{4-[4-(3-trifluoromethylphenyl)piperazin-1-yl]butyl}amino-4-(3-fluorophenyl)pyridine
- 2-{4-[4-(3-trifluoromethylphenyl)piperazin-1-yl)butyl}amino-4-(4-methoxyphenyl)pyridine
- 2-{4-[4-(3-trifluoromethylphenyl)piperazin-1-yl]butyl}amino-4-{3-thienyl)pyridine
- 2-{4-[4-(3-trifluoromethylphenyl)piperazin-1-yl)butyl}amino-4-(2-furyl)pyridine
- 2-{4-[4-(3-trifluoromethylphenyl)piperazin-l-yl]butyl}amino-4-{2-thienyl}pyridine
- 2-{4-[4-(3-trifluoromethylphenyl)piperazin-1-yl]butyl}amino-4-(2-fluorophenyl)pyridine
- 2-{4-[4-(3-trifluoromethylphenyl)piperazin-1-yl]butyl}amino-[4,4']bipyridine
- 2-{4-[4-(3-trifluoromethylphenyl)piperazin-1-yl]butyl}amino-4-(3-methylphenyl)pyridine
- 2-{4-[4-(3-trifluoromethylphenyl)piperazin-1-yl]butyl}amino-4-(3-methoxyphenyl)pyridine
- 2-{4-[4-(3-trifluoromethylphenyl)piperazin-1-yl]butyl}amino-[4,3')bipyridine
- 2-{4-[4-(3-trifluoromethylphenyl)piperazin-1-yl]butyl}amino-4-(4-trifluoromethoxyphenyl)pyridine
- 2-{4-[4-(3-trifluoromethylphenyl)piperazin-1-yl)butyl}amino-4-(4-acetylphenyl)pyridine
- 2-{4-[4-(3-trifluoromethylphenyl)piperazin-1-yl)butyl}amino-4-(3-acetylphenyl)pyridine
- 2-{4-[4-(3-trifluoromethylphenyl)piperazin-1-yl]butyl}amino-4-(4-hydroxymethylphenyl) pyridine
- 2-{4-[4-(3-trifluoromethylphenyl) piperazin-1-yl]butyl}amino-4(benzo[l,3]dioxol-5-yl)pyridine
- 2-{4-[4-(3-trifluoromethylphenyl)piperazin-1-yl]butyl}amino-4-(4-ethoxycarbonylphenyl)pyridine
- 2-{4-[4-(3-trifluoromethylphenyl)piperazin-1-yl]butyl}amino-4-(4-(1-hydroxyethyl)phenyl)pyridine
- 2-{4-[4-(3-trifluoromethylphenyl)piperazin-1-yl]butyl}amino-4-(4-(1-hydroxy-1-methylethyl) phenyl)pyridine
- 2-{4-[4-(3-trifluoromethylphenyl}piperazin-1-yl]butyl}amino-4-(3-(1-hydroxy-1methyl-ethyl) phenyl) pyridine
- sodium salt of 2-{4-[4-(3-trifluoromethylphenyl) piperazin-1-yl]butyl}amino-4-(4-carboxyphenyl)pyridine
- 2-{4-[4-(3-trifluoromethylphenyl}piperazin-1-yl]butyl}amino-4-phenylthiazole
- 2-{4-[4-(3-trifluoromethylphenyl)piperazin-1-yl}butyl}amino-5-phenyloxazole
as well as their stereoisomers or their mixtures, their tautomeric forms, their hydrates, solvates, their salts, free forms, and pharmaceutically acceptable esters.

8. Compounds according to any one of the preceding claims, chosen from:
- 2-{4-[4-(3-trifluoromethylphenyl)piperazin-1-yl]butyl}amino-4-phenylpyridine
- 2-{4-(4-(3-trifluoromethylphenyl) piperazin-1-yl]butyl}amino-4-phenylpyridine dihydrochloride
- 2-{4-[4-(2,3-dichlorophenyl)piperazin-1-yl]butyl}amino-4-phenylpyridine,
as well as their stereoisomers or their mixtures, their tautomeric forms, their hydrates, solvates, their salts, free forms, and pharmaceutically acceptable esters.

9. Process for preparing a compound according to any one of the preceding claims, comprising the stage **characterised in that** it is carried out starting from a compound corresponding to the Formula (III): 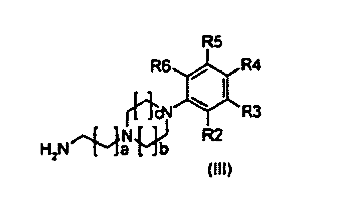 where in the Formula (III), R2, R3, R4, R5, R6, a, b and c have the same meanings as in Formula (I).

10. Process according to claim 9, in which the said compound of the Formula (III) is coupled with a compound corresponding to Formula (II): 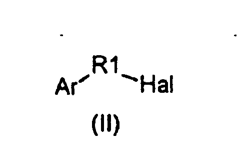 where Ar and R1 are defined as in Formula (I).

11. Process according to claim 10, in which the said coupling reaction is carried out by heating at 300-350°C or by heating in a microwave furnace.

12. Process for preparing a compound according to any one of claims 1 to 8, comprising the stage **characterised in that** it is carried out starting from a compound corresponding to the Formula (VI): 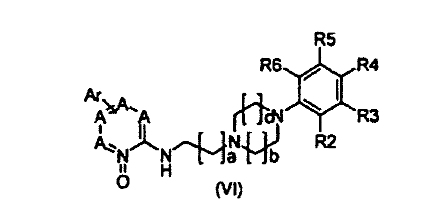 in which R1 as defined in Formula (I) is represented by the formula: 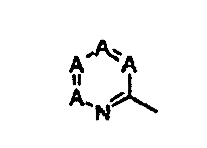 and A denotes independently a carbon or nitrogen atom, where Ar, R2, R3, R4, R5, R6, a, b, c are defined as in Formula (I).

13. Process according to claim 12, in which the N-oxide group is reduced.

14. Process according to any one of claims 9 to 13, comprising in addition the stage of separating the obtained product of the Formula (I).

15. Pharmaceutical composition, **characterised in that** it comprises a therapeutically effective amount of at least one derivative according to any one of claims 1 to 8, in the form of a hydrate, solvate, salt, free form or pharmaceutically acceptable ester with a pharmaceutically acceptable vehicle or excipient.

16. Use of a compound of the general Formula (I): 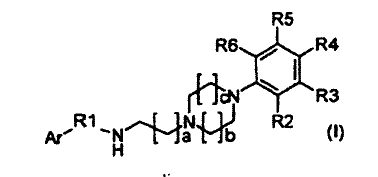 in which:
• R1 denotes a heteroaryl with five or six chain members, containing one or more heteroatoms, chosen from 2-pyridyl, 2-pyrimidinyl, 2-pyridazinyl, 2-pyrazinyl, 2-imidazolyl, 2-oxazolyl, 2-thiazolyl, 3-isoxazolyl, 3-isothiazolyl, 1,2,4-triazol-2-yl, 1,3,4-oxadiazol-2-yl, 1,3,4-thiadiazol-2-yl, optionally substituted by one or more identical or different groupings chosen from a halogen atom or a hydroxy, alkyl, monofluoroalkyl, polyfluoroalkyl, alkoxy, polyfluoroalkoxy, alkylsulfanyl, polyfluoroalkylsulfanyl group;
• Ar is an aryl or heteroaryl, optionally substituted by one or more identical or different substituents chosen from a halogen atom or a hydroxy, alkyl, monofluoroalkyl, polyfluoroalkyl, alkoxy, polyfluoroalkoxy, alkylsulfanyl, polyfluoroalkylsulfanyl, cyano, carbamoyl, dialkylcarbamoyl, alkyl-C(=O)-, alkyl-O-C(=O)-, HO-C(=O)-, (HO)alkyl-, or Ar is a hydrocarbon ring or a saturated, unsaturated or aromatic heterocycle;
• a = 2, 3 or 4;
• b and c are identical or different and are 1 or 2;
• R2, R3, R4, R5 and R6 each independently denotes a hydrogen or halogen atom or a hydroxy, alkyl, monofluoroalkyl, polyfluoroalkyl, alkoxy, polyfluoroalkoxy, alkylsulfanyl, polyfluoroalkylsulfanyl, cyano, -NRR', -COOR, -COR, -CONRR' group, or two of the adjacent R2, R3, R4, R5 and R6 are joined to one another so as to form a hydrocarbon ring or a saturated or unsaturated heterocycle, fused to the phenyl nucleus to which they are attached;
where R, R', which are identical or different, independently denote a hydrogen atom or an alkyl group;
as well as their stereoisomers or their mixtures, their tautomeric forms, their hydrates and solvates, and their pharmaceutically acceptable salts and esters, for the preparation of pharmaceutical compositions intended to act as selective ligand for the dopamine D3 receptor.

17. Use of a compound of the general Formula (I) as defined according to any one of claims 1 to 8, for the preparation of pharmaceutical compositions intended to prevent and/or treat a neuropsychiatric condition.

18. Use of a compound of the general Formula (I) as defined according to any one of claims 1 to 8, for the preparation of pharmaceutical compositions intended to prevent and/or treat conditions involving the dopamine D3 receptor.

19. Use according to claim 18, in which the condition is chosen from drug dependence, sexual disorders, motor disorders, Parkinson's disease, psychosis or psychotic states, depression or drug dependence.

20. Use according to claim 18, in which the said drug dependence comprises any state associated with withdrawal, abstinence and/or detoxification of a patient dependent on any agent, in particular therapeutic active agents such as morphine derivatives and/or drugs such as cocaine, heroin, or also alcohol and/or nicotine.

21. Use according to claim 18, in which the said sexual disorders include impotence, in particular male impotence.

22. Use according to claim 18, in which the said prevention and/or treatment of Parkinsons disease is a complementary treatment for Parkinsons disease.

23. Use according to claim 18, in which the said motor disorders include essential or iatrogenic dyskinesias, and/or essential or iatrogenic tremors.

## Patentansprüche

1. Verbindung mit der Formel (I): 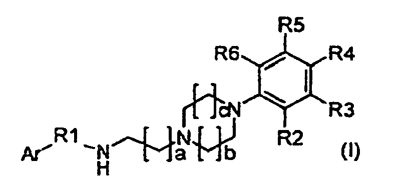 worin:
• R1 ein fünf oder sechs kettiges Heteroaryl bedeutet, welches, ein oder mehrere Heteroatome enthaltend, ausgewählt ist aus der Gruppe: 2-Pyridyl, 2-Pyrimidinyl, 2-Pyridazinyl, 2-Pyrazinyl, 2-Imidazolyl, 2-Oxazolyl, 2-Thiazolyl, 3-Isoxazolyl, 3-Isothiazolyl, 1,2,4-Triazol-2-yl, 1,3,4-Oxadiazol-2-yl, 1,3,4-Thiadiazol-2-yl, wahlweise substituiert durch eine oder mehrere identische oder verschiedene Gruppen ausgewählt aus einem Atom der Halogene oder einer Hydroxyl-, Alkyl-, Monofluoroalkyl-, Polyfluoroalkyl-, Alkoxy-, Polyfluoroalkoxy-, Alkylsulfanyl-, Polyfluoroalkylsulfanyl-Gruppe;
• Ar ist ein Aryl oder Heteroaryl, wahlweise substituiert mit einem oder mehreren identischen oder unterschiedlichen Substituenten ausgewählt aus der Gruppe von einem Halogenatom oder einer Hydroxyl-, Alkyl-, Monofluoroalkyl-, Polyfluoroalkyl-, Alkoxy-, Polyfluoroalkoxy-, Alkylsulfanyl-, Polyfluoroalkylsulfanyl-, Cyano-, Carbamoyl-, Dialkylcarbamoyl-, Alkyl-C(=O)-, Alkyl-O-C(=O)-, HO-C(=O)-, (HO)Alkyl-Gruppe, oder Ar ist mit einem Kohlenwasserstoff-Ring oder einem gesättigten oder ungesättigten oder aromatischen Heteroring verbunden; a = 2, 3 oder 4 ;
• b und c sind identisch oder verschieden und bedeuten 1 oder 2;
• R2, R3, R4, R5 und R6 sind jeweils unabhängig voneinander und bedeuten ein Wasserstoffatom oder ein Halogenatom oder eine Hydroxyl-, Alkyl-, Monofluoroalkyl-, Polyfluoroalkyl-, Alkoxy-, Polyfluoroalkoxy-, Alkylsulfanyl-, Polyfluoroalkylsulfanyl-, Cyano-, -NRR', -COOR, -COR, - CONRR' - Gruppe oder wenigstens zwei benachbarte von R2, R3, R4, R5 und R6 sind miteinander verbunden zu einem Kohlenwasserstoff-Ring oder einem gesättigten oder ungesättigten Heteroring, verbunden mit einem Phenylkern an welchem sie angeordnet sind; wobei R, R' identisch oder unterschiedlich sind und unabhängig voneinander ein Wasserstoffatom oder eine Alkaligruppe bedeuten;
• sowie ihre pharamazeutisch verträglichen Stereoisomere oder Mischungen, tautomere Formen, Hydrate, Lösungen, Salze und Ester.

2. Verbindung nach Anspruch 1, in der R1 2-Pyridyl bedeutet.

3. Verbindung nach Anspruch 1 oder 2, in der Ar ein Aryl ist, möglicherweise substituiert durch ein Wasserstoffatom oder eine Alkylgruppe.

4. Verbindung nach einem der vorherigen Ansprüche, wobei a=3 ist.

5. Verbindung nach einem der vorherigen Ansprüche, wobei b und c 1 bedeuten.

6. Verbindung nach einem der vorherigen Ansprüche, wobei R2, R3, R4, R5 und R6 unabhängig voneinander ein Wasserstoff- oder Halogen-Atom oder eine Polyflouroalkyl-Gruppe bedeuten.

7. Verbindung nach einem der vorherigen Ansprüche, wobei die Verbindung ausgewählt ist aus der Gruppe von:
- 2-{4-[4-(2-Fluorophenyl)piperazin-1-yl]butyl}amino-5-phenylpyridin
- 2-{4-[4-(2-Fluorophenyl)piperazin-1-yl]butyl}amino-4-phenylpyridin
- 2-{4-[4-(3-Trifluoromethylphenyl)piperazin-1-yl]butyl}amino-4-phenylpyridin
- Dichlorhydrat von 2-{4-(4-(3-Trifluoromethylphenyl)piperazin-1-yl]butyl}amino-4-phenylpyridin
- 2-{4-[4-(3-Trifluoromethylphenyl)piperazin-1-yl]butyl}amino-5-phenylpyridin
- 2-{4-[4-(2,3-Dichlorophenyl)piperazin-1-yl]butyl}amino-4-phenylpyridin
- 2-{4-[4-(2,3-Dichlorophenyl)piperazin-1-yl]butyl}amino-5-phenylpyridin
- 2-{4-[4-(2,3-Dichlorophenyl)piperazin-1-yl]butyl}amino-5-(2-methylphenyl)pyridin
- 2-{4-[4-(3-Trifluoromethylphenyl)piperazin-1-yl]butyl}amino-5-(2-methylphenyl)pyridin
- 2-{4-[4-(3-Trifluoromethylphenyl)piperazin-1-yl]butyl}amino-5-(4-fluorophenyl)pyridin
- 2-{4-[4-(3-Trifluoromethylphenyl)piperazin-1-yl]butyl}amino-4-(2-methylphenyl)pyridin
- 2-{4-[4-(2,3-Dichloromethylphenyl)piperazin-1-yl]butyl}amino-4-(2-methyl-phenyl)pyridin
- 2-{4-[4-(2-Cyano-3-trifluoromethylphenyl)piperazin-1-yl]butyl}amino-4-phenylpyridin
- 2-{4-[4-(3-Chlorophenyl)piperazin-1-yl]butyl}-amino-4-phenylpyridin
- Dichlorhydrat von 2-{4-[4-(5,6,7,8-Tetrahydro-1-naphtyl)piperazin-1-yl]butyl}amino-4-phenylpyridin
- 2-{4-[4-(2-Cyano-3-isopropoxyphenyl)piperazin-1-yl]butyl}amino-4-phenylpyridin
- 2-{4-[4-(3-Trifluoromethylphenyl)piperazin-1-yl]butyl}amino-4-(4-fluorophenyl)pyridin
- 2-{4-[4-(3-Trifluoromethylphenyl)piperazin-1-yl]butyl}amino-4-(4-chlororophenyl)pyridine
- 2-{4-[4-(3-Trifluoromethylphenyl)piperazin-l-yl]butyl}amino-4-(3-fluorophenyl)pyridin
- 2-{4-[4-(3-Trifluoromethylphenyl)piperazin-1-yl]butyl}amino-4-(4-methoxyphenyl)pyridin
- 2-{4-[4-(3-Trifluoromethylphenyl)piperazin-1-yl]butyl}amino-4-(3-thienyl)pyridin
- 2-{4-[4-(3-Trifluoromethylphenyl)piperazin-1-yl]butyl}amino-4-(2-furyl)pyridin
- 2-{4-[4-(3-Trifluoromethylphenyl)piperazin-1-yl]butyl}amino-4-(2-thienyl)pyridin
- 2-{4-[4-(3-Trifluoromethylphenyl)piperazin-1-yl]butyl}amino-4-(2-fluorophenyl)pyridin
- 2-{4-[4-(3-Trifluoromethylphenyl)piperazin-1-yl]butyl}amino-[4,4']bipyridin
- 2-{4-[4-(3-Trifluoromethylphenyl)piperazin-1-yl]butyl}amino-4-(3-methylphenyl)pyridin
- 2-{4-[4-(3-Trifluoromethylphenyl)piperazin-1-yl]butyl}amino-4-(3-methoxyphenyl)pyridin
- 2-{4-[4-(3-Trifluoromethylphenyl)piperazin-1-yl]butyl}amino-[4,3']bipyridin
- 2-{4-[4-(3-Trifluoromethylphenyl)piperazin-1-yl]butyl}amino-4-(4-trifluoromethoxyphenyl)pyridin
- 2-{4-[4-(3-Trifluoromethylphenyl)piperazin-1-yl]butyl}amino-4-(4-acetylphenyl)pyridin
- 2-{4-[4-(3-Trifluoromethylphenyl)piperazin-1-yl]butyl}amino-4-(3-acetylphenyl)pyridin
- 2-{4-[4-(3-Trifluoromethylphenyl)piperazin-1-yl]butyl}amino-4-(4-hydroxymethylphenyl)pyridin
- 2-{4-[4-(3-Trifluoromethylphenyl)piperazin-1-yl]butyl}amino-4-(benzo[1,3]dioxol-5-yl)pyridin
- 2-{4-[4-(3-Trifluoromethylphenyl)piperazin-1-yl]butyl}amino-4-(4-ethoxycarbonylphenyl)pyridin
- 2-{4-[4-(3-Trifluoromethylphenyl)piperazin-1-yl]butyl}amino-4-(4-(1-hydroxyethyl)phenyl)pyridin
- 2-{4-(4-(3-Trifluoromethylphenyl)piperazin-1-yl]butyl}amino-4-(4-(1-hydroxy-1-methylethyl)phenyl)pyridin
- 2-{4-[4-(3-Trifluoromethylphenyl)piperazin-1-yl]butyl}amino-4-(3-(1-hydroxy-1-methylethyl)phenyl)pyridin
- Natriumsalz von 2-{4-[4-(3-Trifluoromethylphenyl)piperazin-1-yl]butyl}amino-4-(4-carboxyphenyl) pyridin
- 2-{4-[4-(3-Trifluoromethylphenyl)piperazin-1-yl]butyl} amino-4-phenylthiazol
- 2-{4-[4-(3-Trifluoromethylphenyl)piperazin-1-yl]butyl}amino-5-phenyloxazol
- Chlorhydrat von 2-{4-[4-(3-Trifluoromethylphenyl)piperazin-1-yl]butyl}aminoquinolein
- Chlorhydrat von 1-{4-[4-(3-Trifluoromethylphenyl)piperazin-1-yl]butyl}aminoisoquinolein
- Chlorhydrat von 2-{4-[4-(2,3-Dichlorophenyl)piperazin-1-yl]butyl}aminoquinolein
- Chlorhydrat von 1-{4-[4-(2,3-Dichlorophenyl)piperazin-1-yl]butyl}aminoisoquinolein,
oder ihren pharmazeutisch verträglichen Stereoisomeren oder Mischungen, tautomeren Formen, Hydrate, Lösungen, Salze, freie Formen und Esther.

8. Verbindung nach einem der vorherigen Ansprüche, worin die Verbindung ausgewählt ist aus der Gruppe von:
- 2-{4-[4-(3-Trifluoromethylphenyl)piperazin-1-yl]butyl}amino-4-phenylpyridin
- Dichlorhydrat von 2-{4-[4-(3-Trifluoromethylphenyl)piperazin-l-yl]butyl}amino-4-phenylpyridin
- 2-{4-[4-(2,3-Dichlorophenyl)piperazin-1-yl]butyl}amino-4-phenylpyridin,
oder ihren pharmazeutisch verträglichen Stereoisomeren oder Mischungen, tautomeren Formen, Hydrate, Lösungen, Salze, freie Formen und Ester.

9. Verfahren zur Herstellung einer Verbindung nach einem der vorherigen Ansprüche, wobei das Verfahren eine Stufe umfasst die **dadurch** gekennzeichent ist, dass mit einer Verbindung nach folgender Formel (III) gestartet wird: 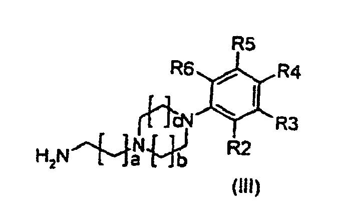 wobei, in der Formel (III), R2, R3, R4, R5, R6, a, b und c die gleiche Bedeutung haben wie in der Formel (I).

10. Verfahren nach Anspruch 9, **dadurch gekennzeichnet, dass** die Verbindung nach Formel (III) mit folgender Verbindung nach Formel (II) gekoppelt wird: 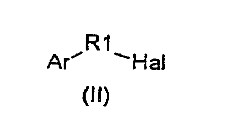 wobei Ar und R1 wie in Formel(I) definiert sind.

11. Verfahren nach Anspruch 10 **dadurch gekennzeichnet, dass** die genannte Kopplungsreaktion durch Heizen auf 300-350°C oder durch Heizen in einem Mikrowellenofen ausgeführt wird.

12. Verfahren zur Herstellung einer Verbindung nach Anspruch 1-8 wobei das Verfahren eine Stufe umfasst die **dadurch gekennzeichnet ist, dass** man mit einer Verbindung nach folgender Formel (VI) startet: 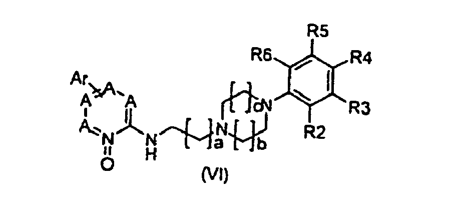 wobei R1 defniert in Formel (I) durch folgende Formel repräsentiert wird: 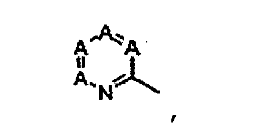 und A unabhängig ein Kohlenstoff- oder Stickstoff-Atom bedeutet, wobei Ar, R2, R3, R4, R5, R6, a, b, c wie in Formel (I) definiert sind.

13. Verfahren nach Anspruch 12, **dadurch gekennzeichnet, dass** die funktionelle N-Oxid Gruppe reduziert wird.

14. Verfahren nach einem der Ansprüche 9-13, **dadurch gekennzeichnet, dass** das Verfahren eine Isolationsstufe der Produkte nach Formel (I) umfasst.

15. Pharmazeutische Verbindung, **dadurch gekennzeichnet, dass** sie eine therapeutisch wirksame Menge wenigstens einer der pharmazeutisch verträglichen Derivate nach Anspruch 1-8 als Hydrat-, lösliche, Salz-, freie Form oder Ester mit einem pharmazeutisch verträglichen Grund- oder Trägerstoff enthält.

16. Verwendung einer Verbindung nach der allgemeinen Formel (I): 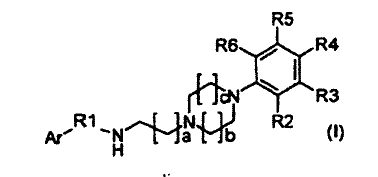 worin :
• R1 ein fünf oder sechs kettiges Heteroaryl bedeutet, welches, ein oder mehrere Heteroatome enthaltend, ausgewählt ist aus der Gruppe: 2-Pyridyl, 2-Pyrimidinyl, 2-Pyridazinyl, 2-Pyrazinyl, 2-Imidazolyl, 2-Oxazolyl, 2-Thiazolyl, 3-Isoxazolyl, 3-Isothiazolyl, 1,2,4-Triazol-2-yl, 1,3,4-Oxadiazol-2-yl, 1,3,4-Thiadiazol-2-yl, wahlweise substituiert durch eine oder mehrere identische oder verschiedene Gruppen ausgewählt aus einem Atom der Halogene oder einer Hydroxyl-, Alkyl-, Monofluoroalkyl-, Polyfluoroalkyl-, Alkoxy-, Polyfluoroalkoxy-, Alkylsulfanyl-, Polyfluoroalkylsulfanyl-Gruppe;
• Ar ist ein Aryl oder Heteroaryl, wahlweise substituiert mit einem oder mehreren identischen oder unterschiedlichen Substituenten ausgewählt aus der Gruppe: einem Halogenatom oder einer Hydroxyl-, Alkyl-, Monofluoroalkyl-, Polyfluoroalkyl-, Alkoxy-, Polyfluoroalkoxy-, Alkylsulfanyl-, Polyfluoroalkylsulfanyl-, Cyano-, Carbamoyl-, Dialkylcarbamoyl-, Alkyl-C(=O)-, Alkyl-O-C(=O)-, HO-C(=O)-, (HO)Alkyl-Gruppe, oder Ar ist verbunden mit einem Kohlenwasserstoff-Ring oder einem gesättigten oder ungesättigten oder aromatischen Heteroring verbunden;
a = 2, 3 oder 4 ;
• b und c sind identisch oder verschieden und bedeuten 1 oder 2;
• R2, R3, R4, R5 und R6 sind jeweils unabhängig voneinander und bedeuten ein Wasserstoffatom oder ein Halogenatom oder eine Hydroxyl-, Alkyl-, Monofluoroalkyl-, Polyfluoroalkyl-, Alkoxy-, Polyfluoroalkoxy-, Alkylsulfanyl-, Polyfluoroalkylsulfanyl-, Cyano-, -NRR', -COOR, -COR, - CONRR' Gruppe oder wenigstens zwei benachbarte von R2, R3, R4, R5 und R6 sind miteinander verbunden zu einem Kohlenwasserstoff-Ring oder einem gesättigten oder ungesättigten Heteroring, verbunden mit einem Phenylkern an welchem sie angeordnet sind;
wobei R, R' identisch oder unterschiedlich sind und unabhängig voneinander ein Wasserstoffatom oder eine Alkaligruppe bedeuten;
sowie ihre pharamazeutisch verträglichen Stereoisomere oder Mischungen, tautomere Formen, Hydrate, Lösungen, Salze und Ester,
für die Herstellung pharmazeutischer Verbindungen, die als Ligand des Dopamin D3 Rezeptor wirken.

17. Verwendung einer Verbindung mit der nach den Ansprüchen 1-8 definierten allgemeinen Formel (I) für die Herstellung einer pharmazeutischen Verbindung bestimmt zur Vorbeugung und/oder Behandlung von neuropsychatrischen Leiden.

18. Verwendung einer Verbindung mit der nach den Ansprüchen 1-8 definierten allgemeinen Formel (I) für die Herstellung einer pharmazeutischen Verbindung bestimmt zur Vorbeugung und/oder Behandlung von Leiden indem an den Dopamin D3 Rezeptor eingegriffen wird.

19. Verwendung nach Anspruch 18 worin besagtes Leiden aus Drogenabhängigkeit, sexuelle Störungen, motorische Störungen, Parkinson, Psychose oder psychotische Zustände oder Depressionen ausgewählt ist.

20. Verwendung nach Anspruch 18, wobei besagte Drogenabhängigkeit jeglichen Zustand des Entzugs, Abstinenz und/oder Entgiftung eines von allen Mitteln, insbesondere therapeutisch aktiven Mitteln , wie Morphine und oder Drogen wie Kokain, Heroin oder auch Alkohol und/oder Nikotin, Abhängigen beinhaltet.

21. Verwendung nach Anspruch 18, wobei besagte sexuelle Störungen die Impotenz, insbesondere die männliche Impotenz beinhaltet.

22. Verwendung nach Anspruch 18, wobei besgate Vorsorge und/oder Behandlung von Parkinson eine ergänzende Behandlung von Parkinson ist.

23. Verwendung nach Anspruch 18, wobei besagte motorische Störungen eine essentielle oder iatrogene Dyskinesie und/oder einen essentiellen oder iatrogenen Tremor beinhaltet.
